# EUROPEAN PATENT APPLICATION

(11) **EP 4 813 969 A1**
(43) Date of publication of application: **30.09.2026**
(21) Application number: 26165497.4
(22) Date of filing: 17.03.2026
(51) Int. Cl.: C07C 201/00, C01B 35/06, C07D 201/00, C07F 5/02, C09K 11/06, H10K 85/00

(54) **LIGHT EMITTING ELEMENT, METHOD OF MANUFACTURING POLYCYCLIC COMPOUND FOR THE SAME, AND ELECTRONIC DEVICE INCLUDING THE SAME**

(30) Priority: 24.03.2025 JP 2025047722
(71) Applicant: Samsung Display Co., Ltd., Yongin-si, Gyeonggi-do (KR)
(72) Inventor: SUZAKI, Yuji, Yokohama-shi (JP); SUGITA, Yoshiro, Yokohama-shi (JP); MIYAZAKI, Yuuki, Yokohama-shi (JP)
(74) Representative: Marks & Clerk LLP

(57) **Abstract**

A method of manufacturing a polycyclic compound represented by Formula 1, the method including providing a first material represented by Formula 2 and adding a second material and a third material different from the second material to the first material. The second material and the third material are each independently represented by Formula 3. Formula 1, Formula 2, and Formula 3 are described herein.

## Description

### BACKGROUND

The present disclosure herein relates to a light emitting element, a method of manufacturing a polycyclic compound present in the light emitting element, and an electronic device including the light emitting element.

An organic electroluminescent display device including an organic electroluminescent element is used as a display device present in many consumer and industrial electronic devices. An organic electroluminescent display device is a display device, at times, referred to as a self-luminous type light emitting element, that provides a display function by injecting holes and electrons from a first electrode and a second electrode, respectively, into a light emitting layer. The light emitting layer emits light from a light emitting material of the light emitting layer.

In the development of a light emitting element to a display device, improvements in light efficiency and lifespan (or device stability) are of importance, and therefore, the continuing development of materials for light emitting elements that provide such improvements are of interest.

### SUMMARY

An object of the present disclosure is to provide a method of manufacturing a polycyclic compound with improved yield.

Another object of the present disclosure is to provide a light emitting element having improved manufacturing process efficiency and an electronic device including the light emitting element.

A method of manufacturing a polycyclic compound represented by Formula 1 below according to an embodiment includes providing a first material represented by Formula 2 and adding a second material and a third material different from the second material to the first material, wherein the second material and the third material are each independently represented by Formula 3.

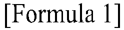

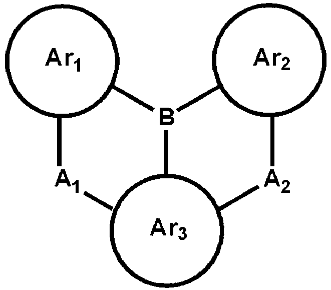

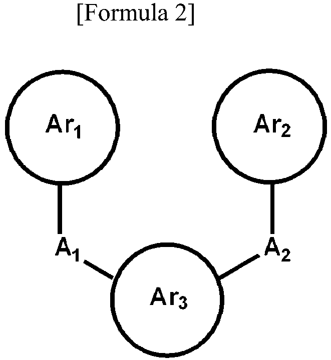

In Formulae 1 and 2,
Ar₁, Ar₂, and Ar₃ are each independently a substituted or unsubstituted hydrocarbon ring of 6 to 30 ring-forming carbon atoms, or a substituted or unsubstituted heterocyclic ring of 2 to 30 ring-forming carbon atoms, A₁ and A₂ are each independently O, S, Se or NRₓ, and Rₓ is a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ can join with an adjacent ring group to form a ring.

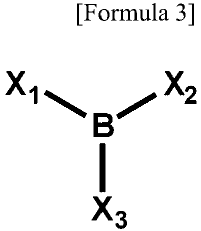

In Formula 3, X₁, X₂, and X₃ are each independently a hydrogen atom, a deuterium atom, a halogen atom, a hydroxyl group, or a substituted or unsubstituted alkyl group of 1 to 20 carbon atoms.

The polycyclic compound represented by Formula 1 may be represented by Formula 1-1, and the first material represented by Formula 2 may be represented by Formula 2-1.

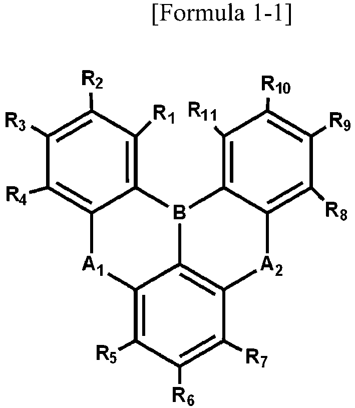

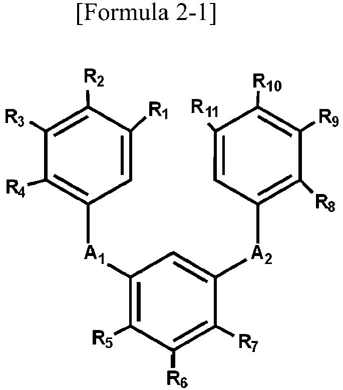

In Formula 1-1 and Formula 2-1,
R₁ to R₁₁ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or any one or more R₁ to R₁₁ can join with an adjacent group to form a ring, and A₁ and A₂ may be the same as defined in Formula 1.

The polycyclic compound represented by Formula 1 may be represented by Formula 1-2, and the first material represented by Formula 2 may be represented by Formula 2-2.

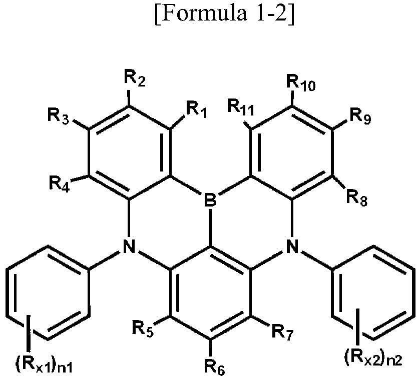

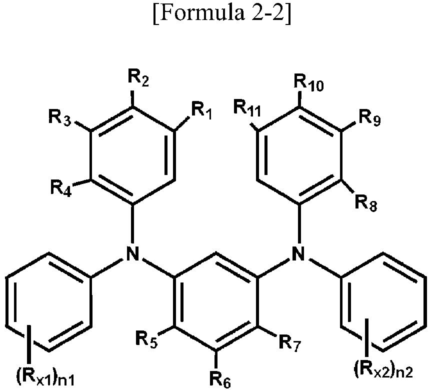

In Formula 1-2 and Formula 2-2,
Rₓ₁ and Rₓ₂ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₁ and Rₓ₂ can join with an adjacent group to form a ring. For example, Rₓ₁ and Rₓ₂ can join with an adjacent Rₓ₁ and Rₓ₂, respectively, to form a ring, or Rₓ₁ can join with R₅ to form a ring, or Rₓ₂ can join with R₇ to form a ring,
n1 and n2 are each independently an integer of 0 to 5, and R₁ to R₁₁ may be the same as defined in Formula 1-1.

The polycyclic compound represented by Formula 1 may be represented by Formula 1-3, and the first material represented by Formula 2 may be represented by Formula 2-3.

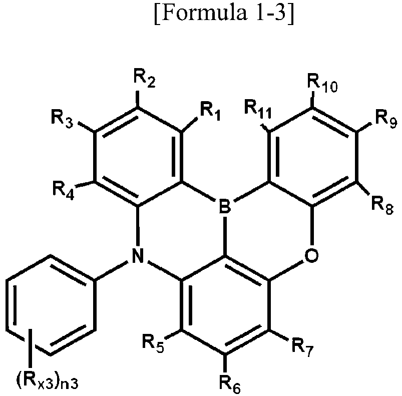

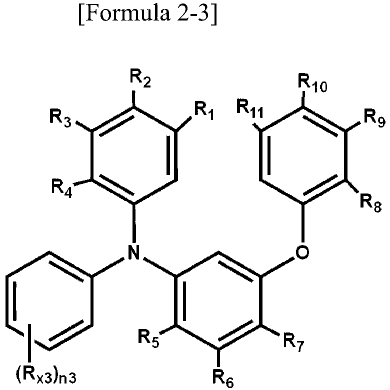

In Formula 1-3 and Formula 2-3,
Rₓ₃ is a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₃ can join with an adjacent Rₓ₃ to form a ring, or Rₓ₃ can join with R₄ or R₅ to form a ring,
n3 is an integer of 0 to 5, and R₁ to R₁₁ may be the same as defined in Formula 1-1.

The polycyclic compound represented by Formula 1 may be represented by Formula 1-4, and the first material represented by Formula 2 may be represented by Formula 2-4.

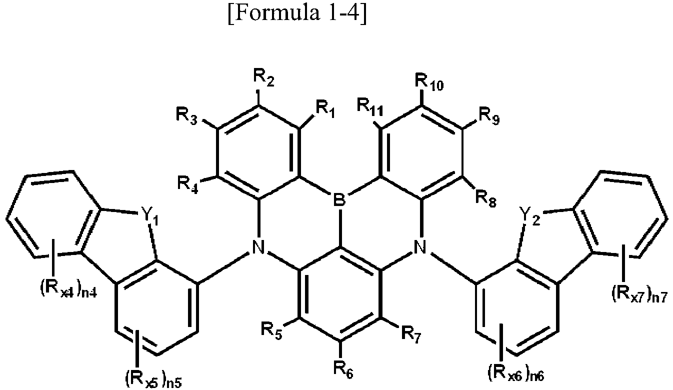

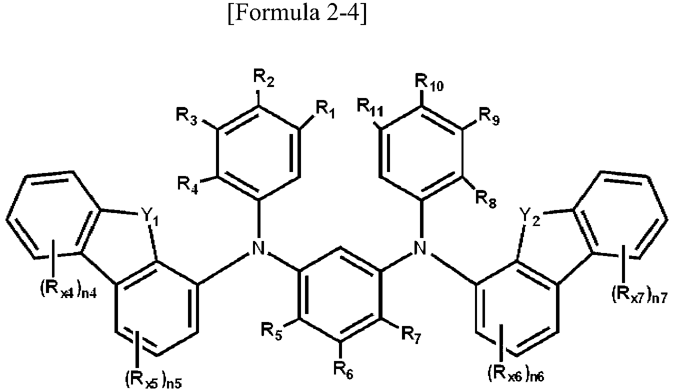

In Formula 1-4 and Formula 2-4,
Rₓ₄ to Rₓ₇ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or each Rₓ₄ to Rₓ₇ can join with an adjacent Rₓ₄ to Rₓ₇, respectively, to form a ring, or Rₓ₅ can join with R₅ to form a ring, or Rₓ₆ can join with an R₇ to form a ring,
Y₁ and Y₂ are each independently O or S,
n4 to n7 are each independently an integer of 0 to 5, and R₁ to R₁₁ may be the same as defined in Formula 1-1.

At least one hydrogen atom included in each of the polycyclic compound and the first material may be substituted with a deuterium atom.

In Formula 3, X₁, X₂, or X₃ may each independently be a fluorine atom, a chlorine atom, a bromine atom or an iodine atom.

The second material may include boron trichloride (BCl₃), and the third material may include boron tribromide (BBr₃).

The polycyclic compound represented by Formula 1 may include at least one of the compounds in Compound Group 1.

A light emitting element according to an embodiment includes a first electrode, a second electrode, and a light emitting layer disposed between the first electrode and the second electrode, wherein the light emitting layer includes a first compound represented by Formula 1. The first compound is derived from a light emitting layer composition, and the light emitting layer composition includes a first material represented by Formula 2, a second material represented by Formula 3, and a third material represented by Formula 3 and different from the second material.

The light emitting layer may further include at least one of a second compound represented by Formula HT-1, and a third compound represented by Formula ET-1.

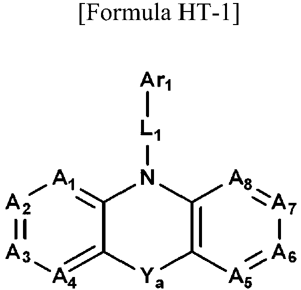

In Formula HT-1,
A₁ to A₈ are each independently N or CR₅₁, L₁ is a direct linkage, a substituted or unsubstituted arylene group of 6 to 30 ring-forming carbon atoms, or a substituted or unsubstituted heteroarylene group of 2 to 30 ring-forming carbon atoms,
Yₐ is a direct linkage, CR₅₂R₅₃, or SiR₅₄R₅₅, Ar₁ is a substituted or unsubstituted aryl group of 6 to 30 ring-forming carbon atoms, or a substituted or unsubstituted heteroaryl group of 2 to 30 ring-forming carbon atoms, and R₅₁ to R₅₅ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group of 1 to 20 carbon atoms, a substituted or unsubstituted alkenyl group of 2 to 20 carbon atoms, a substituted or unsubstituted aryl group of 6 to 60 ring-forming carbon atoms, or a substituted or unsubstituted heteroaryl group of 2 to 60 ring-forming carbon atoms, or any one of R₅₁ to R₅₅ can join with an adjacent group to form a ring.

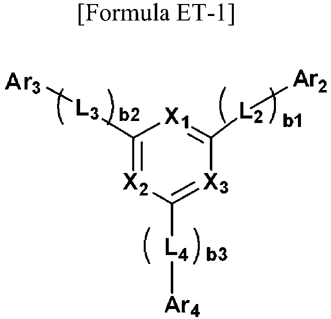

In Formula ET-1, at least one among X₁, X₂, and X₃ is N, and the remainder are CR₅₆, R₅₆ is a hydrogen atom, a deuterium atom, a substituted or unsubstituted alkyl group of 1 to 20 carbon atoms, a substituted or unsubstituted aryl group of 6 to 60 ring-forming carbon atoms, or a substituted or unsubstituted heteroaryl group of 2 to 60 ring-forming carbon atoms, b1, b₂, and b3 are each independently an integer of 0 to 10, Ar₂, Ar₃, and Ar₄ are each independently a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group or non-aromatic ring with 2 to 30 ring-forming carbon atoms, and L₂, L₃, and L₄ are each independently a direct linkage, a substituted or unsubstituted arylene group of 6 to 30 ring-forming carbon atoms, or a substituted or unsubstituted heteroarylene group of 2 to 30 ring-forming carbon atoms.

The light emitting layer may further include a fourth compound represented by Formula D-1 below.

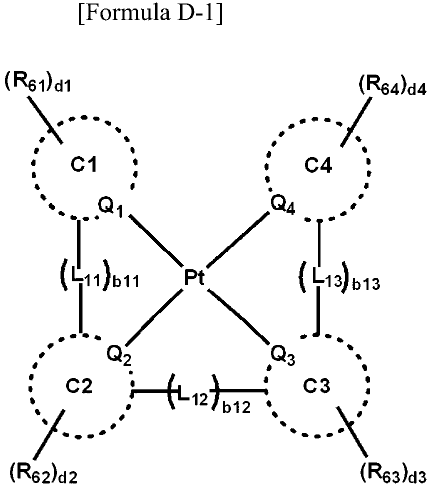

In Formula D-1,
Q₁ to Q₄ are each independently C or N,
C1 to C4 are each independently a substituted or unsubstituted hydrocarbon ring of 5 to 30 ring-forming carbon atoms, a substituted or unsubstituted aryl group having 6 to 30 ring-forming carbon atoms, or a substituted or unsubstituted heterocycle of 2 to 30 ring-forming carbon atoms,
L₁₁, L₁₂, and L₁₃ are each independently a direct linkage, 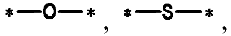 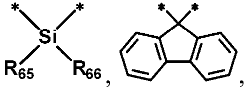 a substituted or unsubstituted divalent alkyl group of 1 to 20 carbon atoms, a substituted or unsubstituted arylene group of 6 to 30 ring-forming carbon atoms, or a substituted or unsubstituted heteroarylene group of 2 to 30 ring-forming carbon atoms, b11, b12, and b13 are each independently 0 or 1, R₆₁ to R₆₆ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group of 1 to 20 carbon atoms, a substituted or unsubstituted alkenyl group of 2 to 20 ring-forming carbon atoms, a substituted or unsubstituted aryl group of 6 to 60 ring-forming carbon atoms, or a substituted or unsubstituted heteroaryl group of 2 to 60 ring-forming carbon atoms, and
d1 to d4 are each independently an integer of 0 to 4.

The light emitting layer may emit delayed fluorescence.

The light emitting layer may emit blue light.

An electronic device according to an embodiment of the inventive concept includes a display module including a plurality of light emitting elements, wherein at least one of the plurality of light emitting elements includes a first electrode, a second electrode disposed on the first electrode, and a light emitting layer disposed between the first electrode and the second electrode and including the first compound represented by Formula 1, the first compound is derived from a light emitting layer composition, and the light emitting layer composition includes a first material represented by Formula 2, a second material represented by Formula 3, and a third material represented by Formula 3 and different from the second material.

The electronic device of an embodiment may further include at least one among a processor, a memory and a power module.

At least some of the above and other features of the invention are set out in the claims.

### BRIEF DESCRIPTION OF THE FIGURES

The accompanying drawings are included to provide a further understanding of the inventive concept and are incorporated in and constitute a part of this specification. The drawings illustrate embodiments of the inventive concept and, together with the description, serve to explain principles of the inventive concept. In the drawings:
FIG. 1 is a block diagram of an electronic device according to an embodiment;
FIG. 2 illustrates schematic diagrams of electronic devices according to embodiments;
FIG. 3 is a plan view showing a display module according to an embodiment;
FIG. 4 is a cross-sectional view showing a portion corresponding to line I-I' in FIG. 3;
FIG. 5 is a cross-sectional view schematically showing a light emitting element according to an embodiment;
FIG. 6 is a cross-sectional view schematically showing a light emitting element according to an embodiment;
FIG. 7 is a cross-sectional view schematically showing a light emitting element according to an embodiment;
FIG. 8 is a cross-sectional view schematically showing a light emitting element according to an embodiment;
FIG. 9 is a cross-sectional view schematically showing a light emitting element according to an embodiment;
FIG. 10 is a cross-sectional view showing a display module according to an embodiment;
FIG. 11 is a cross-sectional view showing a display module according to an embodiment;
FIG. 12 is a cross-sectional view showing a display module according to an embodiment;
FIG. 13 is a cross-sectional view showing a display module according to an embodiment;
FIG. 14 is a perspective view of an electronic device according to an embodiment;
FIG. 15 is a perspective view of an electronic device according to an embodiment; and
FIG. 16 is a diagram showing the interior of a vehicle in which an electronic device according to an embodiment is disposed.

### DETAILED DESCRIPTION

The invention now will be described more fully hereinafter with reference to the accompanying drawings, in which various embodiments are shown. This invention may, however, be embodied in many different forms, and should not be construed as limited to the embodiments set forth herein. Like reference numerals refer to like elements throughout.

Embodiments are described herein with reference to cross section illustrations that are schematic illustrations of embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

It will be understood that when an element (or a region, a layer, a portion, or the like) is referred to as being "on", "connected to" or "coupled to" another element, it may be directly disposed on, connected to, or coupled to the other element, or other elements may be disposed therebetween.

It will be understood that, although the terms "first", "second", etc. may be used herein to describe various elements, the elements are not to be limited by these terms. These terms are only used to distinguish one element, component, region, layer or section from another element, component, region, layer or section. For instance, a first element, component, region, layer or section discussed below could be termed a second element, component, region, layer or section without departing from the scope of the inventive concept. Similarly, a second element, component, region, layer or section could be termed a first element, component, region, layer or section.

The terminology used herein is for the purpose of describing particular embodiments only and is not intended to be limiting. As used herein, the singular forms "a," "an," and "the" are intended to include the plural forms, including "at least one," unless the content clearly indicates otherwise. Therefore, reference to "an" element in a claim followed by reference to "the" element is inclusive of one element as well as a plurality of the elements.

"At least one" is not to be construed as limiting "a" or "an." "Or" means "and/or." As used herein, the term "and/or" includes any and all combinations of one or more of the associated listed items.

It will be further understood that the terms "comprises, includes, has" and/or "comprising, including, having", when used in this specification, specify the presence of stated features, numbers, steps, operations, elements, components or combinations thereof, but do not preclude the possibility of the presence or addition of one or more other features, numbers, steps, operations, elements, components, and/or combinations thereof.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ±10% or ±5% of the stated value.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

In the specification, the term "substituted or unsubstituted" may mean substituted or unsubstituted with at least one substituent selected from the group consisting of a deuterium, a halogen, a cyano group, a nitro group, an amino group, a silyl group, an oxy group, a thio group, a sulfinyl group, a sulfonyl group, a carbonyl group, a boron group, a phosphine oxide group, a phosphine sulfide group, an alkyl group, an alkenyl group, an alkynyl group, a hydrocarbon ring group, an aryl group, and a heterocyclic group. In addition, each of the substituents exemplified above may be substituted or unsubstituted. For example, a biphenyl group may be interpreted as an aryl group or a phenyl group substituted with a phenyl group.

In the specification, the phrase "bonded to an adjacent group to form a ring" may mean that a group is bonded to an adjacent group to form a substituted or unsubstituted hydrocarbon ring, or a substituted or unsubstituted heterocycle. The hydrocarbon ring includes an aliphatic hydrocarbon ring and an aromatic hydrocarbon ring. The heterocycle includes an aliphatic heterocycle and an aromatic heterocycle. The hydrocarbon ring and the heterocycle may be monocyclic or polycyclic. In addition, the rings formed by being bonded to each other or may be connected to another adjacent ring or may join to form a spiro structure.

In the specification, the term "adjacent group" may mean a substituent substituted for an atom which is directly linked to an atom substituted with a corresponding substituent, another substituent substituted for an atom which is substituted with a corresponding substituent, or a substituent sterically positioned at the nearest position to a corresponding substituent. For example, two methyl groups in 1,2-dimethylbenzene may be interpreted as "adjacent groups" to each other and two ethyl groups in 1,1-diethylcyclopentane may be interpreted as "adjacent groups" to each other. In addition, two methyl groups in 4,5-dimethylphenanthrene may be interpreted as "adjacent groups" to each other.

In the specification, examples of the halogen atom may include a fluorine atom, a chlorine atom, a bromine atom, or an iodine atom.

In the specification, the alkyl group may be linear or branched. The number of carbons in the alkyl group is 1 to 50, 1 to 30, 1 to 20, 1 to 10, or 1 to 6. Examples of the alkyl group may include a methyl group, an ethyl group, an n-propyl group, an isopropyl group, an n-butyl group, an s-butyl group, a t-butyl group, an i-butyl group, a 2-ethylbutyl group, a 3,3-dimethylbutyl group, an n-pentyl group, an i-pentyl group, a neopentyl group, a t-pentyl group, a 1-methylpentyl group, a 3-methylpentyl group, a 2-ethylpentyl group, a 4-methyl-2-pentyl group, an n-hexyl group, a 1-methylhexyl group, a 2-ethylhexyl group, a 2-butylhexyl group, an n-heptyl group, a 1-methylheptyl group, a 2,2-dimethylheptyl group, a 2-ethylheptyl group, a 2-butylheptyl group, an n-octyl group, a t-octyl group, a 2-ethyloctyl group, a 2-butyloctyl group, a 2-hexyloctyl group, a 3,7-dimethyloctyl group, an n-nonyl group, an n-decyl group, an adamantyl group, a 2-ethyldecyl group, a 2-butyldecyl group, a 2-hexyldecyl group, a 2-octyldecyl group, an n-undecyl group, an n-dodecyl group, a 2-ethyldodecyl group, a 2-butyldodecyl group, a 2-hexyldocecyl group, a 2-octyldodecyl group, an n-tridecyl group, an n-tetradecyl group, an n-pentadecyl group, an n-hexadecyl group, a 2-ethylhexadecyl group, a 2-butylhexadecyl group, a 2-hexylhexadecyl group, a 2-octylhexadecyl group, an n-heptadecyl group, an n-octadecyl group, an n-nonadecyl group, an n-eicosyl group, a 2-ethyleicosyl group, a 2-butyleicosyl group, a 2-hexyleicosyl group, a 2-octyleicosyl group, an n-henicosyl group, an n-docosyl group, an n-tricosyl group, an n-tetracosyl group, an n-pentacosyl group, an n-hexacosyl group, an n-heptacosyl group, an n-octacosyl group, an n-nonacosyl group, an n-triacontyl group, etc., but the embodiments of the inventive concept are not limited thereto.

In the specification, a cycloalkyl group may mean a cyclic alkyl group. The number of carbons in the cycloalkyl group is 3 to 50, 3 to 30, 3 to 20, or 3 to 10. Examples of the cycloalkyl group may include a cyclopropyl group, a cyclobutyl group, a cyclopentyl group, a cyclohexyl group, a 4-methylcyclohexyl group, a 4-t-butylcyclohexyl group, a cycloheptyl group, a cyclooctyl group, a cyclononyl group, a cyclodecyl group, a norbornyl group, a 1-adamantyl group, a 2-adamantyl group, an isobornyl group, a bicycloheptyl group, etc., but the embodiments of the inventive concept are not limited thereto.

In the specification, an alkenyl group means a hydrocarbon group including at least one carbon double bond in the middle or terminal of an alkyl group having 2 or more carbon atoms. The alkenyl group may be linear or branched. The number of carbon atoms in the alkenyl group is not specifically limited, but is 2 to 30, 2 to 20, or 2 to 10. Examples of the alkenyl group include a vinyl group, a 1-butenyl group, a 1-pentenyl group, a 1,3-butadienyl group, a styrenyl group, a styryl vinyl group, *etc.,* but the embodiments of the inventive concept are not limited thereto.

In the specification, an alkynyl group means a hydrocarbon group including at least one carbon triple bond in the middle or terminal of an alkyl group having 2 or more carbon atoms. The alkynyl group may be linear or branched. Although the number of carbon atoms is not specifically limited, it is 2 to 30, 2 to 20, or 2 to 10. Specific examples of the alkynyl group may include an ethynyl group, a propynyl group, etc., but are not limited thereto.

In the specification, the hydrocarbon ring group means any functional group or substituent derived from an aliphatic hydrocarbon ring. The hydrocarbon ring group may be a saturated hydrocarbon ring group having 5 to 20 ring-forming carbon atoms.

In the specification, an aryl group means any functional group or substituent derived from an aromatic hydrocarbon ring. The aryl group may be a monocyclic aryl group or a polycyclic aryl group. The number of ring-forming carbon atoms in the aryl group may be 6 to 60, 6 to 30, 6 to 20, or 6 to 15. Examples of the aryl group may include a phenyl group, a naphthyl group, a fluorenyl group, an anthracenyl group, a phenanthryl group, a biphenyl group, a terphenyl group, a quaterphenyl group, a quinquephenyl group, a sexiphenyl group, a triphenylenyl group, a pyrenyl group, a benzofluoranthenyl group, a chrysenyl group, *etc.,* but the embodiments of the inventive concept are not limited thereto.

In the specification, the fluorenyl group may be substituted, and two substituents may be bonded to each other to form a spiro structure. Examples of the substituted fluorenyl group are as follows. However, the embodiments of the inventive concept are not limited thereto.

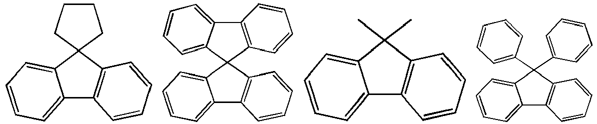

The heterocyclic group herein means any functional group or substituent derived from a ring containing at least one of B, O, N, P, Si, and Se as a heteroatom. The heterocyclic group includes an aliphatic heterocyclic group and an aromatic heterocyclic group. The aromatic heterocyclic group may be a heteroaryl group. The aliphatic heterocycle and the aromatic heterocycle may be monocyclic or polycyclic.

In the specification, the heterocyclic group may contain at least one of B, O, N, P, Si and S as a heteroatom. If the heterocyclic group contains two or more heteroatoms, the two or more heteroatoms may be the same as or different from each other. The heterocyclic group may be a monocyclic heterocyclic group or a polycyclic heterocyclic group, and includes a heteroaryl group. The number of ring-forming carbon atoms in the heterocyclic group may be 2 to 30, 2 to 20, or 2 to 10.

In the specification, the aliphatic heterocyclic group may include at least one of B, O, N, P, Si, and S as a heteroatom. The number of ring-forming carbon atoms in the aliphatic heterocyclic group may be 2 to 30, 2 to 20, or 2 to 10. Examples of the aliphatic heterocyclic group may include an oxirane group, a thiirane group, a pyrrolidine group, a piperidine group, a tetrahydrofuran group, a tetrahydrothiophene group, a thiane group, a tetrahydropyran group, a 1,4-dioxane group, *etc.,* but the embodiments of the inventive concept are not limited thereto.

In the specification, the heteroaryl group may contain at least one of B, O, N, P, Si, and S as a heteroatom. If the heteroaryl group contains two or more heteroatoms, the two or more heteroatoms may be the same as or different from each other. The heteroaryl group may be a monocyclic heterocyclic group or a polycyclic heterocyclic group. The number of ring-forming carbon atoms in the heteroaryl group may be 2 to 30, 2 to 20, or 2 to 10. Examples of the heteroaryl group may include a thiophene group, a furan group, a pyrrole group, an imidazole group, a pyridine group, a bipyridine group, a pyrimidine group, a triazine group, a triazole group, an acridyl group, a pyridazine group, a pyrazinyl group, a quinoline group, a quinazoline group, a quinoxaline group, a phenoxazine group, a phthalazine group, a pyrido pyrimidine group, a pyrido pyrazine group, a pyrazino pyrazine group, an isoquinoline group, an indole group, a carbazole group, an N-arylcarbazole group, an N-heteroarylcarbazole group, an N-alkylcarbazole group, a benzoxazole group, a benzoimidazole group, a benzothiazole group, a benzocarbazole group, a benzothiophene group, a dibenzothiophene group, a thienothiophene group, a benzofuran group, a phenanthroline group, a thiazole group, an isoxazole group, an oxazole group, an oxadiazole group, a thiadiazole group, a phenothiazine group, a dibenzosilole group, a dibenzofuran group, *etc.,* but the embodiments of the inventive concept are not limited thereto.

In the specification, the above description of the aryl group may be applied to an arylene group except that the arylene group is a divalent group. The above description of the heteroaryl group may be applied to a heteroarylene group except that the heteroarylene group is a divalent group.

In the specification, the silyl group includes an alkylsilyl group and an arylsilyl group. Examples of the silyl group may include a trimethylsilyl group, a triethylsilyl group, a t-butyldimethylsilyl group, a vinyldimethylsilyl group, a propyldimethylsilyl group, a triphenylsilyl group, a diphenylsilyl group, a phenylsilyl group, *etc.,* but the embodiments of the inventive concept are not limited thereto.

In the specification, the number of carbon atoms in the carbonyl group is not specifically limited, but may be 1 to 40, 1 to 30, or 1 to 20. For example, the carbonyl group may have the following structures, but the embodiments of the inventive concept are not limited thereto.

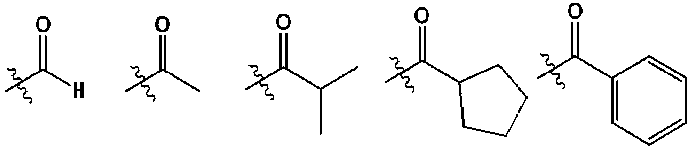

In the specification, the number of carbon atoms in the sulfinyl group and the sulfonyl group is not particularly limited, but may be 1 to 30. The sulfinyl group may include an alkyl sulfinyl group and an aryl sulfinyl group. The sulfonyl group may include an alkyl sulfonyl group and an aryl sulfonyl group.

In the specification, the thio group may include an alkylthio group and an arylthio group. The thio group may mean that a sulfur atom is bonded to the alkyl group or the aryl group as defined above. Examples of the thio group may include a methylthio group, an ethylthio group, a propylthio group, a pentylthio group, a hexylthio group, an octylthio group, a dodecylthio group, a cyclopentylthio group, a cyclohexylthio group, a phenylthio group, a naphthylthio group, but the embodiments of the inventive concept are not limited thereto.

In the specification, an oxy group may mean that an oxygen atom is bonded to the alkyl group or the aryl group as defined above. The oxy group may include an alkoxy group and an aryl oxy group. The alkoxy group may be a linear chain, a branched chain or a ring chain. The number of carbon atoms in the alkoxy group is not specifically limited, but may be, for example, 1 to 20 or 1 to 10. Examples of the oxy group may include methoxy, ethoxy, n-propoxy, isopropoxy, butoxy, pentyloxy, hexyloxy, octyloxy, nonyloxy, decyloxy, benzyloxy, *etc.,* but the embodiments of the inventive concept are not limited thereto.

The boron group herein may mean that a boron atom is bonded to the alkyl group or the aryl group as defined above. The boron group includes an alkyl boron group and an aryl boron group. Examples of the boron group may include a dimethylboron group, a trimethylboron group, a t-butyldimethylboron group, a diphenylboron group, a phenylboron group, etc., but the embodiments of the inventive concept are not limited thereto.

In the specification, the number of carbon atoms in an amine group is not specifically limited, but may be 1 to 30. The amine group may include an alkyl amine group and an aryl amine group. Examples of the amine group may include a methylamine group, a dimethylamine group, a phenylamine group, a diphenylamine group, a naphthylamine group, a 9-methyl-anthracenylamine group, *etc.,* but the embodiments of the inventive concept are not limited thereto.

In the specification, the alkyl group among an alkylthio group, an alkylsulfoxy group, an alkylaryl group, an alkylamino group, an alkyl boron group, an alkyl silyl group, and an alkyl amine group is the same as the examples of the alkyl group described above.

In the specification, the aryl group among an aryloxy group, an arylthio group, an arylsulfoxy group, an arylamino group, an arylboron group, an arylsilyl group, an arylamine group is the same as the examples of the aryl group described above.

In the specification, the phosphine oxide group may be substituted with, for example, at least one of the alkyl groups or aryl groups described above.

In the specification, the phosphine sulfide group may be substituted with, for example, at least one of the alkyl groups or aryl groups described above.

In the specification, a direct linkage may mean a single bond.

In the specification, 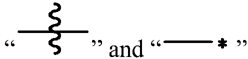 mean a position to be connected.

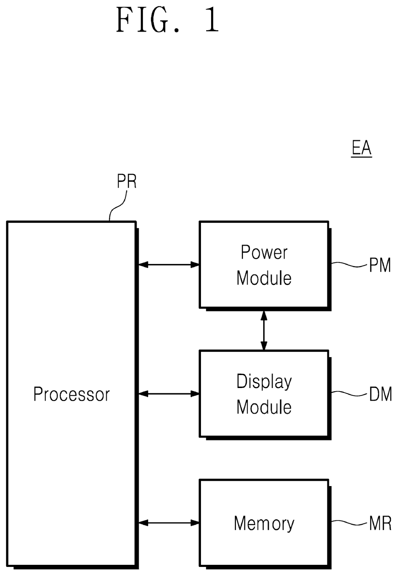

FIG. 1 is a block diagram of an electronic device according to an embodiment. Referring to FIG. 1, an electronic device EA according to an embodiment may include a display module DM, a processor PR, a memory MR, and a power module PM.

The processor PR may include at least one of a central processing unit (CPU), an application processor (AP), a graphic processing unit (GPU), a communication processor (CP), an image signal processor (ISP), and a controller.

The memory MR may store data information necessary for the operation of the processor PR or the display module DM. When the processor PR executes an application stored in the memory MR, an image data signal and/or an input control signal is transmitted to the display module DM, and the display module DM may process the received signal and output image information through a display screen. The display module DM may include a display panel that displays an image.

The power module PM may include a power supply module such as a power adapter or a battery device, and a power conversion module that converts power supplied by the power supply module to generate power required for the operation of the electronic device EA.

At least one of the components of the electronic device EA described above may be included in the display module according to embodiments described below and the display device of an embodiment including the same. In addition, some of individual modules functionally included in one module may be included in a display device, while others may be provided separately from the display device. For example, a display device may include a display module DM, and a processor PR, a memory MR, and a power module PM may be provided in the form of other devices within the electronic device EA other than the display device.

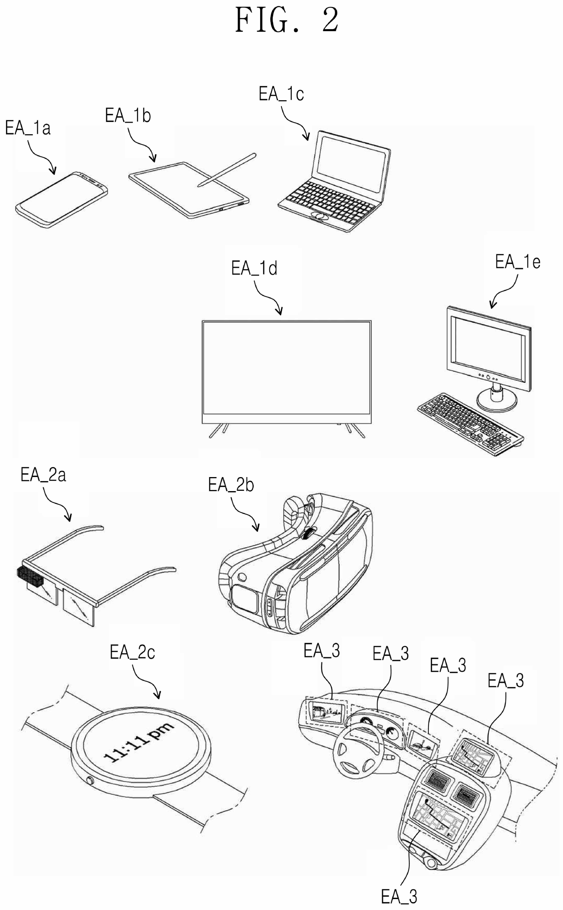

FIG. 2 illustrates schematic diagrams of embodiments of various electronic devices.

Referring to FIG. 2, various electronic devices including a display module according to an embodiment may include not only electronic devices for displaying images, such as a smart phone EA_1a, a tablet PC EA_1b, a laptop EA_1c, a TV EA_1d, and a desk monitor EA_1e, but also wearable electronic devices, such as smart glasses EA_2a, a head-mounted display EA_2b, and a smart watch EA_2c, and vehicle electronic devices EA_3, such as a dashboard of a car, center fascia, a Center Information Display (CID) disposed on a dashboard, and a room mirror display.

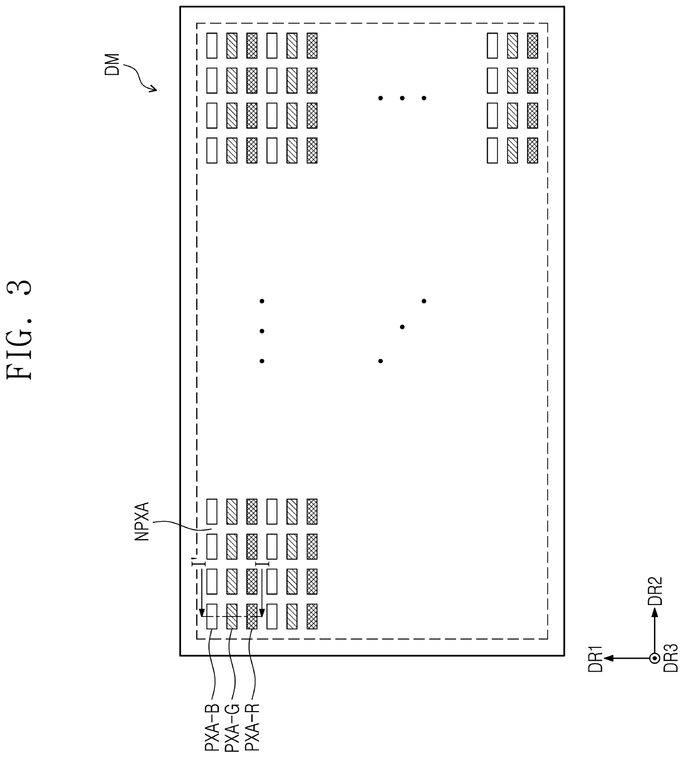

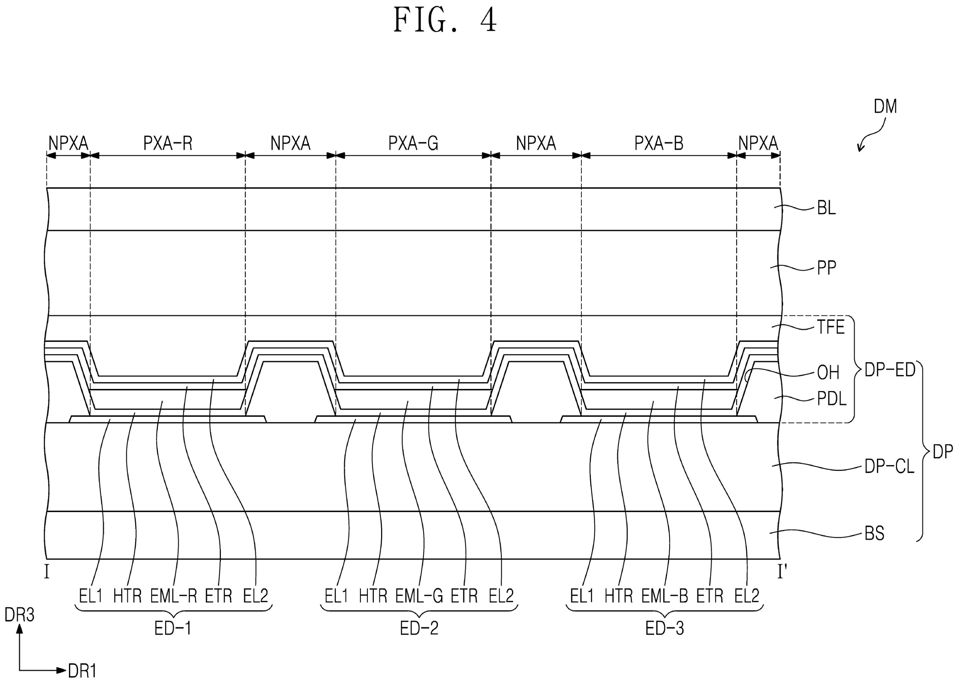

FIG. 3 is a plan view illustrating an embodiment of a display module DM. FIG. 4 is a cross-sectional view of the display module DM of the embodiment. FIG. 4 is a cross-sectional view illustrating a part taken along line I-I' of FIG. 1.

The display module DM may include a display panel DP and an optical layer PP disposed on the display panel DP. The display panel DP includes light emitting devices ED-1, ED-2, and ED-3. The display module DM may include a plurality of light emitting devices ED-1, ED-2, and ED-3. The optical layer PP may be disposed on the display panel DP to control reflected light in the display panel DP due to external light. The optical layer PP may include, for example, a polarization layer or a color filter layer. Unlike the configuration illustrated in the drawing, the optical layer PP may be omitted from the display module DM of an embodiment.

A base substrate BL may be disposed on the optical layer PP. The base substrate BL may be a member which provides a base surface on which the optical layer PP disposed. The base substrate BL may be a glass substrate, a metal substrate, a plastic substrate, *etc.* However, the embodiments of the inventive concept are not limited thereto, and the base substrate BL may be an inorganic layer, an organic layer, or a composite material layer. In addition, unlike the configuration illustrated, in an embodiment, the base substrate BL may be omitted.

The display module DM according to an embodiment may further include a filling layer (not shown). The filling layer (not shown) may be disposed between a display device layer DP-ED and the base substrate BL. The filling layer (not shown) may be an organic material layer. The filling layer (not shown) may include at least one of an acrylic-based resin, a silicone-based resin, and an epoxy-based resin.

The display panel DP may include a base layer BS, a circuit layer DP-CL provided on the base layer BS, and the display device layer DP-ED. The display device layer DP-ED may include a pixel defining film PDL, the light emitting devices ED-1, ED-2, and ED-3 disposed between portions of the pixel defining film PDL, and an encapsulation layer TFE disposed on the light emitting devices ED-1, ED-2, and ED-3.

The base layer BS may be a member which provides a base surface on which the circuit layer DP-CL is disposed. The base layer BS may be a glass substrate, a metal substrate, a plastic substrate, *etc.* However, the embodiments are not limited thereto, and the base layer BS may be an inorganic layer, an organic layer, or a composite material layer.

In an embodiment, the circuit layer DP-CL is disposed on the base layer BS, and the circuit layer DP-CL may include a plurality of transistors (not shown). Each of the transistors (not shown) may include a control electrode, an input electrode, and an output electrode. For example, the circuit layer DP-CL may include a switching transistor and a driving transistor for driving the light emitting devices ED-1, ED-2, and ED-3 of the display device layer DP-ED.

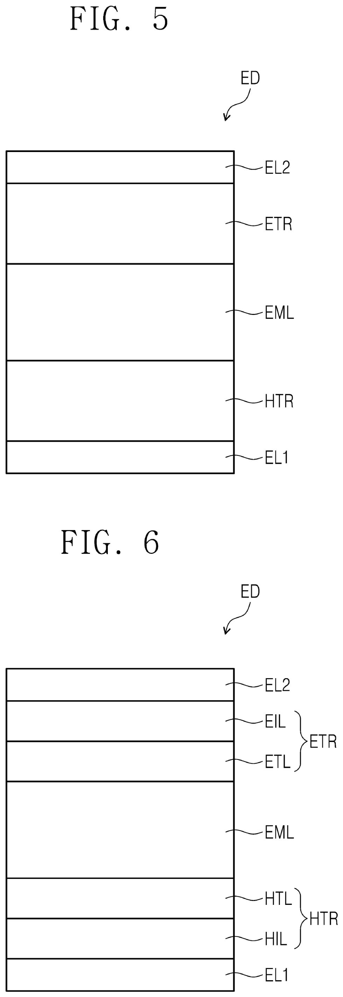

Each of the light emitting devices ED-1, ED-2, and ED-3 may have a structure of each light emitting device ED of embodiments according to FIGS. 5 to 8, which will be described later. Each of the light emitting devices ED-1, ED-2, and ED-3 may include a first electrode EL1, a hole transport region HTR, emission layers EML-R, EML-G, and EML-B, an electron transport region ETR, and a second electrode EL2.

FIG. 4 illustrates an embodiment in which the emission layers EML-R, EML-G, and EML-B of the light emitting devices ED-1, ED-2, and ED-3 are disposed in openings OH defined in the pixel defining film PDL, and the hole transport region HTR, the electron transport region ETR, and the second electrode EL2 are provided as a common layer in the entire light emitting devices ED-1, ED-2, and ED-3. However, the embodiments of the inventive concept are not limited thereto, and unlike the configuration illustrated in FIG. 2, the hole transport region HTR and the electron transport region ETR in an embodiment may be provided by being patterned inside the openings OH defined in the pixel defining film PDL. For example, the hole transport region HTR, the emission layers EML-R, EML-G, and EML-B, and the electron transport region ETR of the light emitting devices ED-1, ED-2, and ED-3 in an embodiment may be provided by being patterned in an inkjet printing method.

The encapsulation layer TFE may cover the light emitting devices ED-1, ED-2 and ED-3. The encapsulation layer TFE may seal the display device layer DP-ED. The encapsulation layer TFE may be a thin film encapsulation layer. The encapsulation layer TFE may be formed by laminating one layer or a plurality of layers. The encapsulation layer TFE includes at least one insulation layer. The encapsulation layer TFE according to an embodiment may include at least one inorganic film (hereinafter, an encapsulation-inorganic film). The encapsulation layer TFE according to an embodiment may also include at least one organic film (hereinafter, an encapsulation-organic film) and at least one encapsulation-inorganic film.

The encapsulation-inorganic film protects the display device layer DP-ED from moisture/oxygen, and the encapsulation-organic film protects the display device layer DP-ED from foreign substances such as dust particles. The encapsulation-inorganic film may include silicon nitride, silicon oxynitride, silicon oxide, titanium oxide, aluminium oxide, or the like, but the embodiment of the inventive concept is not particularly limited thereto. The encapsulation-organic film may include an acrylic-based compound, an epoxy-based compound, or the like. The encapsulation-organic film may include a photopolymerizable organic material, but the embodiment of the inventive concept is not particularly limited thereto.

The encapsulation layer TFE may be disposed on the second electrode EL2 and may be disposed filling the opening OH.

Referring to FIGS. 3 and 4, the display module DM may include a non-light emitting region NPXA and light emitting regions PXA-R, PXA-G, and PXA-B. The light emitting regions PXA-R, PXA-G, and PXA-B may be regions in which light generated by the respective light emitting devices ED-1, ED-2, and ED-3 is emitted. The light emitting regions PXA-R, PXA-G, and PXA-B may be spaced apart from each other on a plane.

Each of the light emitting regions PXA-R, PXA-G, and PXA-B may be a region divided by the pixel defining film PDL. The non-light emitting areas NPXA may be areas between the adjacent light emitting areas PXA-R, PXA-G, and PXA-B, which correspond to the pixel defining film PDL. In the specification, the light emitting regions PXA-R, PXA-G, and PXA-B may respectively correspond to pixels. The pixel defining film PDL may divide the light emitting devices ED-1, ED-2, and ED-3. The emission layers EML-R, EML-G, and EML-B of the light emitting devices ED-1, ED-2, and ED-3 may be disposed in openings OH defined in the pixel defining film PDL and separated from each other.

The light emitting regions PXA-R, PXA-G, and PXA-B may be divided into a plurality of groups according to the color of light generated from the light emitting devices ED-1, ED-2, and ED-3. In the display module DM of an embodiment illustrated in FIGS. 3 and 4, three light emitting regions PXA-R, PXA-G, and PXA-B, which emit red light, green light, and blue light, respectively, are exemplarily illustrated. For example, the display module DM of an embodiment may include the red light emitting region PXA-R, the green light emitting region PXA-G, and the blue light emitting region PXA-B that are separated from each other.

In the display module DM according to an embodiment, the plurality of light emitting devices ED-1, ED-2 and ED-3 may emit light beams having wavelengths different from each other. For example, in an embodiment, the display module DM may include a first light emitting device ED-1 that emits red light, a second light emitting device ED-2 that emits green light, and a third light emitting device ED-3 that emits blue light. That is, the red light emitting region PXA-R, the green light emitting region PXA-G, and the blue light emitting region PXA-B of the display apparatus may correspond to the first light emitting device ED-1, the second light emitting device ED-2, and the third light emitting device ED-3, respectively.

However, the embodiments of the inventive concept are not limited thereto, and the first to third light emitting devices ED-1, ED-2, and ED-3 may emit light beams in the same wavelength range or at least one light emitting device may emit a light beam in a wavelength range different from the others. For example, the first to third light emitting devices ED-1, ED-2, and ED-3 may all emit blue light.

The light emitting regions PXA-R, PXA-G, and PXA-B in the display module DM according to an embodiment may be arranged in a stripe form. Referring to FIG. 3, the plurality of red light emitting regions PXA-R, the plurality of green light emitting regions PXA-G, and the plurality of blue light emitting regions PXA-B each may be arranged along a second directional axis DR2. In addition, the red light emitting region PXA-R, the green light emitting region PXA-G, and the blue light emitting region PXA-B may be alternately arranged in this order along a first directional axis DR1.

FIGS. 3 and 4 illustrate that all the light emitting regions PXA-R, PXA-G, and PXA-B have similar area, but the embodiments of the inventive concept are not limited thereto. Thus, the light emitting regions PXA-R, PXA-G, and PXA-B may have different areas from each other according to the wavelength range of the emitted light. In this case, the areas of the light emitting regions PXA-R, PXA-G, and PXA-B may mean areas when viewed on a plane defined by the first directional axis DR1 and the second directional axis DR2.

An arrangement form of the light emitting regions PXA-R, PXA-G, and PXA-B is not limited to the configuration illustrated in FIG. 3, and the order in which the red light emitting region PXA-R, the green light emitting region PXA-G, and the blue light emitting region PXA-B are arranged may be provided in various combinations according to the characteristics of display quality required in the display module DM. For example, the arrangement form of the light emitting regions PXA-R, PXA-G, and PXA-B may be a pentile (PENTILE^{™}) arrangement form or a diamond (Diamond Pixel^{™}) arrangement form.

In addition, the areas of the light emitting regions PXA-R, PXA-G, and PXA-B may be different from each other. For example, in an embodiment, the area of the green light emitting region PXA-G may be smaller than that of the blue light emitting region PXA-B, but the embodiments of the inventive concept are not limited thereto.

Hereinafter, FIG. 5 to FIG. 9 are cross-sectional views schematically showing light emitting devices according to embodiments. The light emitting device ED of an embodiment may include a first electrode EL1, a hole transport region HTR, an emission layer EML, an electron transport region ETR, and a second electrode EL2 stacked in order.

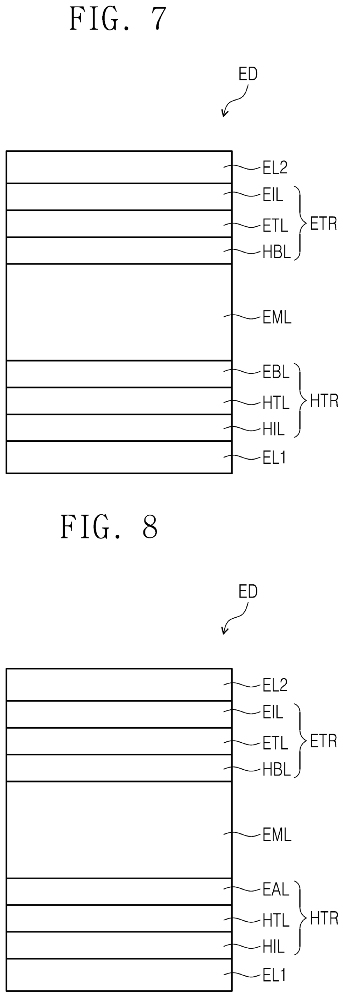

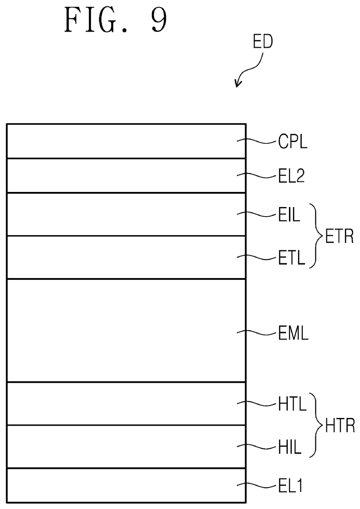

Compared with FIG. 5, FIG. 6 illustrates a cross-sectional view of a light emitting device ED of an embodiment, in which a hole transport region HTR includes a hole injection layer HIL and a hole transport layer HTL, and an electron transport region ETR includes an electron injection layer EIL and an electron transport layer ETL. In addition, compared with FIG. 5, FIG. 7 illustrates a cross-sectional view of a light emitting device ED of an embodiment, in which a hole transport region HTR includes a hole injection layer HIL, a hole transport layer HTL, and an electron blocking layer EBL, and an electron transport region ETR includes an electron injection layer EIL, an electron transport layer ETL, and a hole blocking layer HBL. When compared to FIG. 5, FIG. 8 shows the cross-sectional view of a light emitting element ED of an embodiment wherein a hole transport region HTR includes a hole injection layer HIL, a hole transport layer HTL and a light emitting auxiliary layer EAL, and an electron transport region ETR includes an electron injection layer EIL, an electron transport layer ETL and a hole blocking layer HBL. Compared with FIG. 6, FIG. 9 illustrates a cross-sectional view of a light emitting device ED of an embodiment including a capping layer CPL disposed on a second electrode EL2.

The first electrode EL1 has conductivity. The first electrode EL1 may be formed of a metal material, a metal alloy, or a conductive compound. The first electrode EL1 may be an anode or a cathode. However, the embodiments of the inventive concept are not limited thereto. In addition, the first electrode EL1 may be a pixel electrode. The first electrode EL1 may be a transmissive electrode, a transflective electrode, or a reflective electrode. The first electrode EL1 may include at least one selected from among Ag, Mg, Cu, Al, Pt, Pd, Au, Ni, Nd, Ir, Cr, Li, Ca, LiF, Mo, Ti, W, In, Sn, and Zn, a compound of two or more selected from among these, a mixture of two or more selected from among these, or an oxide thereof.

If the first electrode EL1 is the transmissive electrode, the first electrode EL1 may include a transparent metal oxide such as indium tin oxide (ITO), indium zinc oxide (IZO), zinc oxide (ZnO), or indium tin zinc oxide (ITZO). If the first electrode EL1 is the transflective electrode or the reflective electrode, the first electrode EL1 may include Ag, Mg, Cu, Al, Pt, Pd, Au, Ni, Nd, Ir, Cr, Li, Ca, LiF/Ca (a stacked structure of LiF and Ca), LiF/Al (a stacked structure of LiF and Al), Mo, Ti, W, a compound or mixture thereof (e.g., a mixture of Ag and Mg). Alternatively, the first electrode EL1 may have a multilayer structure including a reflective film or a transflective film formed of the above-described materials, and a transparent conductive film formed of ITO, IZO, ZnO, ITZO, *etc.* For example, the first electrode EL1 may have a three-layer structure of ITO/Ag/ITO, but the embodiments of the inventive concept are not limited thereto. In addition, the embodiments of the inventive concept are not limited thereto, and the first electrode EL1 may include the above-described metal materials, combinations of at least two metal materials of the above-described metal materials, oxides of the above-described metal materials, or the like. The thickness of the first electrode EL1 may be from about 700 Å to about 10,000 Å. For example, the thickness of the first electrode EL1 may be from about 1,000 Å to about 3,000 Å.

The hole transport region HTR is provided on the first electrode EL1. The hole transport region HTR may include at least one of a hole injection layer HIL, a hole transport layer HTL, a buffer layer or an emission-auxiliary layer (not shown), and an electron blocking layer. The thickness of the hole transport region HTR may be, for example, from about 50 Å to about 15,000 Å.

The hole transport region HTR may have a single layer formed of a single material, a single layer formed of a plurality of different materials, or a multilayer structure including a plurality of layers formed of a plurality of different materials.

For example, the hole transport region HTR may have a single layer structure of the hole injection layer HIL or the hole transport layer HTL, or may have a single layer structure formed of a hole injection material or a hole transport material. In addition, the hole transport region HTR may have a single layer structure formed of a plurality of different materials, or a structure in which a hole injection layer HIL/hole transport layer HTL, a hole injection layer HIL/hole transport layer HTL/buffer layer (not shown), a hole injection layer HIL/buffer layer (not shown), a hole transport layer HTL/buffer layer (not shown), or a hole injection layer HIL /hole transport layer HTL/electron blocking layer are stacked in order from the first electrode EL1, but the embodiments of the inventive concept are not limited thereto.

The hole transport region HTR may be formed using various methods such as a vacuum deposition method, a spin coating method, a cast method, a Langmuir-Blodgett (LB) method, an inkjet printing method, a laser printing method, and a laser induced thermal imaging (LITI) method.

The hole transport region HTR may include a compound represented by Formula H-1:

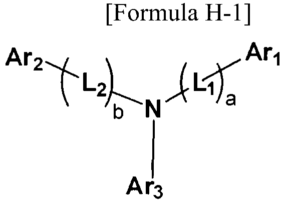

In Formula H-1, L₁ and L₂ may be each independently a direct linkage, a substituted or unsubstituted arylene group having 6 to 30 ring-forming carbon atoms, or a substituted or unsubstituted heteroarylene group having 2 to 30 ring-forming carbon atoms. a and b may be each independently an integer of 0 to 10. When a or b is an integer of 2 or greater, a plurality of L₁'s and L₂'s may be each independently a substituted or unsubstituted arylene group having 6 to 30 ring-forming carbon atoms, or a substituted or unsubstituted heteroarylene group having 2 to 30 ring-forming carbon atoms.

In Formula H-1, Ar₁ and Ar₂ may be each independently a substituted or unsubstituted aryl group having 6 to 30 ring-forming carbon atoms, or a substituted or unsubstituted heteroaryl group having 2 to 30 ring-forming carbon atoms. In addition, in Formula H-1, Ar₃ may be a substituted or unsubstituted aryl group having 6 to 30 ring-forming carbon atoms, or a substituted or unsubstituted heteroaryl group having 2 to 30 ring-forming carbon atoms.

The compound represented by Formula H-1 may be a monoamine compound. Alternatively, the compound represented by Formula H-1 may be a diamine compound in which at least one of Ar₁, Ar₂, and Ar₃ includes the amine group as a substituent. In addition, the compound represented by Formula H-1 may be a carbazole-based compound including a substituted or unsubstituted carbazole group in at least one of Ar₁ and Ar₂, or a fluorene-based compound including a substituted or unsubstituted fluorene group in at least one of Ar₁ and Ar₂.

The compound represented by Formula H-1 may be represented by any one among the compounds in Compound Group H. However, the compounds listed in Compound Group H below are examples, and the compounds represented by Formula H-1 are not limited to those represented by Compound Group H:

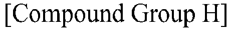

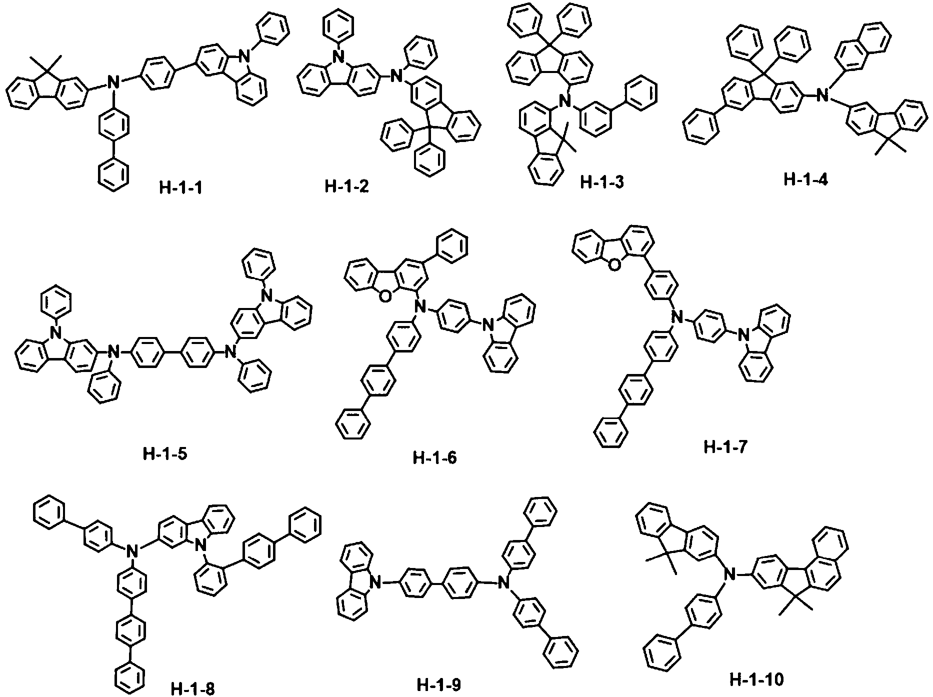

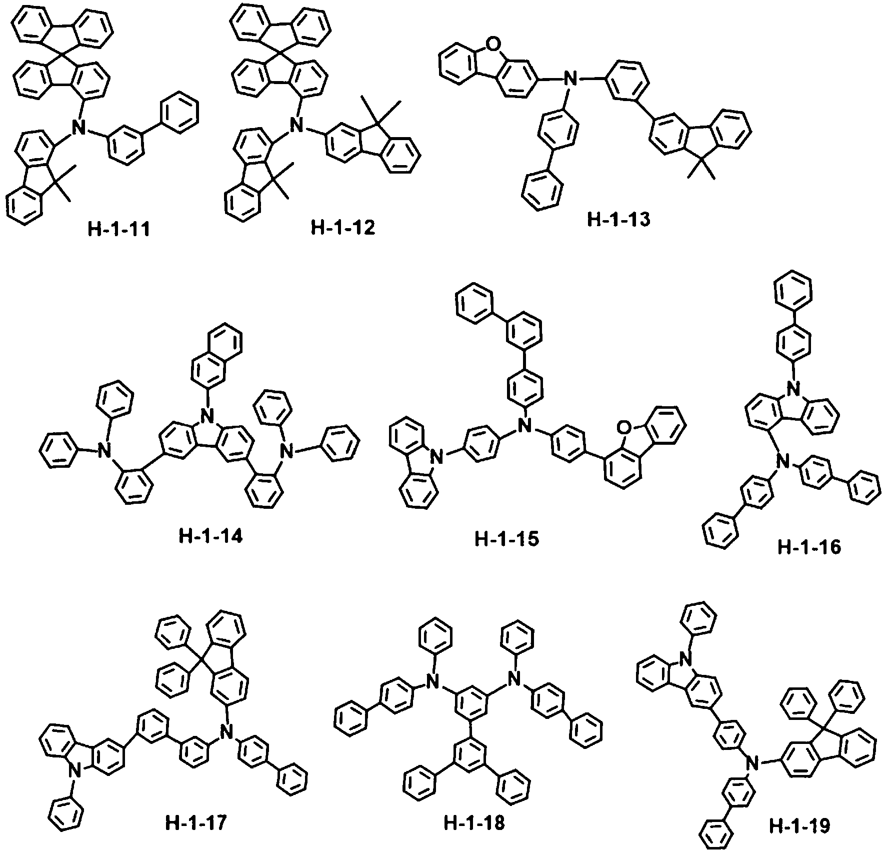

The hole transport region HTR may include a phthalocyanine compound such as copper phthalocyanine; N¹,N¹'-([1,1'-biphenyl]-4,4'-diyl)bis(N¹-phenyl-N⁴,N⁴-di-m-tolylbenzene-1,4-diamine) (DNTPD), 4,4',4"-[tris(3-methylphenyl)phenylamino] triphenylamine (m-MTDATA), 4,4',4"-tris(N,N-diphenylamino)triphenylamine (TDATA), 4,4',4"-tris[N(2-naphthyl)-N-phenylamino]-triphenylamine (2-TNATA), poly(3,4-ethylenedioxythiophene)/poly(4-styrenesulfonate) (PEDOT/PSS), polyaniline/dodecylbenzenesulfonic acid (PANI/DBSA), polyaniline/camphor sulfonic acid (PANI/CSA), polyaniline/poly(4-styrenesulfonate) (PANI/PSS), N,N'-di(naphthalene-1-yl)-N,N'-diphenyl-benzidine (NPB), triphenylamine-containing polyetherketone (TPAPEK), 4-isopropyl-4'-methyldiphenyliodonium [tetrakis(pentafluorophenyl)borate], dipyrazino[2,3-f: 2',3'-h]quinoxaline-2,3,6,7,10,11-hexacarbonitrile (HATCN), *etc.*

The hole transport region HTR may include a carbazole-based derivative such as N-phenyl carbazole or polyvinyl carbazole, a fluorene-based derivative, a triphenylamine-based derivative such as N,N'-bis(3-methylphenyl)-N,N'-diphenyl-[1,1'-biphenyl]-4,4'-diamine (TPD) or 4,4',4"-tris(N-carbazolyl)triphenylamine (TCTA), N,N'-di(naphthalene-1-yl)-N,N'-diphenyl-benzidine (NPB), 4,4'-cyclohexylidene bis[N,N-bis(4-methylphenyl)benzenamine] (TAPC), 4,4'-bis[N,N'-(3-tolyl)amino]-3,3'-dimethylbiphenyl (HMTPD), 1,3-bis(N-carbazolyl)benzene (mCP), *etc.*

In addition, the hole transport region HTR may include 9-(4-*tert-*butylphenyl)-3,6-bis(triphenylsilyl)-9H-carbazole (CzSi), 9-phenyl-9H-3,9'-bicarbazole (CCP), 1,3-bis(1,8-dimethyl-9H-carbazol-9-yl)benzene (mDCP), etc.

The hole transport region HTR may include the above-described compounds of the hole transport region HTR in at least one of a hole injection layer HIL, a hole transport layer HTL, and an electron blocking layer EBL.

The thickness of the hole transport region HTR may be from about 100 Å to about 10,000 Å, for example, from about 100 Å to about 5,000 Å. If the hole transport region HTR includes the hole injection layer HIL, the hole injection layer HIL may have, for example, a thickness of about 30 Å to about 1,000 Å. If the hole transport region HTR includes the hole transport layer HTL, the hole transport layer HTL may have a thickness of about 250 Å to about 1,000 Å. For example, if the hole transport region HTR includes the electron blocking layer EBL, the electron blocking layer EBL may have a thickness of about 10 Å to about 1,000 Å. If the thicknesses of the hole transport region HTR, the hole injection layer HIL, the hole transport layer HTL and the electron blocking layer EBL satisfy the above-described ranges, satisfactory hole transport properties may be achieved without a substantial increase in driving voltage.

The hole transport region HTR may further include a charge generating material to increase conductivity in addition to the above-described materials. The charge generating material may be dispersed uniformly or non-uniformly in the hole transport region HTR. The charge generating material may be, for example, a p-dopant. The p-dopant may include at least one of a halogenated metal compound, a quinone derivative, a metal oxide, and a cyano group-containing compound, but the embodiments of the inventive concept are not limited thereto. For example, the p-dopant may include a metal halide compound such as CuI or RbI, a quinone derivative such as tetracyanoquinodimethane (TCNQ) or 2,3,5,6-tetrafluoro-7,7,8,8-tetracyanoquinodimethane (F4-TCNQ), a metal oxide such as tungsten oxide or molybdenum oxide, a cyano group-containing compound such as dipyrazino[2,3-f: 2',3'-h] quinoxaline-2,3,6,7,10,11-hexacarbonitrile (HATCN) or 4-[[2,3-bis[cyano-(4-cyano-2,3,5,6-tetrafluorophenyl)methylidene]cyclopropylidene]-cyanomethyl]-2,3,5,6-tetrafluorobenzonitrile (NDP9), *etc.,* but the embodiments of the inventive concept are not limited thereto.

As described above, the hole transport region HTR may further include at least one of the buffer layer (not shown) and the electron blocking layer EBL in addition to the hole injection layer HIL and the hole transport layer HTL. The buffer layer (not shown) may compensate for a resonance distance according to the wavelength of light emitted from the emission layer EML and may thus increase light emission efficiency. A material that may be included in the hole transport region HTR may be used as a material to be included in the buffer layer (not shown). The electron blocking layer EBL is a layer that serves to prevent the electron injection from the electron transport region ETR to the hole transport region HTR.

In the light emitting element of an embodiment, the light emitting layer (also referred to herein as an emission layer) EML includes the polycyclic compound according to an embodiment. In the light emitting element of an embodiment, the light emitting layer EML may include a first compound, which is the polycyclic compound of an embodiment, and at least one compound among a second compound and a third compound. In addition, in the light emitting element of an embodiment, the light emitting layer EML may further include a fourth compound. The second compound may include a fused ring of three rings, including a nitrogen atom as a ring-forming atom. The third compound may include a hexagonal ring group including at least one nitrogen atom as a ring-forming atom. The fourth compound may include an organometallic complex. The second to fourth compounds will be described in more detail below.

In this specification, the first compound may be referred to as the polycyclic compound of an embodiment. The polycyclic compound of an embodiment may include a fused structure of a plurality of rings via a boron atom and two heteroatoms. Specifically, the polycyclic compound of an embodiment may include a fused structure of first to third rings via one boron atom, a first heteroatom and a second heteroatom. The first heteroatom and the second heteroatom may each independently be an oxygen atom, a sulfur atom, a selenium atom, or a nitrogen atom. The first to third rings may each be connected to the boron atom, the first ring and the third ring may be connected to each other via the first heteroatom, and the second ring and the third ring may be connected to each other via the second heteroatom. In this specification, the boron atom, the first heteroatom, the second heteroatom, and the fused structure of the first to third rings via the boron atom, the first heteroatom, and the second heteroatom, may be referred to as a "fused ring core." A light emitting element of an embodiment, including the polycyclic compound of an embodiment may exhibit high light emitting efficiency and long lifespan characteristics. The polycyclic compound of an embodiment may exhibit thermally-activated delayed fluorescence (TADF) light emitting characteristics due to reverse intersystem crossing.

The light emitting element ED of an embodiment includes the polycyclic compound of an embodiment. The polycyclic compound of an embodiment is represented by Formula 1.

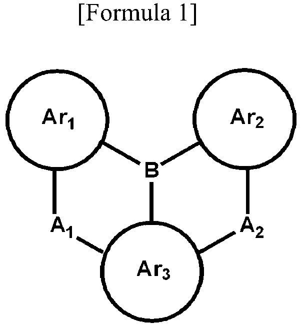

The polycyclic compound of an embodiment, represented by Formula 1 may include a fused structure of three rings via one boron atom, a first heteroatom, and a second heteroatom.

In Formula 1, Ar₁, Ar₂, and Ar₃ are each independently a substituted or unsubstituted hydrocarbon ring of 6 to 30 ring-forming carbon atoms, or a substituted or unsubstituted heterocycle of 2 to 30 ring-forming carbon atoms. For example, Ar₁, Ar₂, and Ar₃ may each independently be a substituted or unsubstituted benzene ring.

In Formula 1, A₁ and A₂ are each independently O, S, Se or NRₓ. For example, A₁ and A₂ may each independently be O, or NRₓ.

In Formula 1, Rₓ is a hydrogen, a deuterium, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring. For example, Rₓ may be a substituted or unsubstituted aryl group of 6 to 30 ring-forming carbon atoms, or a substituted or unsubstituted heteroaryl group of 2 to 30 ring-forming carbon atoms. Otherwise, Rₓ may be combined with an adjacent group to form a ring. For example, Rₓ may have a structure forming a ring through the combination with adjacent Ar₁, Ar₂ or Ar₃ from each other. Accordingly, Formula 1 containing Rₓ may provide a polycyclic fused ring including B and at least one among O, S, Se and N.

In an embodiment, at least one hydrogen included in the polycyclic compound of an embodiment, represented by Formula 1 may be substituted with a deuterium.

In this specification, Ar₁ and Ar₃ in Formula 1 may correspond to the above-described first to third rings, and A₁ and A₂ in Formula 1 may correspond to the above-described first heteroatom and second heteroatom.

In an embodiment, the polycyclic compound represented by Formula 1 may be represented by Formula 1-1.

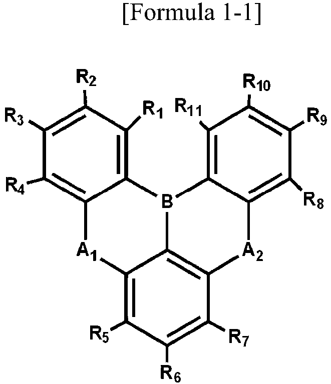

Formula 1-1 represents Formula 1 where the types of substituents represented by Ar₁, Ar₂, and Ar₃ are specified.

In Formula 1-1, R₁ to R₁₁ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, R₁ to R₁₁ may each independently be a deuterium, a halogen, a substituted or unsubstituted oxy group, a substituted or unsubstituted alkyl group of 1 to 20 carbon atoms, or a substituted or unsubstituted aryl group of 6 to 30 ring-forming carbon atoms. Otherwise, R₁ to R₁₁ may each independently be combined with an adjacent group to form a ring. For example, R₄ and R₈ may each independently be combined with Rₓ from to form a ring.

In Formula 1-1, the same contents explained in Formula 1 may be applied to A₁ and A₂.

In an embodiment, the polycyclic compound represented by Formula 1 may be represented by one of Formula 1-2, Formula 1-3, or Formula 1-4 below.

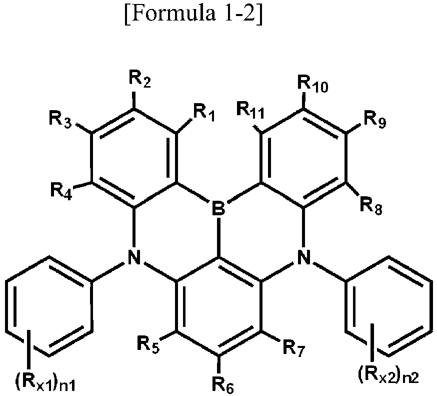

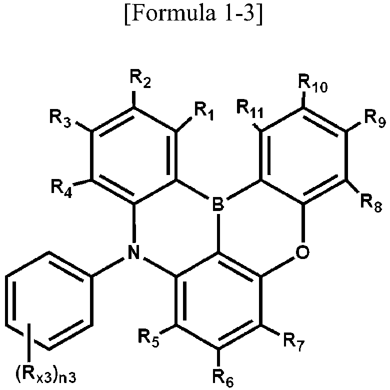

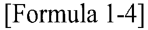

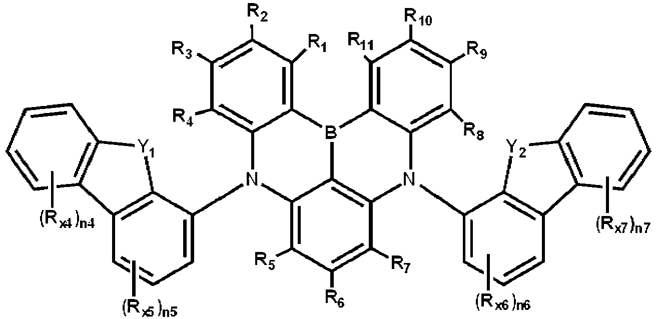

Formula 1-2, Formula 1-3, and Formula 1-4 represent Formula 1-1 where the types of the substituents represented by A₁ and A₂ are specified.

In Formula 1-2, Formula 1-3, and Formula 1-4, Rₓ₁ to Rₓ₇ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. In Formula 1-4, Y₁ and Y₂ are each independently O or S.

For example, Rₓ₁, Rₓ₂, and Rₓ₃ may each independently be a hydrogen atom, a deuterium atom, a halogen atom, a substituted or unsubstituted oxy group, a substituted or unsubstituted thio group, a substituted or unsubstituted alkyl group of 1 to 20 carbon atoms, or a substituted or unsubstituted aryl group of 6 to 30 ring-forming carbon atoms. For example, Rₓ₄ to Rₓ₇ may each independently be a hydrogen atom, a deuterium atom, a substituted or unsubstituted alkyl group of 1 to 20 carbon atoms, or a substituted or unsubstituted aryl group of 6 to 30 ring-forming carbon atoms. Otherwise, Rₓ₁ to Rₓ₇ may each independently be combined with an adjacent group to form a ring. For example, Rₓ₁ to Rₓ₃ may each independently be combined with an adjacent group to form a ring.

In Formula 1-2, Formula 1-3, and Formula 1-4, n1 to n7 are each independently an integer of 0 to 5. If each of n1 to n7 is 0, the polycyclic compound of an embodiment may not be substituted with each of Rₓ₁ to Rₓ₇. A case where each of n1 to n7 is 5, and each of Rₓ₁ to Rₓ₇ is a hydrogen atom, may be the same as a case where each of n1 to n7 is 0. If n1 to n7 are integers of 2 or more, each of Rₓ₁ to Rₓ₇ provided in plurality may be all the same, or at least one of the plurality of Rₓ₁ to Rₓ₇ may be different.

In Formula 1-2, Formula 1-3, and Formula 1-4, the same contents explained in Formula 1-1 may be applied to R₁ to R₁₁.

In an embodiment, the polycyclic compound represented by Formula 1 may be represented by Formula 1-A or Formula 1-B.

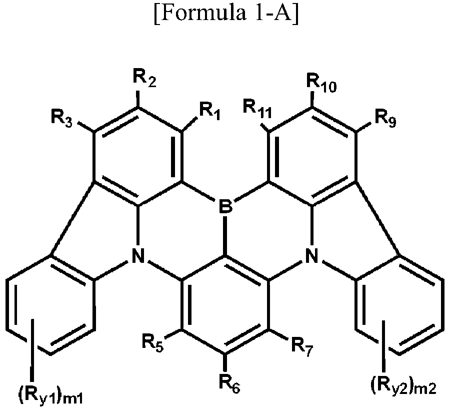

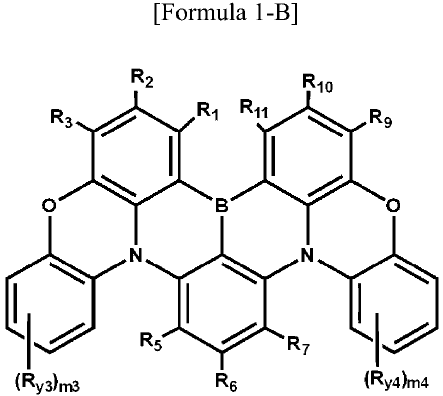

Formula 1-A and Formula 1-B represent Formula 1-2 where the types of the substituents represented by R₄, R₈, Rₓ₁, and Rₓ₂ are specified.

In Formula 1-A and Formula 1-B, R_{y1} to R_{y4} are each independently a hydrogen, a deuterium, a halogen, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, R_{y1} to R_{y4} may each independently be a hydrogen or a deuterium. In Formula 1-A and Formula 1-B, m1 to m4 are each independently an integer of 0 to 4.

The polycyclic compound of an embodiment may be represented by one among the compounds in Compound Group 1. A light emitting element ED according to an embodiment may include at least one of the compounds in Compound Group 1. In Compound Group 1, D is a deuterium.

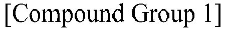

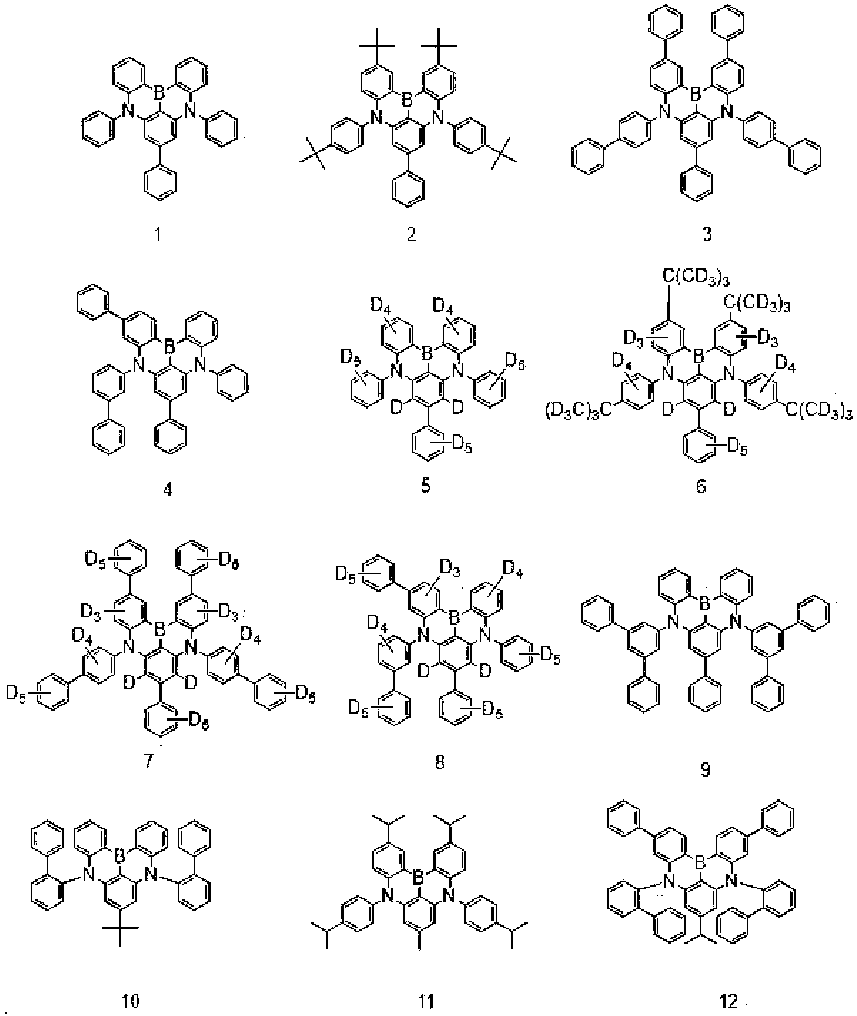

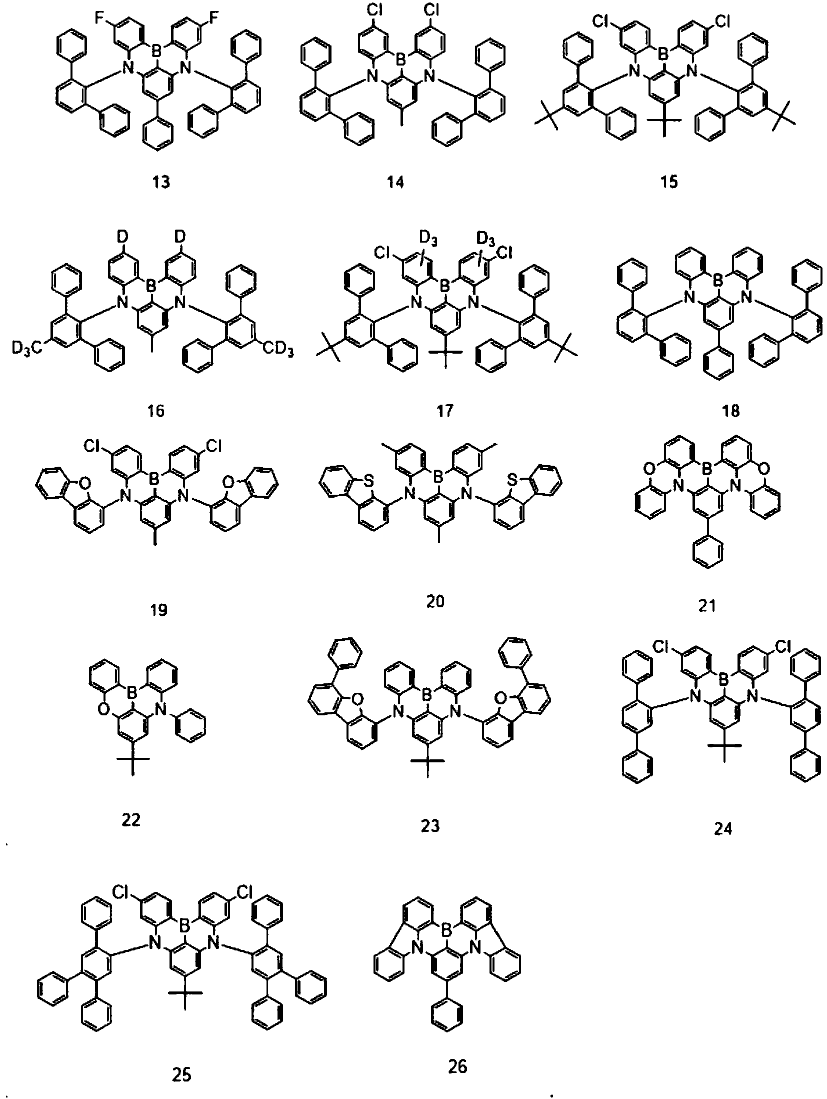

The polycyclic compound of an embodiment is derived from a light emitting layer composition including a first material, a second material, and a third material. A method of manufacturing the polycyclic compound of an embodiment includes providing a first material and adding a second material and a third material to the first material.

The first material of an embodiment is represented by Formula 2 below. In this specification, the first material may be referred to as a reactant.

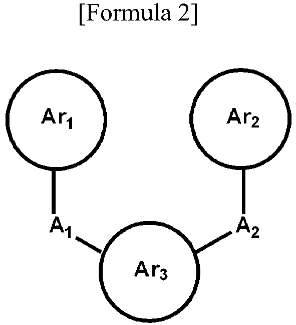

In Formula 2, Ar₁ to Ar₃ are each independently a substituted or unsubstituted hydrocarbon ring of 6 to 30 ring-forming carbon atoms, or a substituted or unsubstituted heterocycle of 2 to 30 ring-forming carbon atoms. For example, Ar₁ to Ar₃ may each independently be a substituted or unsubstituted benzene ring.

In Formula 2, A₁ and A₂ are each independently O, S, Se or NRₓ. For example, A₁ and A₂ may each independently be O, or NRₓ.

In Formula 2, Rₓ is a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or combined with an adjacent group to form a ring. For example, Rₓ may be a substituted or unsubstituted aryl group of 6 to 30 ring-forming carbon atoms, or a substituted or unsubstituted heteroaryl group of 2 to 30 ring-forming carbon atoms. Otherwise, Rₓ may be combined with an adjacent group to form a ring. For example, Rₓ may be combined with an adjacent Ar₁, Ar₂ or Ar₃ to form a ring.

In an embodiment, at least one hydrogen atom included in the first material of an embodiment, represented by Formula 2 may be substituted with a deuterium atom.

In an embodiment, the first material represented by Formula 2 may be represented by Formula 2-1.

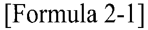

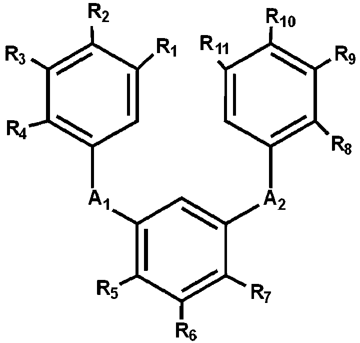

Formula 2-1 represents Formula 2 where the types of the substituents represented by Ar₁ to Ar₃ are specified.

In Formula 2-1, R₁ to R₁₁ are each independently a hydrogen, a deuterium, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, R₁ to R₁₁ may each independently be a deuterium, a halogen, a substituted or unsubstituted oxy group, a substituted or unsubstituted alkyl group of 1 to 20 carbon atoms, or a substituted or unsubstituted aryl group of 6 to 30 ring-forming carbon atoms. Otherwise, R₁ to R₁₁ may each independently be joined to an adjacent group to form a ring. For example, R₄ and R₈ may each independently be joined to an adjacent Rₓ from each other to form a ring.

In Formula 2-1, the same contents explained in Formula 2 may be applied to A₁ and A₂.

In an embodiment, the first material represented by Formula 2 may be represented by one among Formula 2-2 to Formula 2-4.

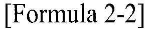

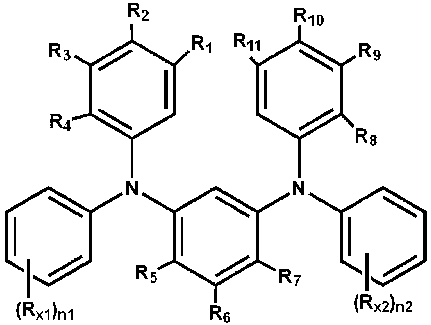

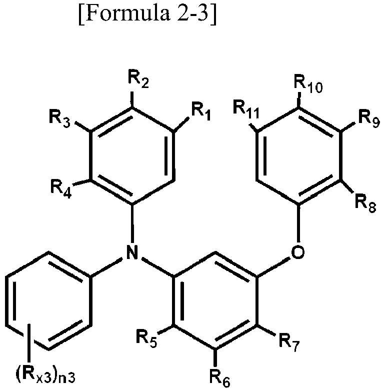

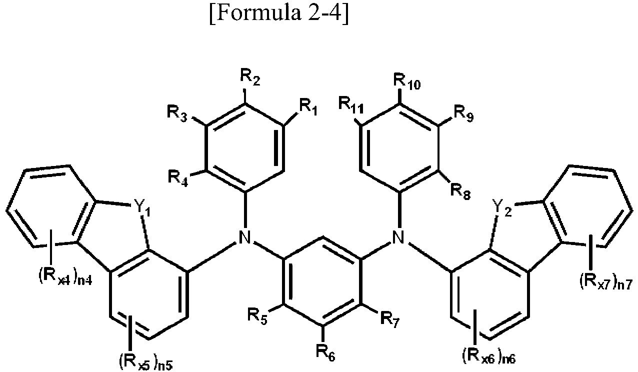

Formula 2-2, Formula 2-3, and Formula 2-4 represent Formula 2-1 where the types of the substituents represented by A₁ and A₂ are specified.

In Formula 2-2, Formula 2-3, and Formula 2-4, Rₓ₁ to Rₓ₇ are each independently a hydrogen, a deuterium, a halogen, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, Rₓ₁, Rₓ₂, and Rₓ₃ may each independently be a hydrogen atom, a deuterium atom, a substituted or unsubstituted oxy group, a substituted or unsubstituted thio group, a substituted or unsubstituted alkyl group of 1 to 20 carbon atoms, or a substituted or unsubstituted aryl group of 6 to 30 ring-forming carbon atoms. For example, Rₓ₄ to Rₓ₇ may each independently be a hydrogen, a deuterium, a substituted or unsubstituted alkyl group of 1 to 20 carbon atoms, or a substituted or unsubstituted aryl group of 6 to 30 ring-forming carbon atoms. Otherwise, Rₓ₁ to Rₓ₇ may each independently be combined with an adjacent group to form a ring. For example, Rₓ₁, Rₓ₂, and Rₓ₃ may each independently be combined with an adjacent group to form a ring. In Formula 2-4, Y₁ and Y₂ are each independently O or S.

In Formula 2-2, Formula 2-3, and Formula 2-4, n1 to n7 are each independently an integer of 0 to 5. If each of n1 to n7 is 0, the first material of an embodiment may not be substituted with each of Rₓ₁ to Rₓ₇. A case where each of n1 to n7 is 5, and each of Rₓ₁ to Rₓ₇ is a hydrogen atom, may be the same as a case where each of n1 to n7 is 0. If n1 to n7 are integers of 2 or more, each of Rₓ₁ to Rₓ₇ provided in plurality may be all the same, or at least one of the plurality of Rₓ₁ to Rₓ₇ may be different.

In Formula 2-2, Formula 2-3, and Formula 2-4, the same contents explained in Formula 2-1 may be applied to R₁ to R₁₁.

In an embodiment, the first material represented by Formula 2 may be represented by Formula 2-A or Formula 2-B.

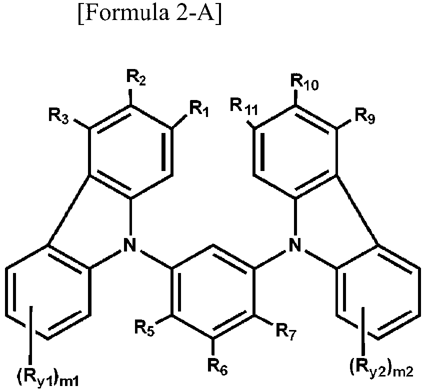

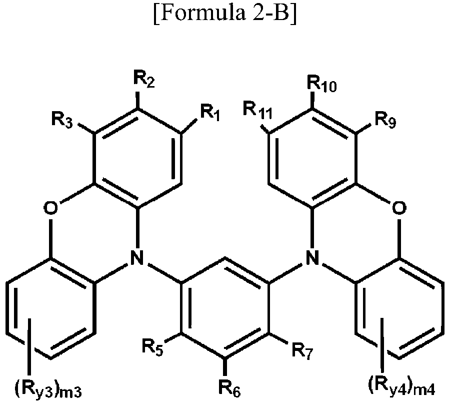

Formula 2-A and Formula 2-B represent Formula 2-2 where the types of the substituents represented by R₄, R₈, Rₓ₁, and Rₓ₂ are specified.

In Formula 2-A and Formula 2-B, R_{y1} to R_{y4} are each independently a hydrogen, a deuterium, a halogen, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms. For example, R_{y1} to R_{y4} may each independently be a hydrogen or a deuterium. In Formula 2-A and Formula 2-B, m1 to m4 are each independently an integer of 0 to 4.

The second material and the third material of an embodiment may each independently be represented by Formula 3 below.

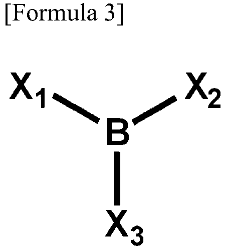

In Formula 3, X₁, X₂, and X₃ are each independently a hydrogen, a deuterium, a halogen, a hydroxyl group, or a substituted or unsubstituted alkyl group of 1 to 20 carbon atoms. For example, X₁, X₂, and X₃ may all be halogen. X₁, X₂, and X₃ may each independently be a fluorine, a chlorine, a bromine or an iodine. X₁, X₂, and X₃ may be all identical. For example, X₁, X₂, and X₃ may be all fluorine, X₁, X₂, and X₃ may be all chlorine, X₁, X₂, and X₃ may be all bromine, or X₁, X₂, and X₃ may be all iodine.

The second material and the third material are different materials. The second material represented by Formula 3 may be represented by BY₃, and the third material represented by Formula 3 may be represented by BZ₃. In BY₃ and BZ₃, Y and Z may each independently be a fluorine atom, a chlorine atom, a bromine atom, or an iodine atom. In this case, Y and Z may be different from each other. For example, the second material may include boron trichloride (BCl₃), and the third material may include boron tribromide (BBr₃). The method of manufacturing the polycyclic compound according to an embodiment of the inventive concept may increase process efficiency by including the second material and the third material, which are different materials.

The polycyclic compound of an embodiment may be used as a delayed fluorescence material.

For example, the polycyclic compound of an embodiment may be used as a thermally activated delayed fluorescence (TADF) material. TADF uses reverse intersystem crossing (RISC) to enable efficient harvesting of triplet excitons, a process whereby triplet excitons, which would otherwise be wasted as non-emissive states, are upconverted to singlets, thus contributing to delayed fluorescence. This process is thermally driven because the difference in energy between the lowest singlet (S₁) and triplet (T₁) states is small, which facilitates RISC. Donor-Acceptor TADF molecules are a class of TADF molecules having a relatively high capacity for efficient exciton harvesting through RISC. The molecules consist of distinct electron-donating (D) and electron-accepting (A) moieties connected by a conjugated linker, which enables spatial separation of the HOMO (localized on the donor) and LUMO (localized on the acceptor). This separation minimizes the singlet-triplet energy gap (ΔE_{ST}), promoting effective RISC and delayed fluorescence.

A light emitting element including the polycyclic compound according to an embodiment may exhibit high efficiency and long-life characteristics. A light emitting element according to an embodiment, including a polycyclic compound according to an embodiment, having excellent light emitting efficiency and improved material stability in a light emitting layer may exhibit high light emitting efficiency and excellent lifespan characteristics. The polycyclic compound of an embodiment may exhibit high light emitting efficiency and excellent lifespan characteristics in a blue light wavelength range.

In the light emitting element according to an embodiment, the light emitting layer EML may be a delayed fluorescence emitting layer including a host and a dopant. More specifically, the light emitting layer EML may emit thermally activated delayed fluorescence. The polycyclic compound according to an embodiment may be a thermally activated delayed fluorescence dopant.

The light emitting layer EML may include the polycyclic compound according to an embodiment as a dopant. The polycyclic compound of an embodiment may emit blue light. For example, the polycyclic compound of an embodiment may be a light emitting material having a maximum light emitting wavelength in a wavelength range of about 430 nm to about 490 nm. Specifically, the polycyclic compound of an embodiment may be a light emitting material having a maximum light emitting wavelength in a wavelength range of about 440 nm to about 470 nm.

In an embodiment, the light emitting layer EML may include the polycyclic compound of an embodiment and at least one among the second to fourth compounds.

In an embodiment, the emission layer EML may include the second compound represented by Formula HT-1 below. In an embodiment, the second compound may be used as a hole transporting host material of the emission layer EML.

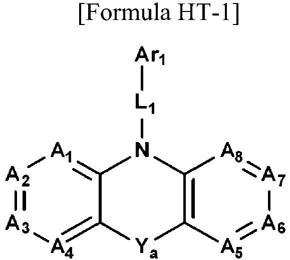

In Formula HT-1, A₁ to A₈ may be each independently N or CR₅₁. For example, all of A₁ to A₈ may be CR₅₁. Alternatively, any one of A₁ to A₈ may be N, and the rest may be CR₅₁.

In Formula HT-1, L₁ may be a direct linkage, a substituted or unsubstituted arylene group having 6 to 30 ring-forming carbon atoms, or a substituted or unsubstituted heteroarylene group having 2 to 30 ring-forming carbon atoms. For example, L₁ may be a direct linkage, a substituted or unsubstituted phenylene group, a substituted or unsubstituted divalent biphenyl group, a substituted or unsubstituted divalent carbazole group, etc., but the embodiments of the inventive concept are not limited thereto.

In Formula HT-1, Yₐ may be a direct linkage, CR₅₂R₅₃, or SiR₅₄R₅₅. That is, it may mean that the two benzene rings linked to the nitrogen atom in Formula HT-1 are linked via a direct linkage, 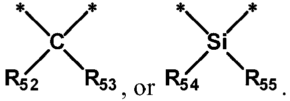 In Formula HT-1, when Yₐ is a direct linkage, the substituent represented by Formula HT-1 may include a carbazole moiety.

In Formula HT-1, Ar₁ may be a substituted or unsubstituted aryl group having 6 to 30 ring-forming carbon atoms, or a substituted or unsubstituted heteroaryl group having 2 to 30 ring-forming carbon atoms. For example, Ar₁ may be a substituted or unsubstituted carbazole group, a substituted or unsubstituted dibenzofuran group, a substituted or unsubstituted dibenzothiophene group, a substituted or unsubstituted biphenyl group, *etc.,* but the embodiments of the inventive concept are not limited thereto.

In Formula HT-1, R₅₁ to R₅₅ may be each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms. Alternatively, each of R₅₁ to R₅₅ may be bonded to an adjacent group to form a ring. For example, R₅₁ to R₅₅ may be each independently a hydrogen atom or a deuterium atom. R₅₁ to R₅₅ may be each independently an unsubstituted methyl group or an unsubstituted phenyl group.

In an embodiment, the second compound represented by Formula HT-1 may be represented by any one of the compounds represented by Compound Group 2. The emission layer EML may include at least one among the compounds represented by Compound Group 2 as a hole transporting host material.

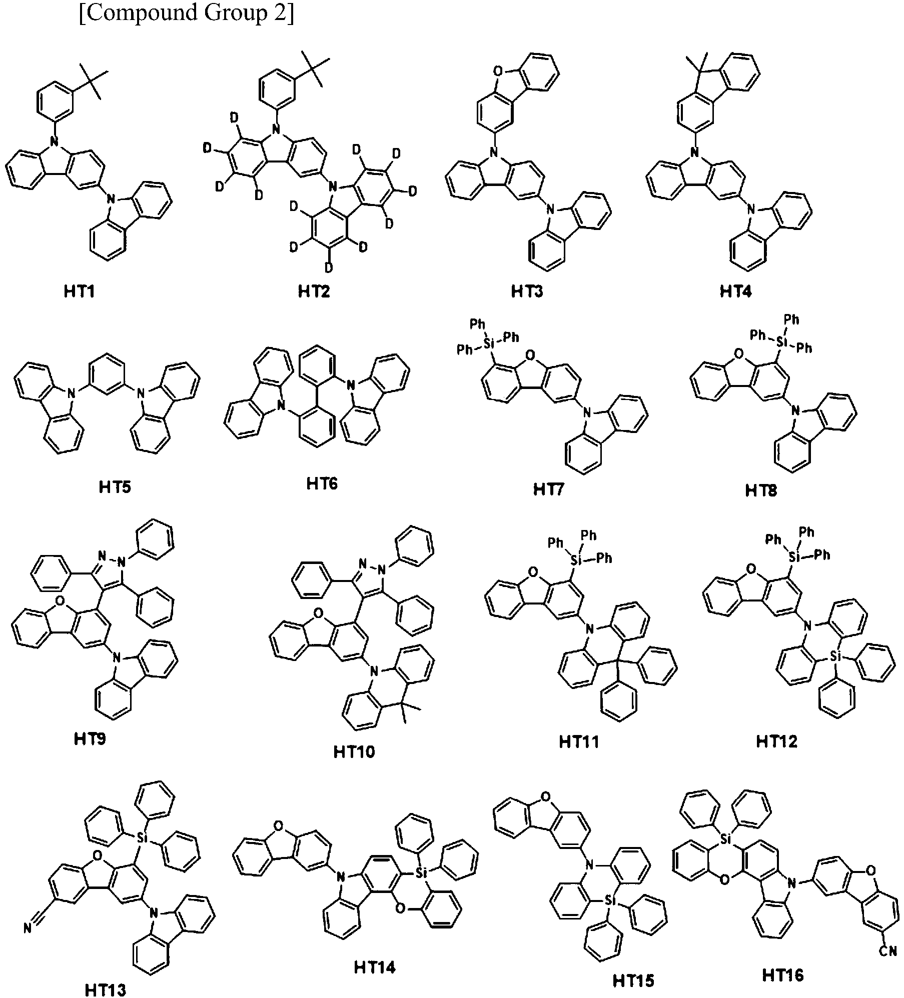

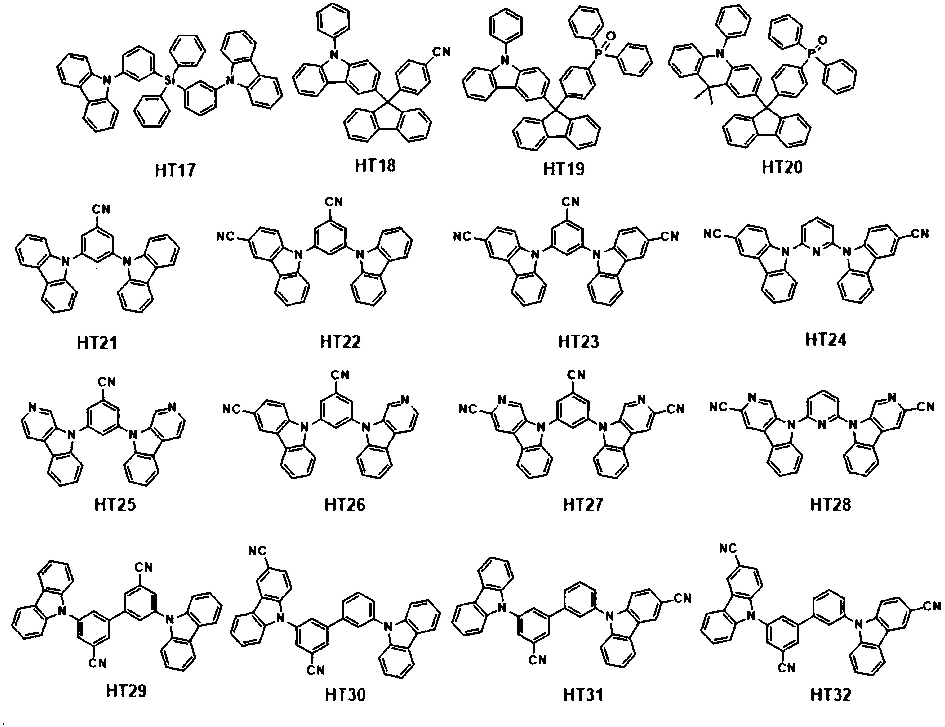

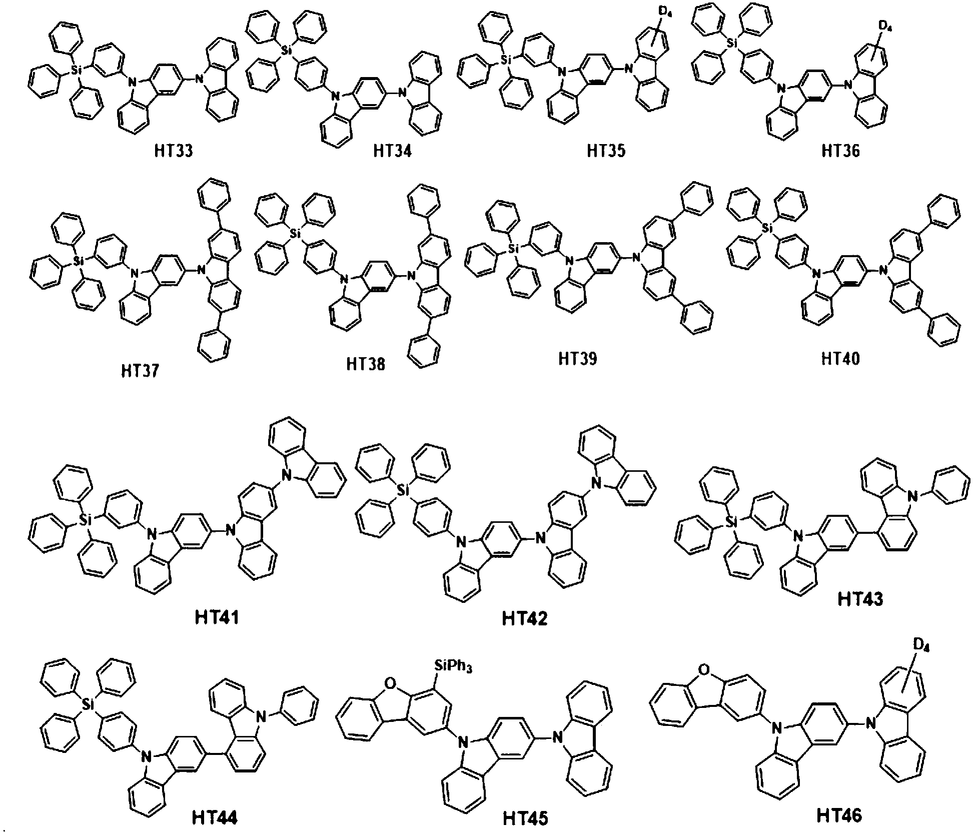

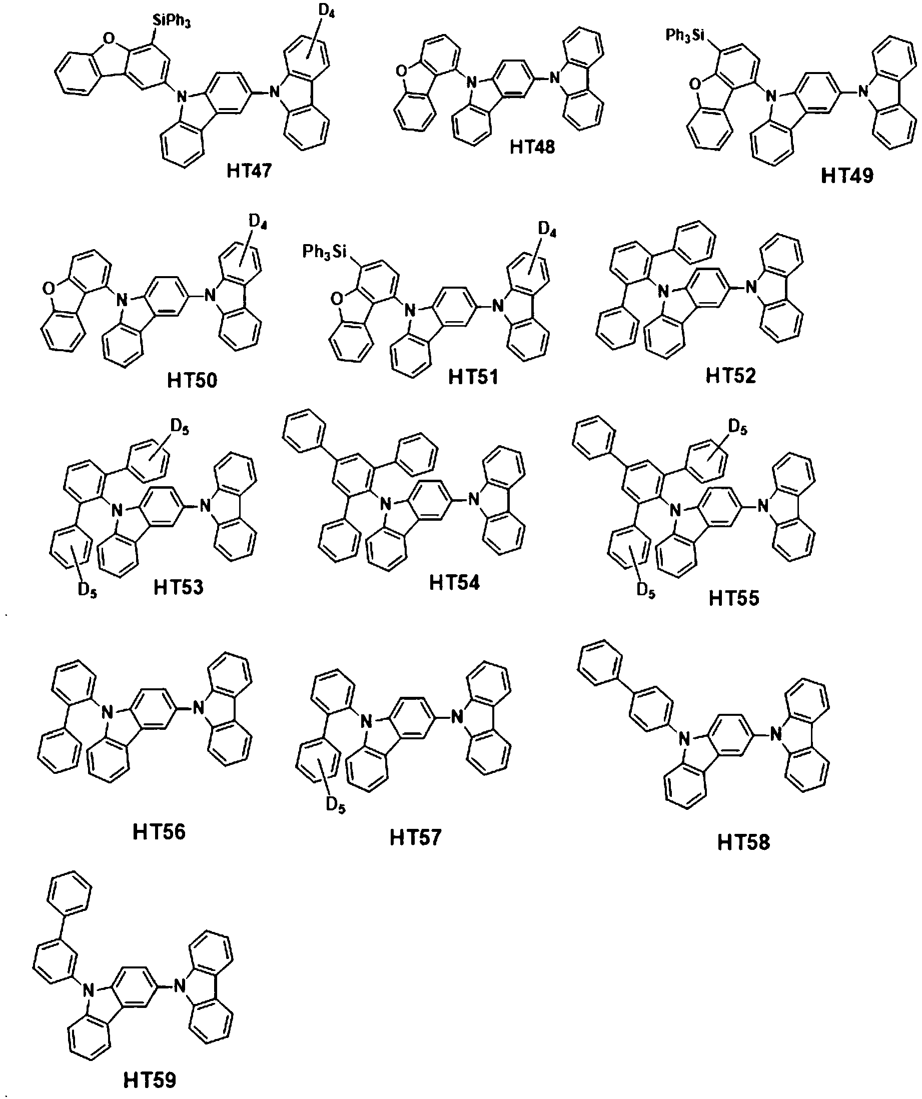

In embodiment compounds presented in Compound Group 2, "D" may mean a deuterium atom, and "Ph" may mean a substituted or unsubstituted phenyl group. For example, in embodiment compounds presented in Compound Group 2, "Ph" may mean an unsubstituted phenyl group.

In an embodiment, the emission layer EML may include the third compound represented by Formula ET-1. For example, the third compound may be used as an electron transport host material for the emission layer EML.

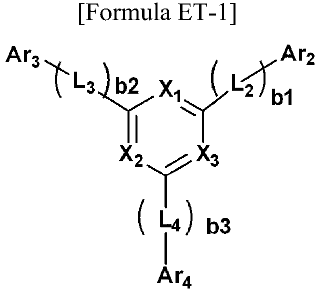

In Formula ET-1, at least one of X₁, X₂, and X₃ is N, and the rest are CR₅₆. For example, any one of X₁, X₂, or X₃ may be N, and the rest may be each independently CR₅₆. In this case, the third compound represented by Formula ET-1 may include a pyridine moiety. Alternatively, two of X₁, X₂, or X₃ may be N, and the rest may be CR₅₆. In this case, the third compound represented by Formula ET-1 may include a pyrimidine moiety. Alternatively, X₁, X₂, or X₃ may all be N. In this case, the third compound represented by Formula ET-1 may include a triazine moiety.

In Formula ET-1, R₅₆ may be a hydrogen, a deuterium, a substituted or unsubstituted alkyl group having 1 to 20 carbon atoms, a substituted or unsubstituted aryl group having 6 to 60 ring-forming carbon atoms, or a substituted or unsubstituted heteroaryl group having 2 to 60 ring-forming carbon atoms.

In Formula ET-1, b1, b2, and b3 may be each independently an integer of 0 to 10.

In Formula ET-1, Ar₂, Ar₃, and Ar₄ may be each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. For example, Ar₂, Ar₃, and Ar₄ may be each independently a substituted or unsubstituted phenyl group, or a substituted or unsubstituted carbazole group.

In Formula ET-1, L₂, L₃, and L₄ may be each independently a direct linkage, a substituted or unsubstituted arylene group having 6 to 30 ring-forming carbon atoms, or a substituted or unsubstituted heteroarylene group having 2 to 30 ring-forming carbon atoms. When b1, b2, and b3 are integers of 2 or greater, L₂, L₃, and L₄ may be each independently a substituted or unsubstituted arylene group having 6 to 30 ring-forming carbon atoms, or a substituted or unsubstituted heteroarylene group having 2 to 30 ring-forming carbon atoms.

In an embodiment, the third compound may be represented by any one among compounds in Compound Group 3 below. The light emitting device ED of an embodiment may include any one among the compounds in Compound Group 3 below.

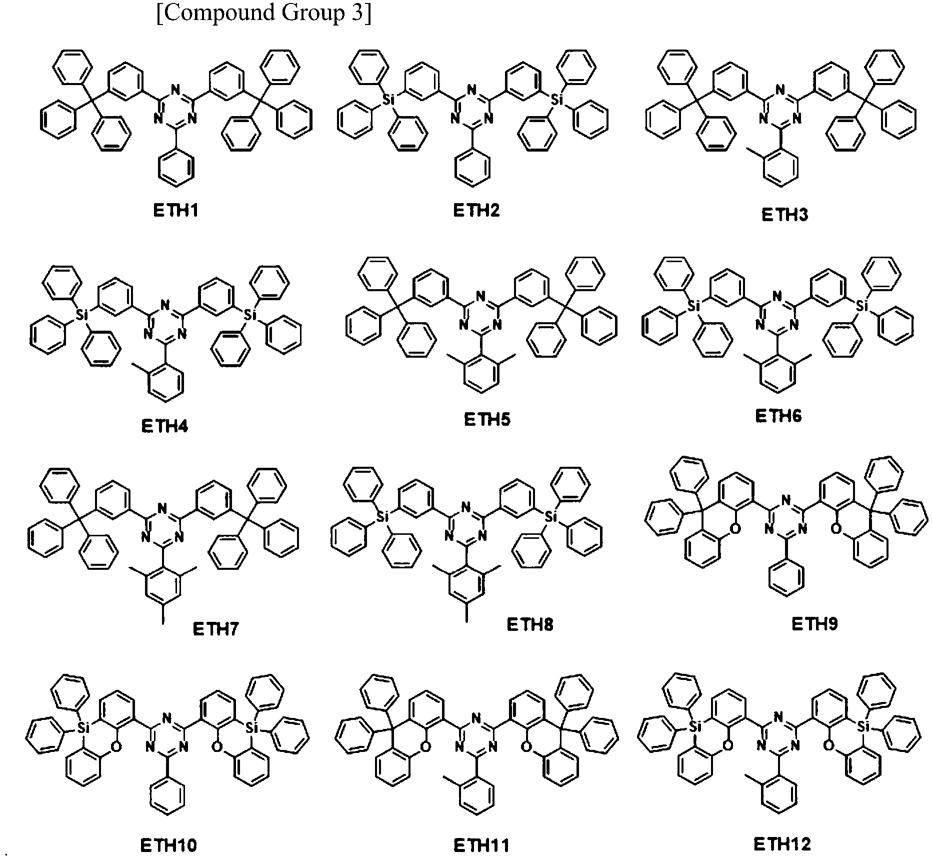

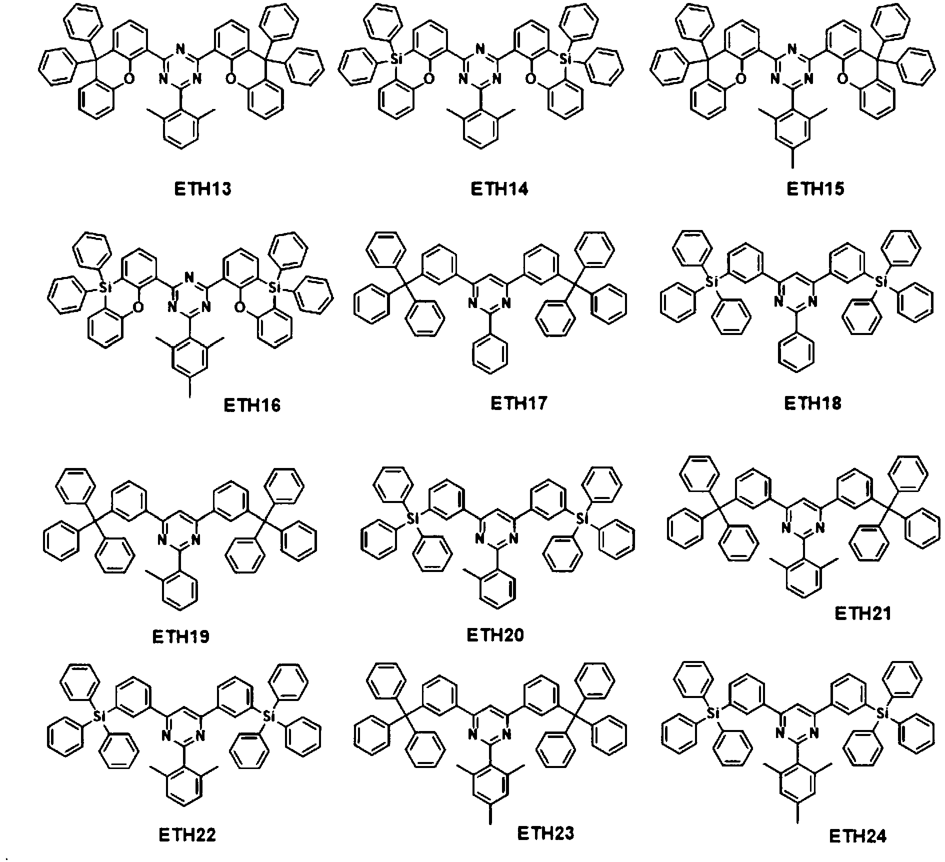

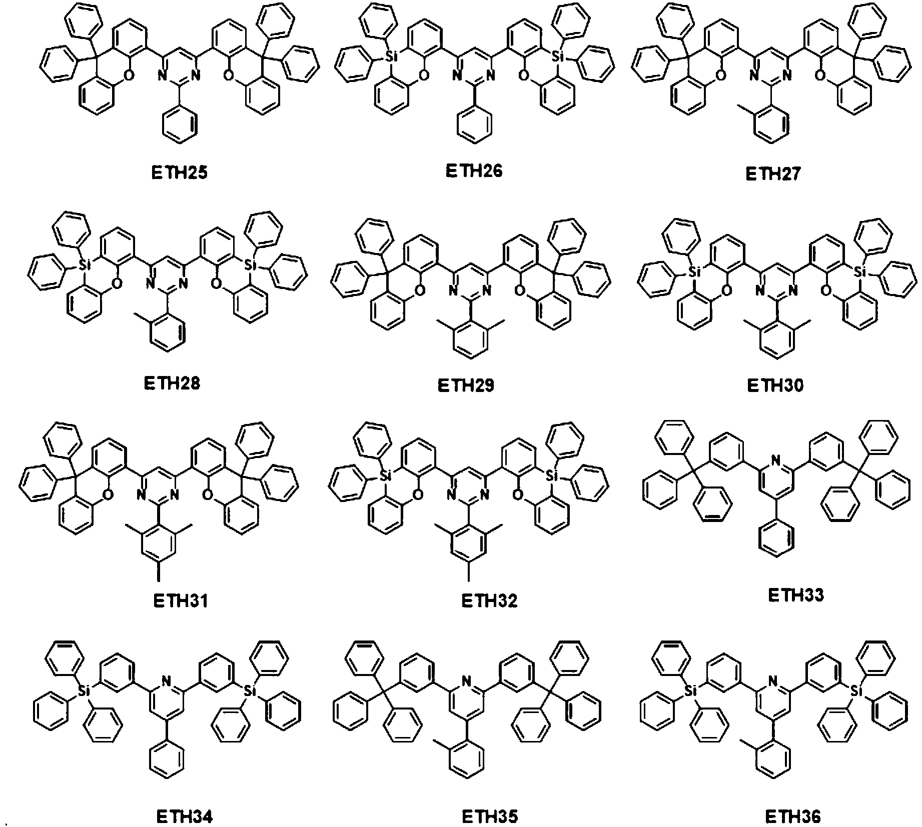

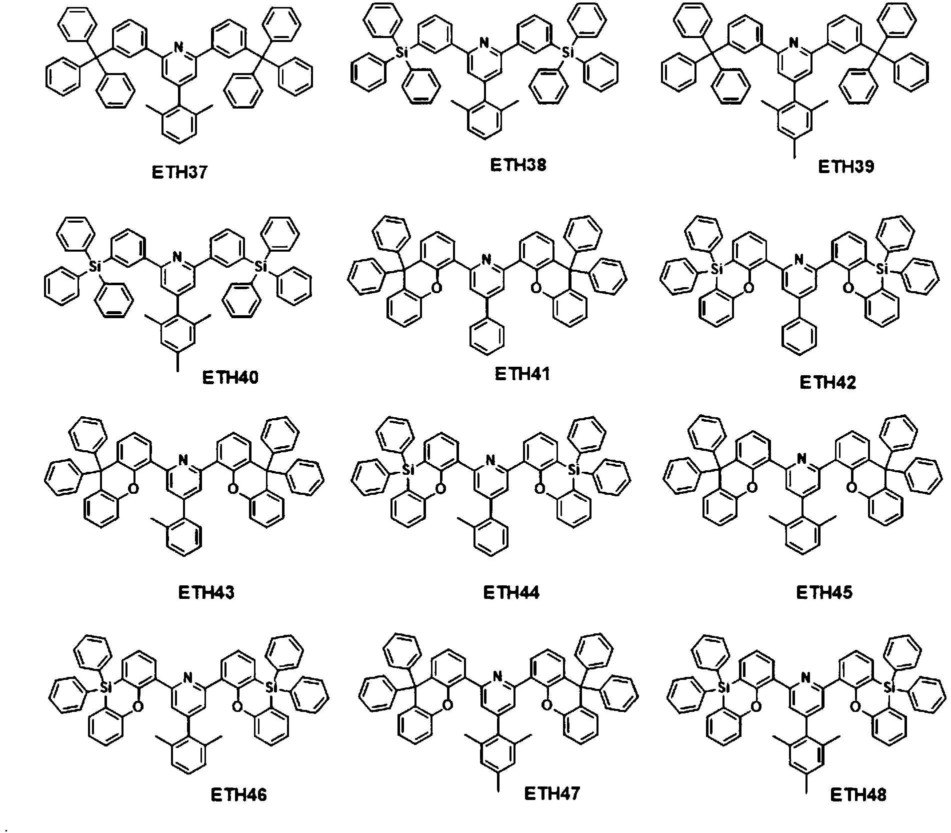

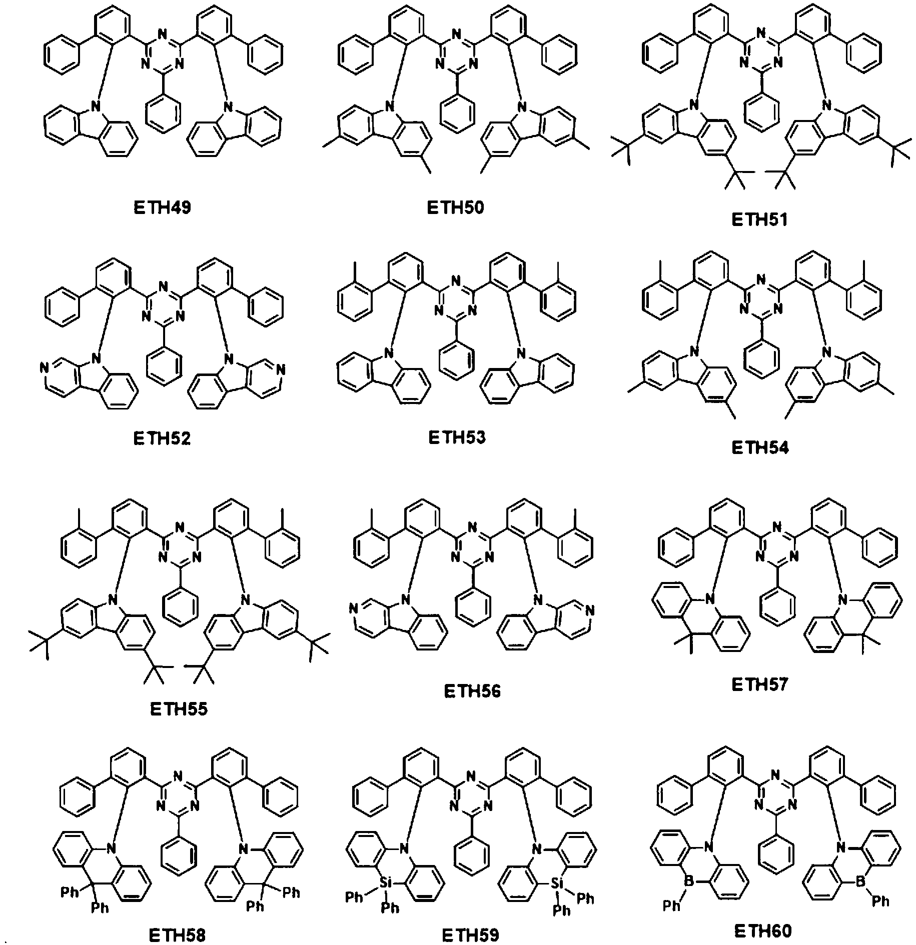

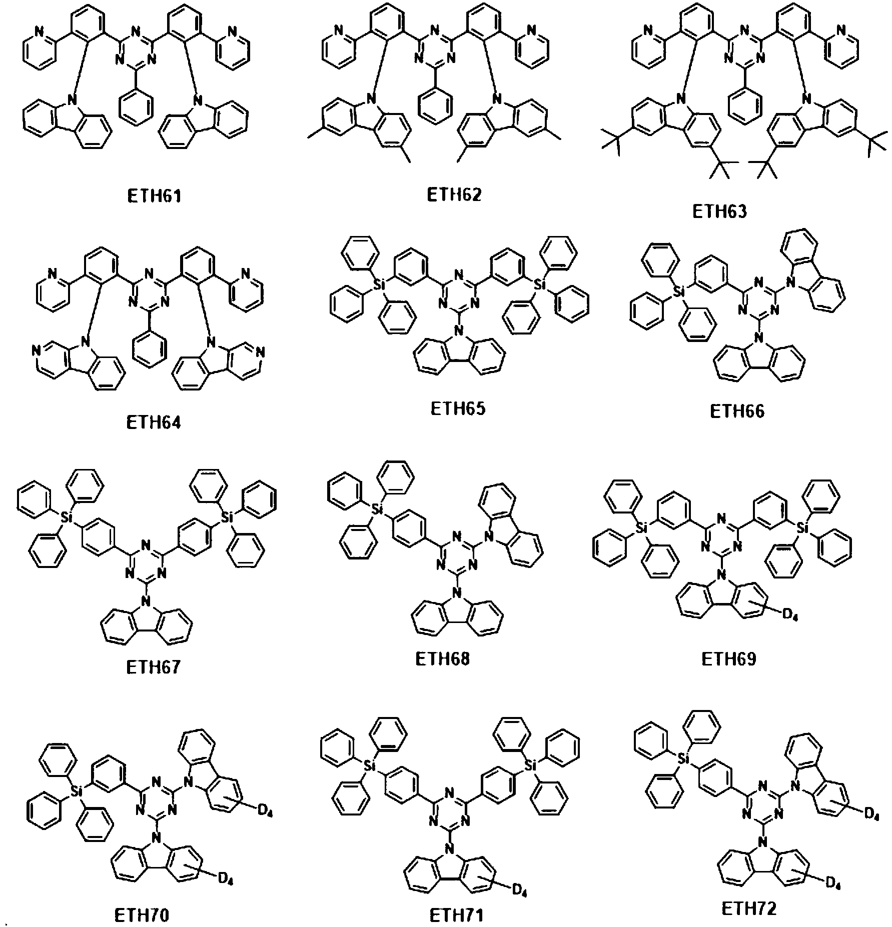

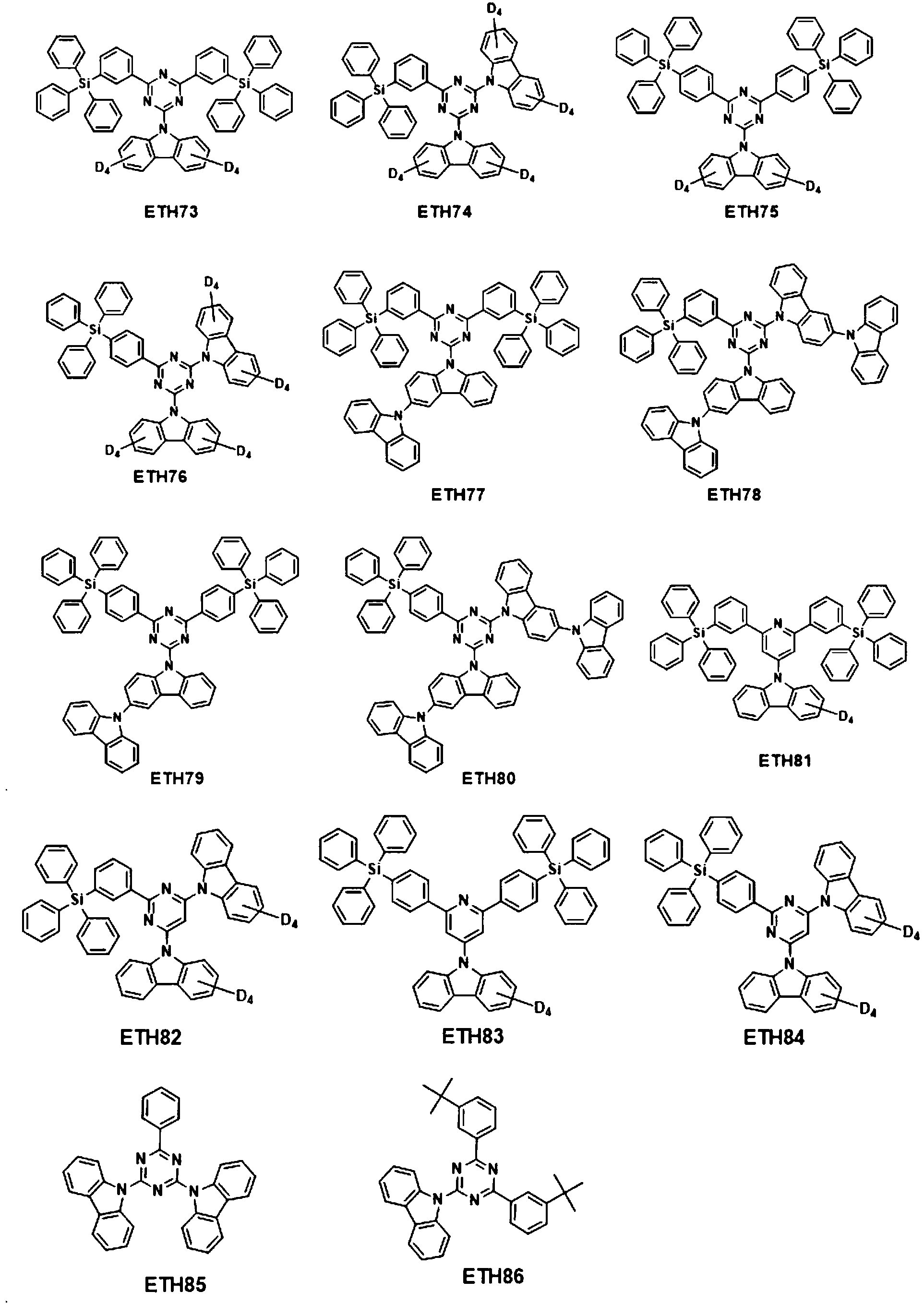

In the embodiment compounds presented in Compound Group 3, "D" refers to a deuterium atom and "Ph" refers to an unsubstituted phenyl group.

The emission layer EML may include the second compound and the third compound, and the second compound and the third compound may form an exciplex. In the emission layer EML, an exciplex may be formed by the hole transport host and the electron transport host. In this case, a triplet energy of the exciplex formed by the hole transporting host and the electron transporting host may correspond to the difference between a lowest unoccupied molecular orbital (LUMO) energy level of the electron transporting host and a highest occupied molecular orbital (HOMO) energy level of the hole transporting host.

For example, the absolute value of the triplet energy (E_{T}) of the exciplex formed by the hole transporting host and the electron transporting host may be about 2.4 eV to about 3.0 eV. In addition, the triplet energy of the exciplex may be a value smaller than an energy gap of each host material. The exciplex may have a triplet energy of about 3.0 eV or less that is an energy gap between the hole transporting host and the electron transporting host.

In an embodiment, the emission layer EML may include a fourth compound in addition to the first compound to the third compound as described above. The fourth compound may be used as a phosphorescent sensitizer of the emission layer EML. The energy may be transferred from the fourth compound to the first compound, thereby emitting light.

For example, the emission layer EML may include, as the fourth compound, an organometallic complex containing platinum (Pt) as a central metal atom and ligands linked to the central metal atom. The emission layer EML in the light emitting device ED of an embodiment may include, as the fourth compound, a compound represented by Formula D-1:

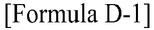

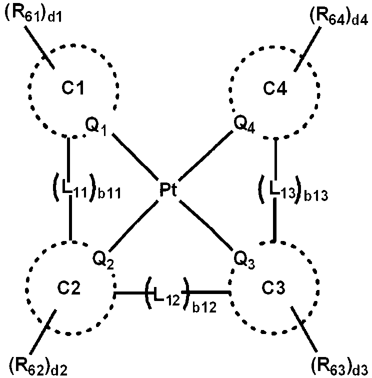

In Formula D-1, Q₁ to Q₄ may be each independently C or N. For example, if Q₁ is N then Q₃ is C and if Q₂ is C then Q₄ is N. Moreover, for example, one of Q₁ and Q₂ is N and the other is C, and one of Q₃ and Q₄ is N and the other is C.

In Formula D-1, C1 to C4 may be each independently a substituted or unsubstituted hydrocarbon ring having 5 to 30 ring-forming carbon atoms, a substituted or unsubstituted aryl group having 6 to 30 ring-forming carbon atoms, or a substituted or unsubstituted heterocycle having 2 to 30 ring-forming carbon atoms.

In Formula D-1, L₁₁, L₁₂, and L₁₃ may be each independently a direct linkage, 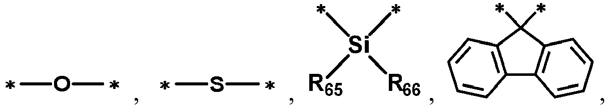 a substituted or unsubstituted divalent alkyl group having 1 to 20 carbon atoms, a substituted or unsubstituted arylene group having 6 to 30 ring-forming carbon atoms, or a substituted or unsubstituted heteroarylene group having 2 to 30 ring-forming carbon atoms. In L₁₁ to L₁₃, "-* " means a part linked to C1 to C4.

In Formula D-1, b11, b12, and b13 may be each independently 0 or 1. If b11 is 0, C1 and C2 may not be linked to each other. If b12 is 0, C2 and C3 may not be linked to each other. If b13 is 0, C3 and C4 may not be linked to each other.

In Formula D-1, R₆₁ to R₆₆ may be each independently a hydrogen, a deuterium, a halogen, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group having 1 to 20 carbon atoms, a substituted or unsubstituted alkenyl group having 2 to 20 carbon atoms, a substituted or unsubstituted aryl group having 6 to 60 ring-forming carbon atoms, or a substituted or unsubstituted heteroaryl group having 2 to 60 ring-forming carbon atoms. Alternatively, each of R₆₁ to R₆₆ may be bonded to an adjacent group to form a ring. For example, R₆₁ to R₆₆ may be each independently a substituted or unsubstituted methyl group or a substituted or unsubstituted t-butyl group, each of which, optionally includes, at least one hydrogen substituted by deuterium.

In Formula D-1, d1 to d4 are each independently an integer of 0 to 4. In Formula D-1, if each of d1 to d4 is 0, the fourth compound may not be substituted with each of R₆₁ to R₆₄. The case where each of d1 to d4 is 4 and R₆₁'s to R₆₄' are each hydrogen atoms may be the same as the case where each of d1 to d4 is 0. If each of d1 to d4 is an integer of 2 or more, a plurality of R₆₁'s to R₆₄'s may each be the same or at least one of the plurality of R₆₁'s to R₆₄'s, respectively, may be different from the others.

In Formula D-1, C1 to C4 may be each independently a substituted or unsubstituted hydrocarbon ring or a substituted or unsubstituted heterocycle represented by any one of C-1 to C-4:

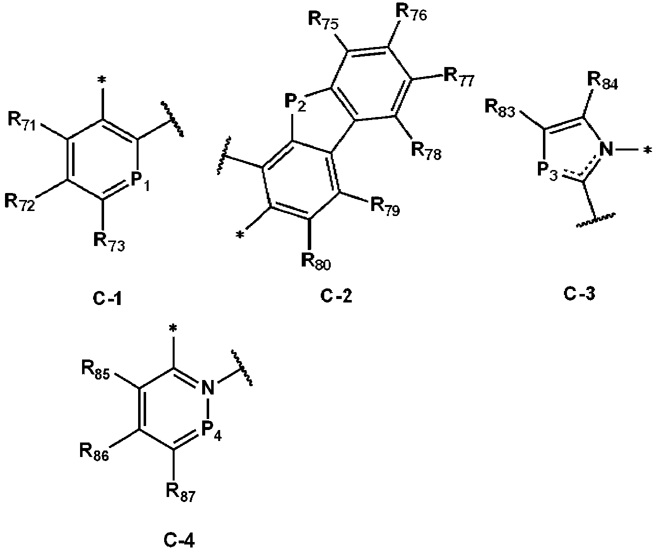

In C-1 to C-4, P₁ may be C-* or CR₇₄, P₂ may be N-* or NR₈₁, P₃ may be N-* or NR₈₂, and P₄ may be C-* or CR₈₈. R₇₁ to R₈₈ may be each independently a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or may be bonded to an adjacent group to form a ring.

In addition, in C-1 to C-4, 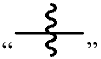 corresponds to a part linked to Pt that is a central metal atom, and "-*" corresponds to a part linked to a neighboring cyclic group (C1 to C4) or a linker (L₁₁ to L₁₃).

The emission layer of an embodiment may include the first compound, which is a fused polycyclic compound, and at least one of the second to fourth compounds. For example, the emission layer may include the first compound, the second compound, and the third compound. In the emission layer, the second compound and the third compound may form an exciplex, and the energy may be transferred from the exciplex to the first compound, thereby emitting light.

In addition, the emission layer may include the first compound, the second compound, the third compound, and the fourth compound. In the emission layer, the second compound and the third compound may form an exciplex, and the energy may be transferred from the exciplex to the fourth compound and the first compound, thereby emitting light. In an embodiment, the fourth compound may be a sensitizer. The fourth compound included in the emission layer in the light emitting device of an embodiment may serve as a sensitizer to deliver energy from the host to the first compound that is a light emitting dopant. That is, the fourth compound serving as an auxiliary dopant accelerates energy delivery to the first compound that is a light emitting dopant, thereby increasing the emission ratio of the first compound. Therefore, the emission layer of an embodiment may improve luminous efficiency. In addition, when the energy delivery to the first compound is increased, an exciton formed in the emission layer is not accumulated inside the emission layer and emits light rapidly, and thus deterioration of the device may be reduced. Therefore, the service life of the light emitting device of an embodiment may increase.

The light emitting device of an embodiment may include all of the first compound, the second compound, the third compound, and the fourth compound, and the emission layer may include the combination of two host materials and two dopant materials. In the light emitting device of an embodiment, the emission layer may simultaneously include the second compound and the third compound, which are two different hosts, the first compound that emits a delayed fluorescence, and the fourth compound including an organometallic complex, thereby exhibiting excellent luminous efficiency characteristics.

In an embodiment, the fourth compound represented by Formula D-1 may represented by at least one of the compounds represented by Compound Group 4. The emission layer may include at least one of the compounds represented by Compound Group 4 as a sensitizer material.

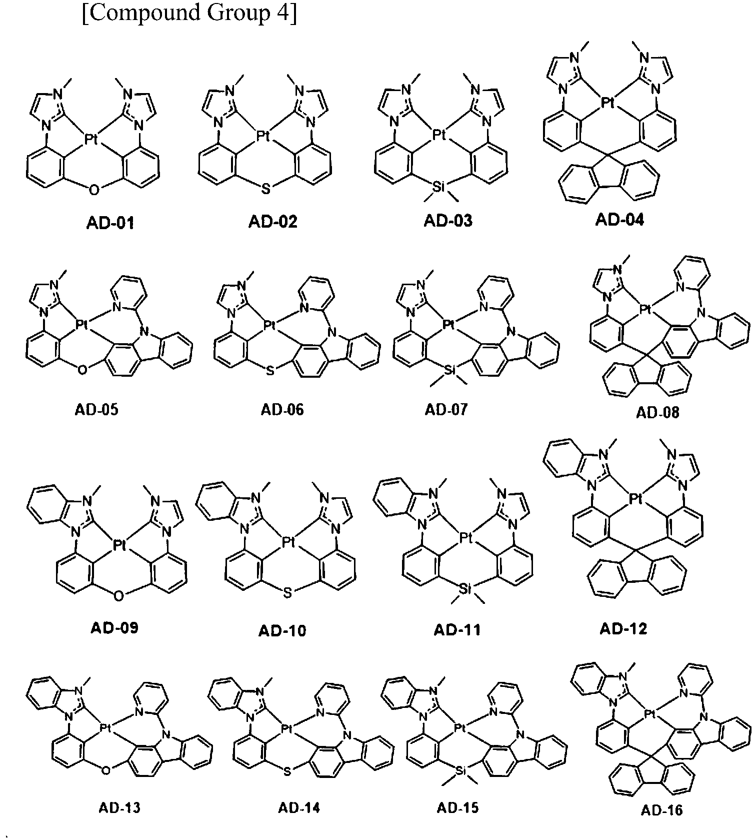

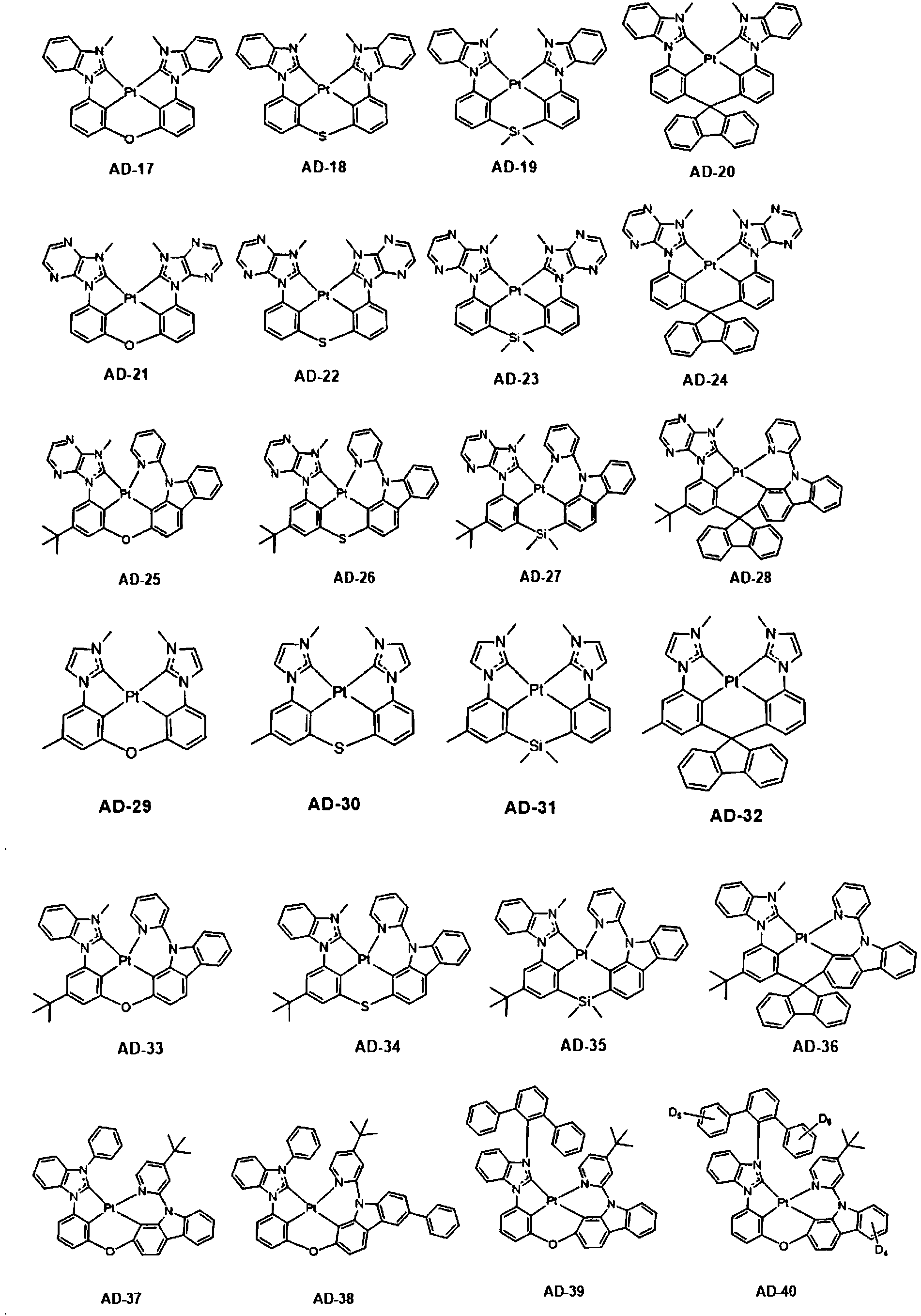

In the embodiment compounds presented in Compound Group 4, "D" means a deuterium atom.

The light emitting element of an embodiment may include a plurality of emission layers. The plurality of emission layers may be sequentially stacked and provided, and for example, the light emitting element including the plurality of emission layers may emit white light. The light emitting element including the plurality of emission layers may be a light emitting element having a tandem structure. When the light emitting element includes a plurality of emission layers, at least one emission layer may include the first compound represented by Formula 1 of an embodiment. In addition, when the light emitting element includes the plurality of emission layers, at least one emission layer may include all of the first compound, the second compound, the third compound, and the fourth compound as described above.

If the emission layer in the light emitting device of an embodiment includes each of the first compound, the second compound, and the third compound, with respect to the total weight of the first compound, the second compound, and the third compound, the content of the first compound may be about 0.1 wt% to about 5 wt%. However, the embodiments of the inventive concept are not limited thereto. If the content of the first compound satisfy the above-described proportion, the energy transfer from the second compound and the third compound to the first compound may increase, and thus the luminous efficiency and device service life may increase.

The contents of the second compound and the third compound in the emission layer may be about 65 wt% to about 95 wt% with respect to the total weight of the first compound, the second compound, and the third compound.

For example, in regard to the respective weights of the second compound and the third compound, the weight ratio of the second compound and the third compound may be about 3:7 to about 7:3.

If the contents of the second compound and the third compound satisfy the above-described weight percent and weight ratios, a charge balance characteristic in the emission layer may be improved, and thus the luminous efficiency and device service life may increase. If the contents of the second compound and the third compound deviate from the above-described ratio range, a charge balance in the emission layer is disrupted , and thus the luminous efficiency may be reduced and the device may more easily deteriorate, i.e., have a lower operational device stability.

If the emission layer includes the fourth compound, the content of the fourth compound in the emission layer may be about 10 wt% to about 30 wt% with respect to the total weight of the first compound, the second compound, the third compound, and the fourth compound. However, the embodiments of the inventive concept are not limited thereto. If the content of the fourth compound satisfies the above-described content, the energy delivery from the host to the first compound which is a light emitting dopant may be increased, thereby a luminous ratio may be improved, and thus the luminous efficiency of the emission layer may be improved. If the first compound, the second compound, the third compound, and the fourth compound included in the emission layer EML satisfy the above-described weight percent and content ratio ranges, excellent luminous efficiency and long service life may be achieved.

The light emitting layer EML may have a thickness of, for example, about 100 Å to about 1000 Å, or about 100 Å to about 300 Å. The light emitting layer EML may have a single layer composed of a single material, a single layer composed of multiple different materials, or a multilayer structure having multiple layers composed of multiple different materials.

In the light emitting elements of embodiments illustrated in FIGS. 5 to 9, the light emitting layer EML may include the polycyclic compound of an embodiment described above as a dopant. In addition, in the light emitting elements ED of embodiments illustrated in FIGS. 5 to 9, the light emitting layer EML may include at least one of a first compound, which is the polycyclic compound of an embodiment, a second compound represented by Formula HT-1, and a third compound represented by Formula ET-1. In addition, in the light emitting elements ED of embodiments illustrated in FIGS. 5 to 9, the light emitting layer EML may include a first compound, which is the polycyclic compound of an embodiment, a second compound represented by Formula HT-1, a third compound represented by Formula ET-1, and a fourth compound represented by Formula D-1. In the light emitting device ED of an embodiment, the emission layer EML may include an anthracene derivative, a pyrene derivative, a fluoranthene derivative, a chrysene derivative, a dihydrobenzanthracene derivative, or a triphenylene derivative. Specifically, the emission layer EML may include the anthracene derivative or the pyrene derivative.

In each light emitting device ED of embodiments illustrated in FIGS. 5 to 9, the emission layer EML may further include a known host and dopant besides the above-described host and dopant, and for example the emission layer EML may include a compound represented by Formula E-1. The compound represented by Formula E-1 below may be used as a fluorescent host material.

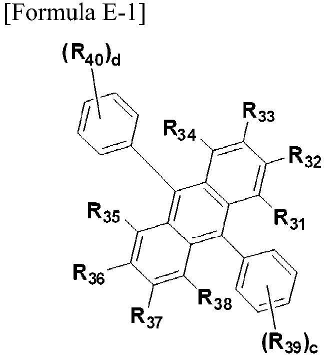

In Formula E-1, R₃₁ to R₄₀ may be each independently a hydrogen, a deuterium, a halogen, a substituted or unsubstituted silyl group, a substituted or unsubstituted thio group, a substituted or unsubstituted oxy group, a substituted or unsubstituted alkyl group having 1 to 10 carbon atoms, a substituted or unsubstituted alkenyl group having 2 to 10 carbon atoms, a substituted or unsubstituted aryl group having 6 to 30 ring-forming carbon atoms, or a substituted or unsubstituted heteroaryl group having 2 to 30 ring-forming carbon atoms, or R₃₁ to R₄₀ may be bonded to an adjacent ring group or R₃₁ to R₄₀ may be joined to an adjacent R₃₁ to R₄₀ to form a ring. For example, R₃₁ to R₄₀ may be bonded to an adjacent group to form a saturated hydrocarbon ring or an unsaturated hydrocarbon ring, a saturated heterocycle, or an unsaturated heterocycle.

In Formula E-1, c and d may be each independently an integer of 0 to 5.

Formula E-1 may be represented by any one of Compound E1 to Compound E19 below:

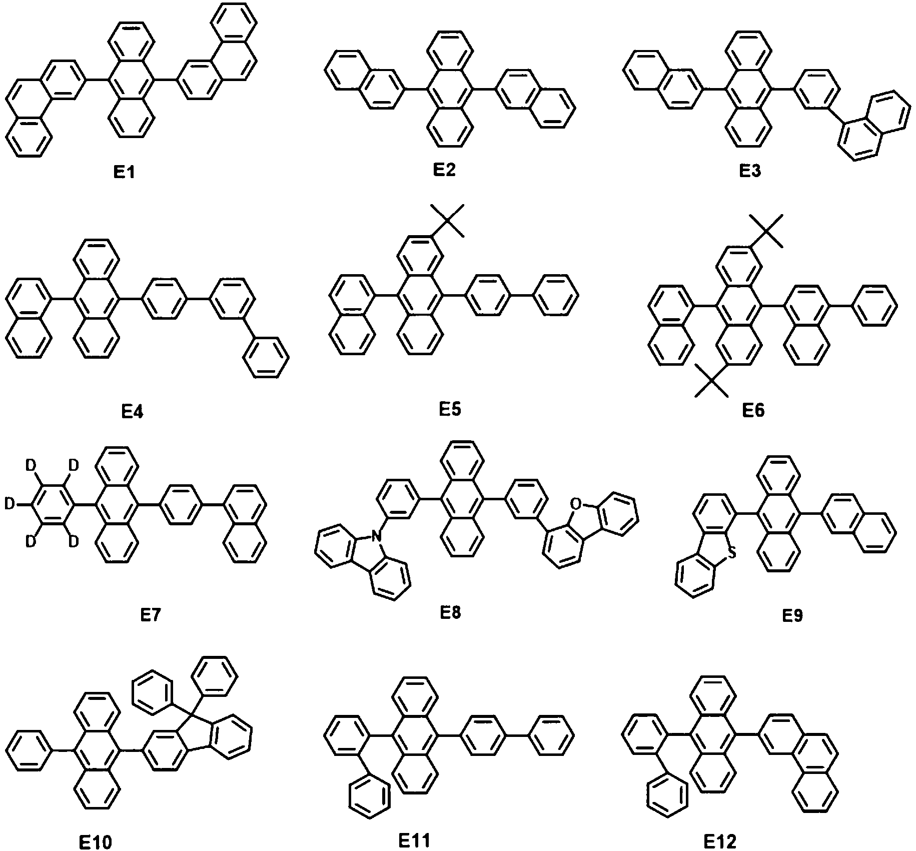

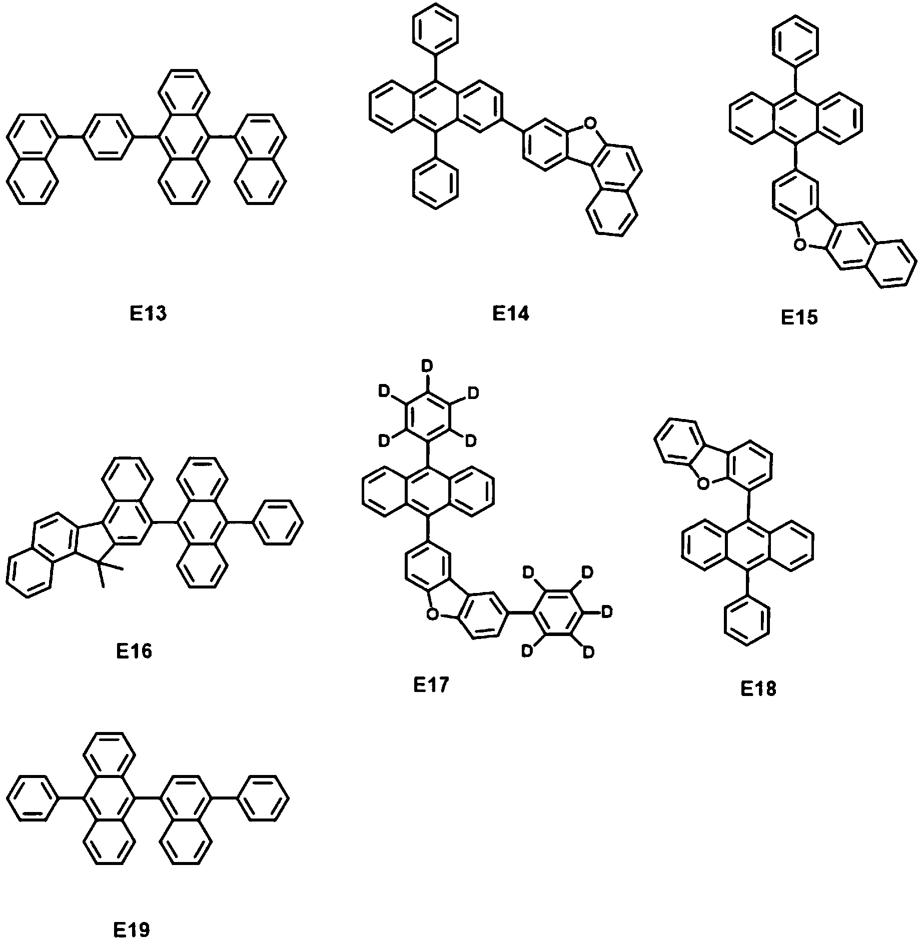

In an embodiment, the emission layer EML may include a compound represented by Formula E-2a or Formula E-2b. The compound represented by Formula E-2a or Formula E-2b may be used as a phosphorescent host material.

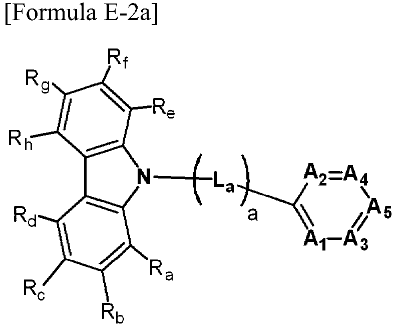

In Formula E-2a, a may be an integer of 0 to 10, and Lₐ may be a direct linkage, a substituted or unsubstituted arylene group having 6 to 30 ring-forming carbon atoms, or a substituted or unsubstituted heteroarylene group having 2 to 30 ring-forming carbon atoms. When a is an integer of 2 to 10, a plurality of Lₐ's may be each independently a substituted or unsubstituted arylene group having 6 to 30 ring-forming carbon atoms, or a substituted or unsubstituted heteroarylene group having 2 to 30 ring-forming carbon atoms.

In addition, in Formula E-2a, A₁ to A₅ may be each independently N or CRᵢ. Rₐ to Rᵢ may be each independently a hydrogen, a deuterium, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or two adjacent Rₐ to Rᵢ may join to form a ring. For example, two adjacent Rₐ to Rᵢ may join to form a hydrocarbon ring or a heterocycle containing N, O, S, *etc.*, as a ring-forming atom.

In Formula E-2a, two or three selected from among A₁ to A₅ may be N, and the rest may be CRᵢ.

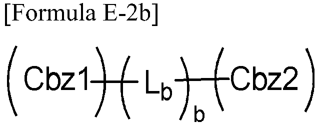

In Formula E-2b, Cbz1 and Cbz2 may be each independently an unsubstituted carbazole group, or a carbazole group substituted with an aryl group having 6 to 30 ring-forming carbon atoms. L_{b} is a direct linkage, a substituted or unsubstituted arylene group having 6 to 30 ring-forming carbon atoms, or a substituted or unsubstituted heteroarylene group having 2 to 30 ring-forming carbon atoms. b is an integer of 0 to 10, and when b is an integer of 2 to 10, a plurality of L_{b}'s may be each independently a substituted or unsubstituted arylene group having 6 to 30 ring-forming carbon atoms, or a substituted or unsubstituted heteroarylene group having 2 to 30 ring-forming carbon atoms.

The compound represented by Formula E-2a or Formula E-2b may be represented by any one among the compounds of Compound Group E-2 below. However, the compounds listed in Compound Group E-2 below are examples, and the compound represented by Formula E-2a or Formula E-2b is not limited to those represented in Compound Group E-2 below.

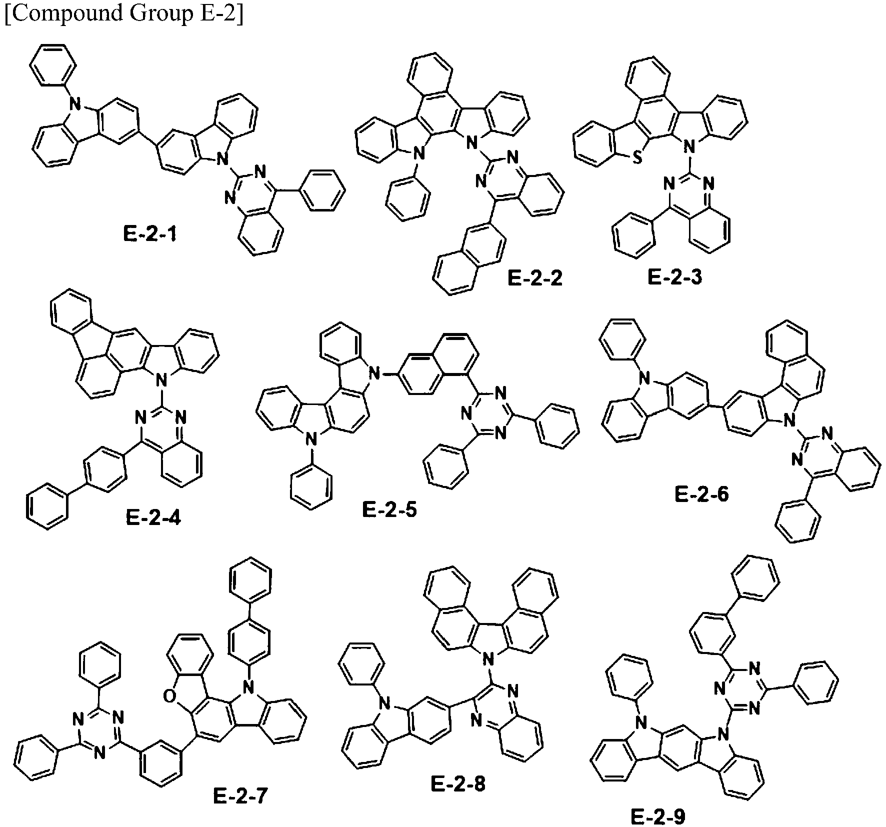

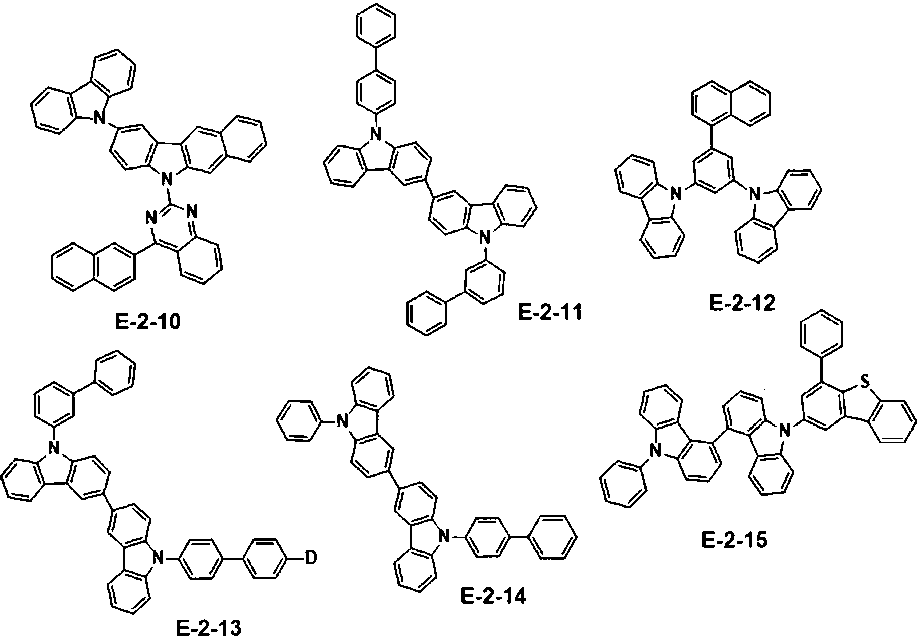

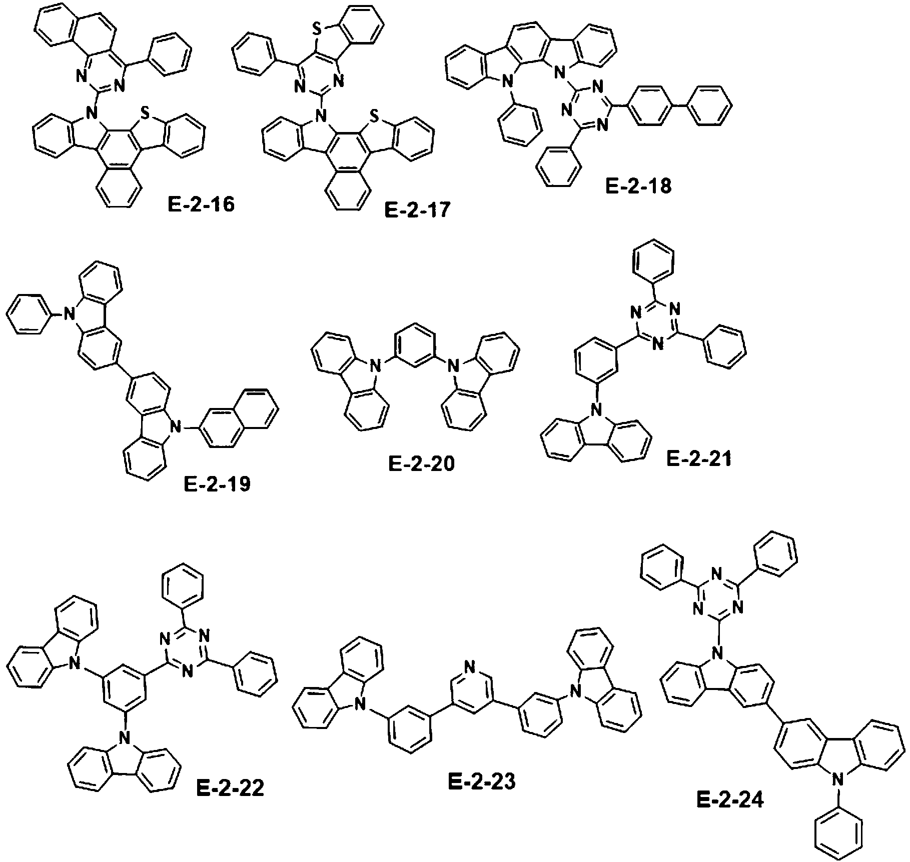

The emission layer EML may include the compound represented by Formula M-a. The compound represented by Formula M-a below may be used as a phosphorescent dopant material.

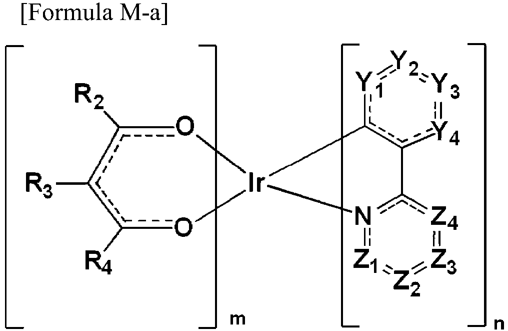

In Formula M-a above, Y₁ to Y₄ and Z₁ to Z₄ may be each CR₁ or N, R₁ to R₄ may be each independently a hydrogen atom, a deuterium atom, a substituted or unsubstituted amine group, a substituted or unsubstituted thio group, a substituted or unsubstituted oxy group, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or two adjacent R₁ to R₄ may join to form a ring. In Formula M-a, m is 0 or 1, and n is 2 or 3. In Formula M-a, when m is 0, n is 3, and when m is 1, n is 2.

The compound represented by Formula M-a may be used as a phosphorescent dopant.

The compound represented by Formula M-a may be represented by any one among Compound M-a1 to Compound M-a25 below. However, Compounds M-a1 to M-a25 below are examples, and the compound represented by Formula M-a is not limited to those represented by Compounds M-a1 to M-a25 below.

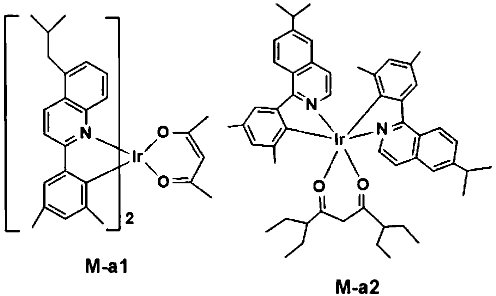

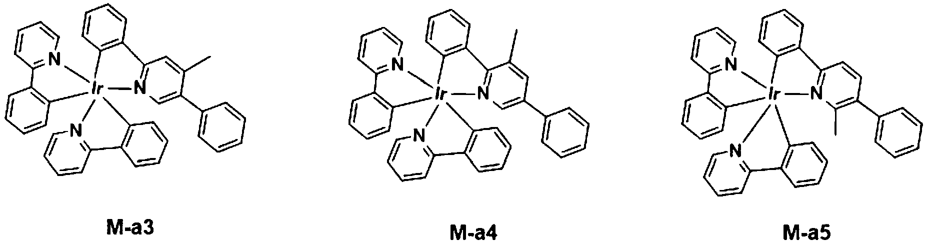

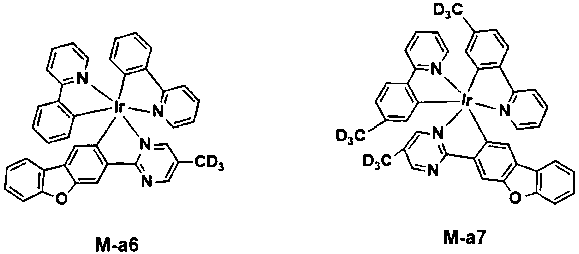

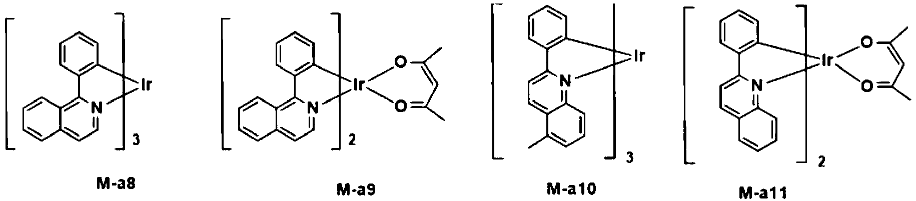

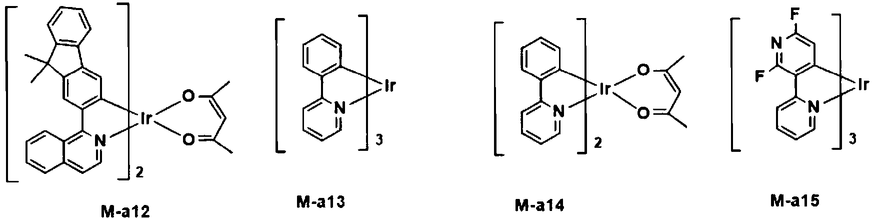

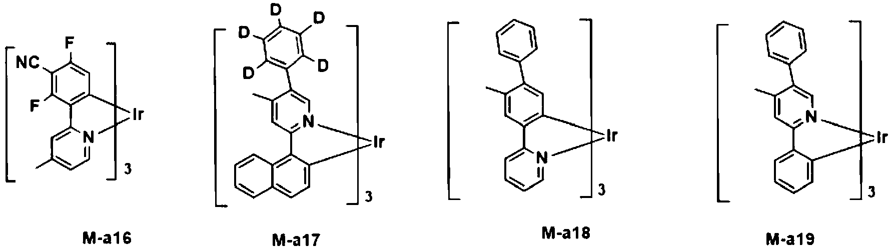

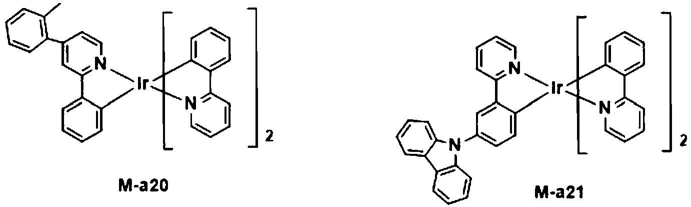

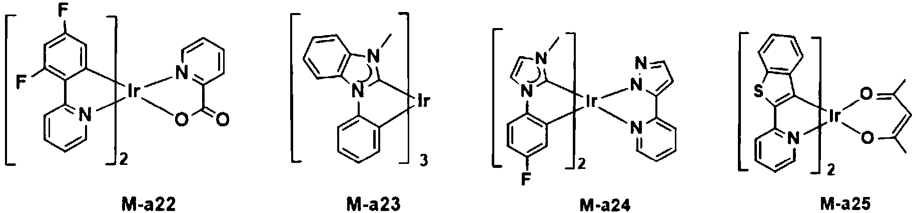

The emission layer EML may include a compound represented by any one among Formula F-a, Formula F-b, and Formula F-c. The compound represented by Formula F-a, Formula F-b, and Formula F-c may be used as a fluorescence dopant material.

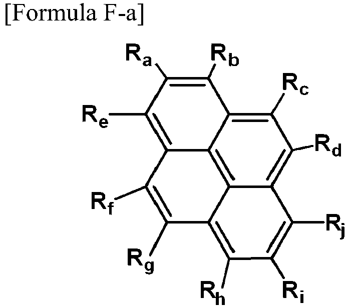

In Formula F-a above, two among Rₐ to Rⱼ may each independently be substituted with *-NAr₁Ar₂. The remaining of groups Rₐ to Rⱼ, which are not substituted with *-NAr₁Ar₂, may be each independently a hydrogen atom, a deuterium atom, a halogen atom, a cyano group, a substituted or unsubstituted amine group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Alternatively, any of the remaining adjacent groups Rₐ to Rⱼ may join to form a ring.

In *-NAr₁Ar₂, Ar₁ and Ar₂ may be each independently a substituted or unsubstituted aryl group having 6 to 30 ring-forming carbon atoms, or a substituted or unsubstituted heteroaryl group having 2 to 30 ring-forming carbon atoms. For example, at least one of Ar₁ and Ar₂ may be a heteroaryl group containing O or S as a ring-forming atom.

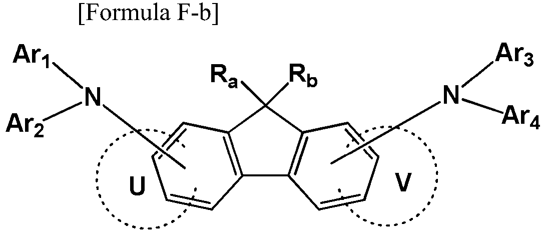

In Formula F-b, Rₐ and R_{b} may be each independently a hydrogen atom, a deuterium atom, a substituted or unsubstituted alkyl group having 1 to 20 carbon atoms, a substituted or unsubstituted alkenyl group having 2 to 20 carbon atoms, a substituted or unsubstituted aryl group having 6 to 30 ring-forming carbon atoms, or a substituted or unsubstituted heteroaryl group having 2 to 30 ring-forming carbon atoms, or Rₐ and R_{b} may be bonded to an adjacent group to form a ring. Ar₁ to Ar₄ may be each independently a substituted or unsubstituted aryl group having 6 to 30 ring-forming carbon atoms, or a substituted or unsubstituted heteroaryl group having 2 to 30 ring-forming carbon atoms.

In Formula F-b, U and V may be each independently a substituted or unsubstituted hydrocarbon ring having 5 to 30 ring-forming carbon atoms, or a substituted or unsubstituted heterocycle having 2 to 30 ring-forming carbon atoms. At least one among Ar₁ to Ar₄ may be a heteroaryl group containing O or S as a ring-forming atom.

In Formula F-b, the number of rings represented by U and V may be each independently 0 or 1. For example, in Formula F-b, it means that when the number of U or V is 1, one ring constitutes a fused ring at a portion indicated by U or V, and when the number of U or V is 0, a ring indicated by U or V does not exist. Specifically, when the number of U is 0 and the number of V is 1, or when the number of U is 1 and the number of V is 0, the fused ring having a fluorene core in Formula F-b may be a cyclic compound having four rings. In addition, when each number of U and V is 0, the fused ring in Formula F-b may be a cyclic compound having three rings. In addition, when each number of U and V is 1, the fused ring having a fluorene core in Formula F-b may be a cyclic compound having five rings.

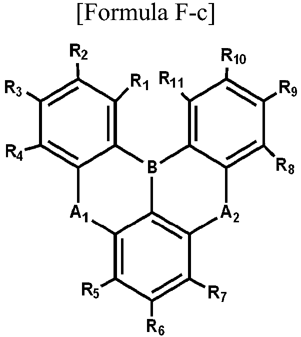

In Formula F-c, A₁ and A₂ may be each independently O, S, Se, or NRₘ, and Rₘ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. R₁ to R₁₁ are each independently a hydrogen atom, a deuterium atom, a halogen atom, a cyano group, a substituted or unsubstituted amine group, a substituted or unsubstituted boryl group, a substituted or unsubstituted oxy group, a substituted or unsubstituted thio group, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms, or any two adjacent R₁ to R₁₁ may join to form a ring.

In Formula F-c, A₁ and A₂ may each independently be bonded to substituents of an adjacent ring to form a fused ring. For example, when A₁ and A₂ are each independently NRₘ, A₁ may be bonded to R₄ or R₅ to form a ring. In addition, A₂ may be bonded to R₇ or R₈ to form a ring. In an embodiment, the emission layer EML may further include, as a dopant material, a styryl derivative (*e.g*., 1,4-bis[2-(3-N-ethylcarbazolyl)vinyl]benzene (BCzVB), 4-(di-p-tolylamino)-4'-[(di-p-tolylamino)styryl]stilbene (DPAVB), and N-(4-((E)-2-(6-((E)-4-(diphenylamino)styryl)naphthalen-2-yl)vinyl)phenyl)-N-phenylbenzenamine (N-BDAVBi), 4,4'-bis[2-(4-(N,N-diphenylamino)phenyl)vinyl]biphenyl (DPAVBi), perylene and a derivative thereof (*e.g.*, 2,5,8,11-tetra-t-butylperylene (TBP)), pyrene and a derivative thereof (*e.g.*, 1,1'-dipyrene, 1,4-dipyrenylbenzene, 1,4-bis(N,N-diphenylamino)pyrene), *etc.*

The emission layer EML may further include a phosphorescence dopant material. For example, a metal complex containing iridium (Ir), platinum (Pt), osmium (Os), gold (Au), titanium (Ti), zirconium (Zr), hafnium (Hf), europium (Eu), terbium (Tb), or thulium (Tm) may be used as a phosphorescent dopant. Specifically, iridium(III) bis(4,6-difluorophenylpyridinato-N,C2) picolinate (FIrpic), bis(2,4-difluorophenylpyridinato)-tetrakis(1-pyrazolyl)borate iridium(III) (FIr6), or platinum octaethyl porphyrin (PtOEP) may be used as a phosphorescent dopant. However, the embodiments of the inventive concept are not limited thereto.

The emission layer EML may include a quantum dot material. The core of the quantum dot may be selected from a Group II-VI compound, a Group III-VI compound, a Group I-III-VI compound, a Group III-V compound, a Group III-II-V compound, a Group IV-VI compound, a Group IV element, a Group IV compound, or a combination thereof.

The Group II-VI compound may be selected from the group consisting of a binary compound selected from the group consisting of CdSe, CdTe, CdS, ZnS, ZnSe, ZnTe, ZnO, HgS, HgSe, HgTe, MgSe, MgS, and a mixture thereof, a ternary compound selected from the group consisting of CdSeS, CdSeTe, CdSTe, ZnSeS, ZnSeTe, ZnSTe, HgSeS, HgSeTe, HgSTe, CdZnS, CdZnSe, CdZnTe, CdHgS, CdHgSe, CdHgTe, HgZnS, HgZnSe, HgZnTe, MgZnSe, MgZnS, and a mixture thereof, and a quaternary compound selected from the group consisting of HgZnTeS, CdZnSeS, CdZnSeTe, CdZnSTe, CdHgSeS, CdHgSeTe, CdHgSTe, HgZnSeS, HgZnSeTe, and a mixture thereof.

The Group III-VI compound may include a binary compound such as In₂S₃ or In₂Se₃, a ternary compound such as InGaS₃ or InGaSe₃, or any combination thereof.

The Group I-III-VI compound may be selected from a ternary compound selected from the group consisting of AgInS, AgInS₂, CuInS, CuInS₂, AgGaS₂, CuGaS₂, CuGaO₂, AgGaO₂, AgAlO₂, and a mixture thereof, or a quaternary compound such as AgInGaS₂ or CuInGaS₂.

The Group III-V compound may be selected from the group consisting of a binary compound selected from the group consisting of GaN, GaP, GaAs, GaSb, AlN, AlP, AlAs, AlSb, InN, InP, InAs, InSb, and a mixture thereof, a ternary compound selected from the group consisting of GaNP, GaNAs, GaNSb, GaPAs, GaPSb, AlNP, AlNAs, AlNSb, AlPAs, AlPSb, InGaP, InAlP, InNP, InNAs, InNSb, InPAs, InPSb, and a mixture thereof, and a quaternary compound selected from the group consisting of GaAlNP, GaAlNAs, GaAlNSb, GaAlPAs, GaAlPSb, GaInNP, GaInNAs, GaInNSb, GaInPAs, GaInPSb, InAlNP, InAlNAs, InAlNSb, InAlPAs, InAlPSb, and a mixture thereof. The Group III-V compound may further include a Group II metal. For example, InZnP, *etc.,* may be selected as a Group III-II-V compound.

The Group IV-VI compound may be selected from the group consisting of a binary compound selected from the group consisting of SnS, SnSe, SnTe, PbS, PbSe, PbTe, and a mixture thereof, a ternary compound selected from the group consisting of SnSeS, SnSeTe, SnSTe, PbSeS, PbSeTe, PbSTe, SnPbS, SnPbSe, SnPbTe, and a mixture thereof, and a quaternary compound selected from the group consisting of SnPbSSe, SnPbSeTe, SnPbSTe, and a mixture thereof. The Group IV element may be selected from the group consisting of Si, Ge, and a mixture thereof. The Group IV compound may be a binary compound selected from the group consisting of SiC, SiGe, and a mixture thereof.

Each element included in a compound such as the binary compound, the ternary compound, or the quaternary compound may be present in a particle with a uniform or non-uniform concentration distribution. That is, the formulae mean the types of elements included in the compounds, and the elemental ratio in the compound may be different. For example, AgInGaS₂ may mean AgInₓGa₁₋ₓS₂ (where x is a real number of 0 to 1).

The quantum dot may have a single structure or a double structure of core-shell in which the concentration of each element included in the quantum dot is uniform. For example, the material included in the core may be different from the material included in the shell.

The shell of the quantum dot may serve as a protection layer to prevent the chemical deformation of the core to maintain semiconductor properties, and/or a charging layer to impart electrophoresis properties to the quantum dot. The shell may be a single layer or multiple layers. An interface between the core and the shell may have a concentration gradient in which the concentration of an element present in the shell becomes lower towards the center.

In some embodiments, the quantum dot may have the above-described core/shell structure including a core containing nanocrystals and a shell surrounding the core. An example of the shell of the quantum dots may include a metal or non-metal oxide, a semiconductor compound, or a combination thereof.

For example, the metal or non-metal oxide may be a binary compound such as SiO₂, Al₂O₃, TiO₂, ZnO, MnO, Mn₂O₃, Mn₃O₄, CuO, FeO, Fe₂O₃, Fe₃O₄, CoO, Co₃O₄, or NiO, or a ternary compound such as MgAl₂O₄, CoFe₂O₄, NiFe₂O₄, or CoMn₂O₄, but the embodiments of the inventive concept are not limited thereto.

Also, examples of the semiconductor compound may include CdS, CdSe, CdTe, ZnS, ZnSe, ZnTe, ZnSeS, ZnTeS, GaAs, GaP, GaSb, HgS, HgSe, HgTe, InAs, InP, InGaP, InSb, AlAs, AlP, AlSb, *etc.,* but the embodiments of the inventive concept are not limited thereto.

Each element included in a compound such as the binary compound, or the ternary compound may be present in a particle with a uniform or non-uniform concentration distribution. That is, the formulae mean the types of elements included in the compounds, and the elemental ratio in the compound may be different.

The quantum dot may have a full width of half maximum (FWHM) of a light emitting wavelength spectrum of about 45 nm or less, preferably about 40 nm or less, and more preferably about 30 nm or less, and color purity or color reproducibility may be improved in the above range. In addition, light emitted through such quantum dot is emitted in all directions so that a wide viewing angle may be improved.

In addition, although the form of the quantum dot is not particularly limited as long as it is a form commonly used in the art, more specifically, the quantum dot in the form of spherical, pyramidal, multi-arm, or cubic nanoparticles, nanotubes, nanowires, nanofibers, nanoplate particles, *etc.* may be used.

As the size of the quantum dot is adjusted or the elemental ratio in the quantum dot compound is adjusted, it is possible to control the energy band gap, and thus light in various wavelength ranges may be obtained in the quantum dot emission layer. Therefore, the quantum dot as above (using different sizes of quantum dots or different elemental ratios in the quantum dot compound) is used, and thus the light emitting device, which emits light in various wavelengths, may be implemented. Specifically, the adjustment of the size of the quantum dot or the elemental ratio in the quantum dot compound may be selected to emit red, green, and/or blue light. In addition, the quantum dots may be configured to emit white light by combining various colors of light.

In each of the light emitting devices ED of embodiments illustrated in FIGS. 5 to 9, the electron transport region ETR is provided on the emission layer EML. The electron transport region ETR may include at least one of the hole blocking layer HBL, the electron transport layer ETL, and the electron injection layer EIL, but the embodiments of the inventive concept are not limited thereto.

The electron transport region ETR may have a single layer formed of a single material, a single layer formed of a plurality of different materials, or a multilayer structure including a plurality of layers formed of a plurality of different materials.

For example, the electron transport region ETR may have a single layer structure of the electron injection layer EIL or the electron transport layer ETL, and may have a single layer structure formed of an electron injection material and an electron transport material. In addition, the electron transport region ETR may have a single layer structure formed of a plurality of different materials, or may have a structure in which an electron transport layer ETL/electron injection layer EIL, a hole blocking layer HBL/electron transport layer ETL/electron injection layer EIL are stacked in order from the emission layer EML, but the embodiments of the inventive concept are not limited thereto. The electron transport region ETR may have a thickness, for example, from about 1,000 Å to about 1,500 Å.

The electron transport region ETR may be formed using various methods such as a vacuum deposition method, a spin coating method, a cast method, a Langmuir-Blodgett (LB) method, an inkjet printing method, a laser printing method, and a laser induced thermal imaging (LITI) method.

The electron transport region ETR may include a compound represented by Formula ET-2:

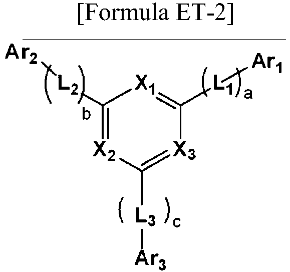

In Formula ET-2, at least one of X₁, X₂, and X₃ is N, and the remaining are CRₐ. Rₐ may be a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms. Ar₁, Ar₂, and Ar₃ may be each independently a hydrogen atom, a deuterium atom, a substituted or unsubstituted alkyl group having 1 to 20 carbon atoms, a substituted or unsubstituted aryl group having 6 to 30 ring-forming carbon atoms, or a substituted or unsubstituted heteroaryl group having 2 to 30 ring-forming carbon atoms.

In Formula ET-2, a, b, and c may be each independently an integer of 0 to 10. In Formula ET-2, L₁, L₂, and L₃ may be each independently a direct linkage, a substituted or unsubstituted arylene group having 6 to 30 ring-forming carbon atoms, or a substituted or unsubstituted heteroarylene group having 2 to 30 ring-forming carbon atoms. If a, b, and c are each independently an integer of 2 or more, L₁, L₂, and L₃ may be each independently a substituted or unsubstituted arylene group having 6 to 30 ring-forming carbon atoms, or a substituted or unsubstituted heteroarylene group having 2 to 30 ring-forming carbon atoms.

The electron transport region ETR may include an anthracene-based compound. However, the embodiments of the inventive concept are not limited thereto, and the electron transport region ETR may include, for example, tris(8-hydroxyquinolinato)aluminium (Alq₃), 1,3,5-tri[(3-pyridyl)-phen-3-yl]benzene, 2,4,6-tris(3'-(pyridin-3-yl)biphenyl-3-yl)-1,3,5-triazine, 2-(4-(N-phenylbenzoimidazol-1-yl)phenyl)-9,10-dinaphthylanthracene, 1,3,5-tri(1-phenyl-1H-benzo[d]imidazol-2-yl)benzene (TPBi), 2,9-dimethyl-4,7-diphenyl-1,10-phenanthroline (BCP), 4,7-diphenyl-1,10-phenanthroline (Bphen), 3-(4-biphenylyl)-4-phenyl-5-tert-butylphenyl-1,2,4-triazole (TAZ), 4-(naphthalen-1-yl)-3,5-diphenyl-4H-1,2,4-triazole (NTAZ), 2-(4-biphenylyl)-5-(4-*tert*-butylphenyl)-1,3,4-oxadiazole (tBu-PBD), bis(2-methyl-8-quinolinolato-N1,O8)-(1,1'-biphenyl-4-olato)aluminum (BAlq), beryllium bis(benzoquinolin-10-olate) (Bebq₂), 9,10-di(naphthalene-2-yl)anthracene (ADN), 1,3-bis[3,5-di(pyridin-3-yl)phenyl]benzene (BmPyPhB), or a mixture thereof.

The electron transport region ETR may include at least one among Compound ET1 to Compound ET36:

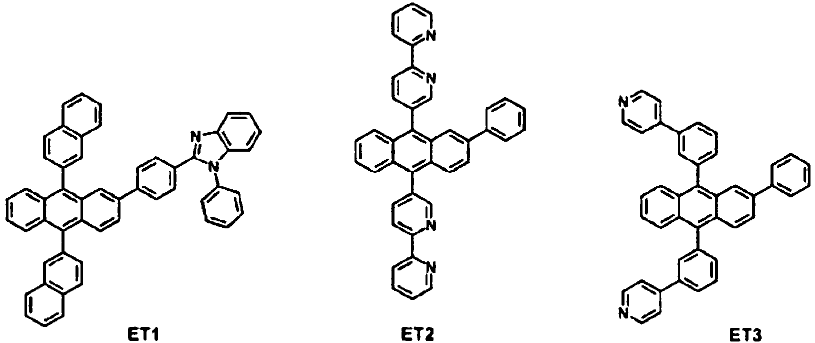

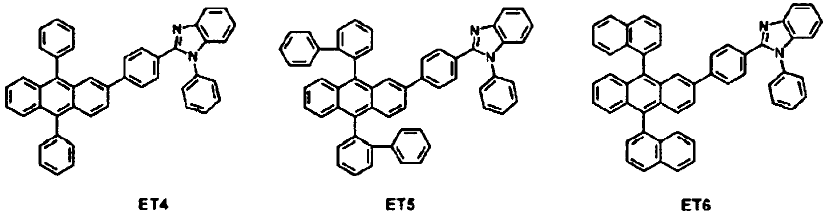

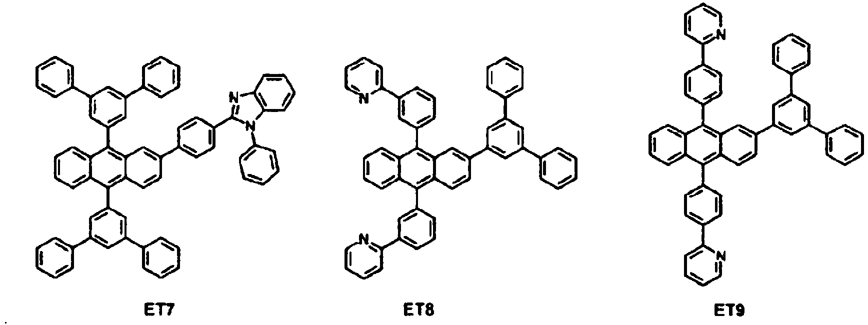

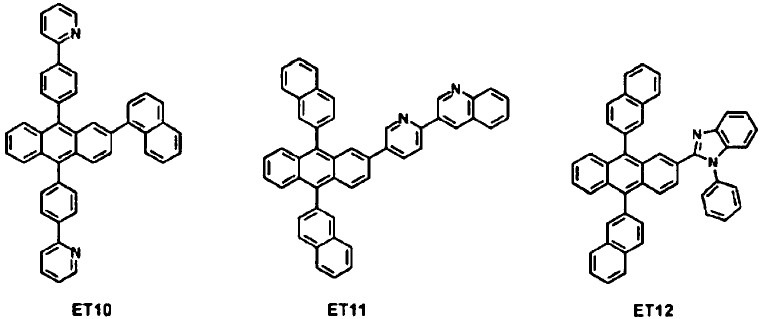

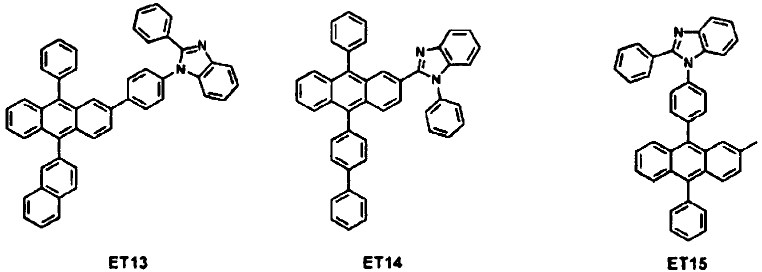

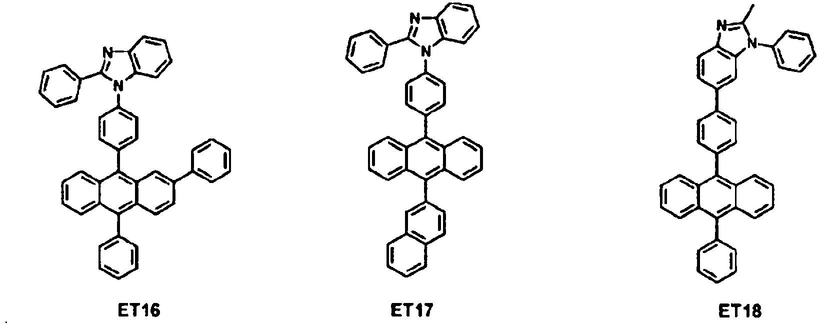

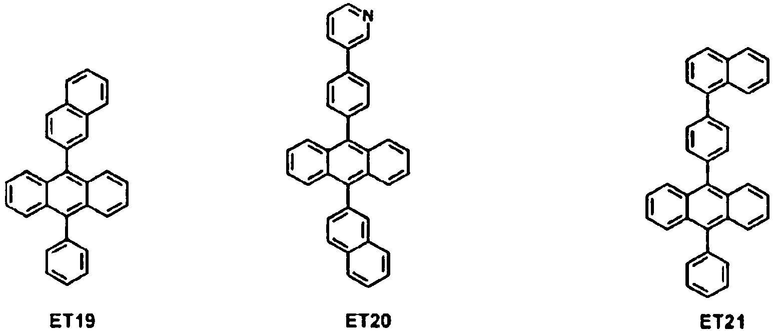

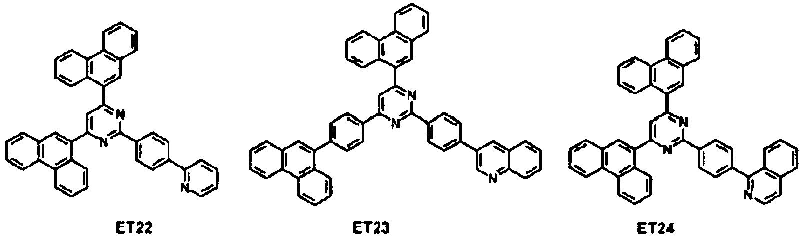

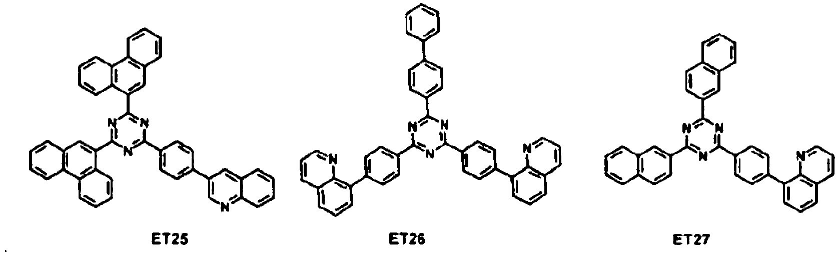

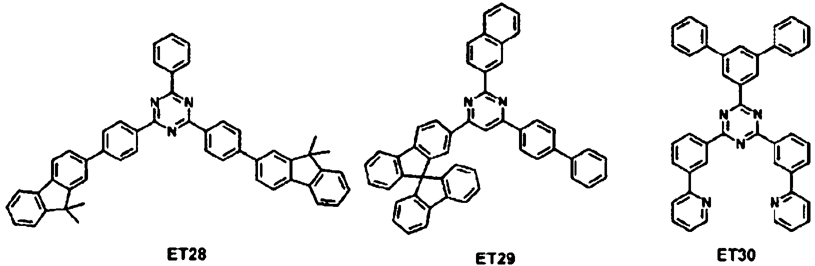

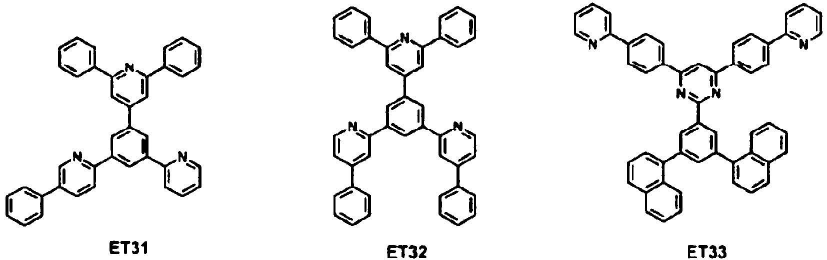

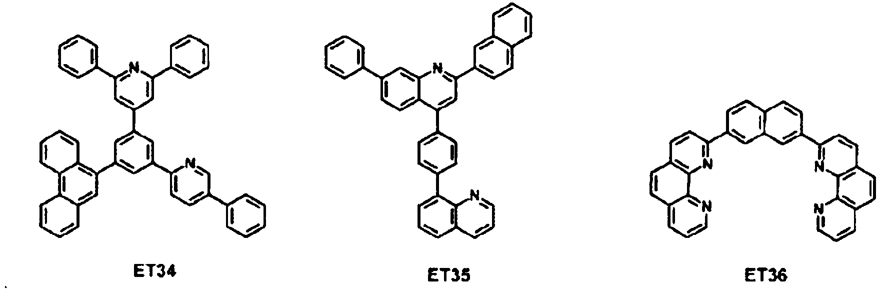

In addition, the electron transport region ETR may include a metal halide such as LiF, NaCl, CsF, RbCl, RbI, CuI, and KI, a lanthanide metal such as Yb, and a co-deposited material of the metal halide and the lanthanide metal. For example, the electron transport region ETR may include KI:Yb, RbI:Yb, LiF:Yb, *etc.,* as a co-deposited material. The electron transport region ETR may be formed using a metal oxide such as Li₂O or BaO, or 8-hydroxyl-lithium quinolate (Liq), *etc.,* but the embodiments of the inventive concept are not limited thereto. The electron transport region ETR may also be formed of a mixture material of an electron transport material and an insulating organometallic salt. The organometallic salt may be a material having an energy band gap of about 4 eV or more. Specifically, the organometallic salt may include, for example, a metal acetate, a metal benzoate, a metal acetoacetate, a metal acetylacetonate, or a metal stearate.

The electron transport region ETR may further include at least one of 2,9-dimethyl-4,7-diphenyl-1,10-phenanthroline (BCP), diphenyl(4-(triphenylsilyl)phenyl)phosphine oxide (TSPO1), and 4,7-diphenyl-1,10-phenanthroline (Bphen) in addition to the above-described materials, but the embodiments of the inventive concept are not limited thereto.

The electron transport region ETR may include the above-described compounds of the hole transport region HTR in at least one of the electron injection layer EIL, the electron transport layer ETL, and the hole blocking layer HBL.

If the electron transport region ETR includes the electron transport layer ETL, the electron transport layer ETL may have a thickness of about 100 Angstroms (Å) to about 1,000 Å, for example, about 150 Å to about 500 Å. If the thickness of the electron transport layer ETL satisfies the aforementioned range, satisfactory electron transport characteristics may be obtained without a substantial increase in driving voltage. if the electron transport region ETR includes the electron injection layer EIL, the electron injection layer EIL may have a thickness of about 1 Å to about 100 Å, for example, about 3 Å to about 90 Å. If the thickness of the electron injection layer EIL satisfies the above-described range, satisfactory electron injection characteristics may be obtained without a substantial increase in driving voltage.

The second electrode EL2 is provided on the electron transport region ETR. The second electrode EL2 may be a common electrode. The second electrode EL2 may be a cathode or an anode, but the embodiments of the inventive concept are not limited thereto. For example, when the first electrode EL1 is an anode, the second electrode EL2 may be a cathode, and when the first electrode EL1 is a cathode, the second electrode EL2 may be an anode.

The second electrode EL2 may be a transmissive electrode, a transflective electrode, or a reflective electrode. When the second electrode EL2 is the transmissive electrode, the second electrode EL2 may be formed of a transparent metal oxide, for example, indium tin oxide (ITO), indium zinc oxide (IZO), zinc oxide (ZnO), indium tin zinc oxide (ITZO), *etc.*

If the second electrode EL2 is the transflective electrode or the reflective electrode, the second electrode EL2 may include Ag, Mg, Cu, Al, Pt, Pd, Au, Ni, Nd, Ir, Cr, Li, Ca, LiF/Ca, LiF/Al, Mo, Ti, Yb, W, or a compound or mixture thereof (e.g., AgMg, AgYb, or MgYb). Alternatively, the second electrode EL2 may have a multilayer structure including a reflective film or a transflective film formed of the above-described materials, and a transparent conductive film formed of ITO, IZO, ZnO, ITZO, *etc.* For example, the second electrode EL2 may include the above-described metal materials, combinations of at least two metal materials of the above-described metal materials, oxides of the above-described metal materials, or the like.

Although not shown, the second electrode EL2 may be connected with an auxiliary electrode. If the second electrode EL2 is connected with the auxiliary electrode, the resistance of the second electrode EL2 may be decreased.

A capping layer CPL as shown in FIG. 9 may further be disposed on the second electrode EL2 of the light emitting device ED of an embodiment. The capping layer CPL may include a multilayer or a single layer.

In an embodiment, the capping layer CPL may be an organic layer or an inorganic layer. For example, when the capping layer CPL contains an inorganic material, the inorganic material may include an alkaline metal compound (*e.g*., LiF), an alkaline earth metal compound (*e.g.,* MgF₂), SiON, SiNₓ, SiOy, *etc.*

For example, when the capping layer CPL includes an organic material, the organic material may include α-NPD, NPB, TPD, m-MTDATA, Alq₃, CuPc, N4,N4,N4',N4'-tetra(biphenyl-4-yl)biphenyl-4,4'-diamine (TPD15), 4,4',4"-tris(carbazol-9-yl)triphenylamine (TCTA), etc., or an epoxy resin, or acrylate such as methacrylate. However, the embodiments of the inventive concept are not limited thereto, and the capping layer CPL may include at least one among Compounds P1 to P5:

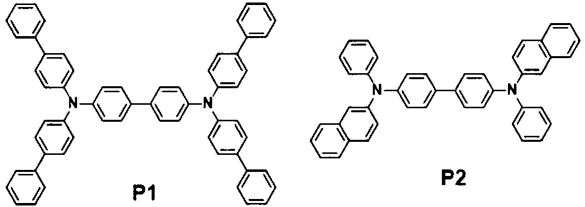

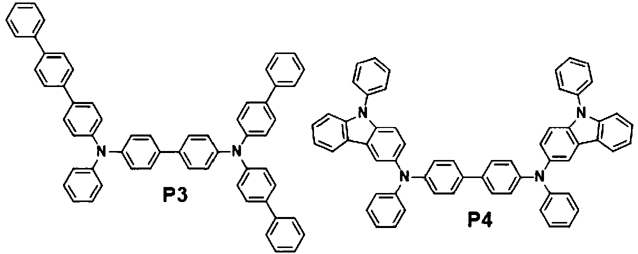

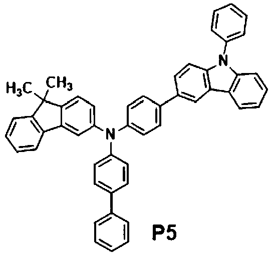

The refractive index of the capping layer CPL may be about 1.6 or more. Specifically, the refractive index of the capping layer CPL may be about 1.6 or more with respect to light in a wavelength range of about 550 nm to about 660 nm.

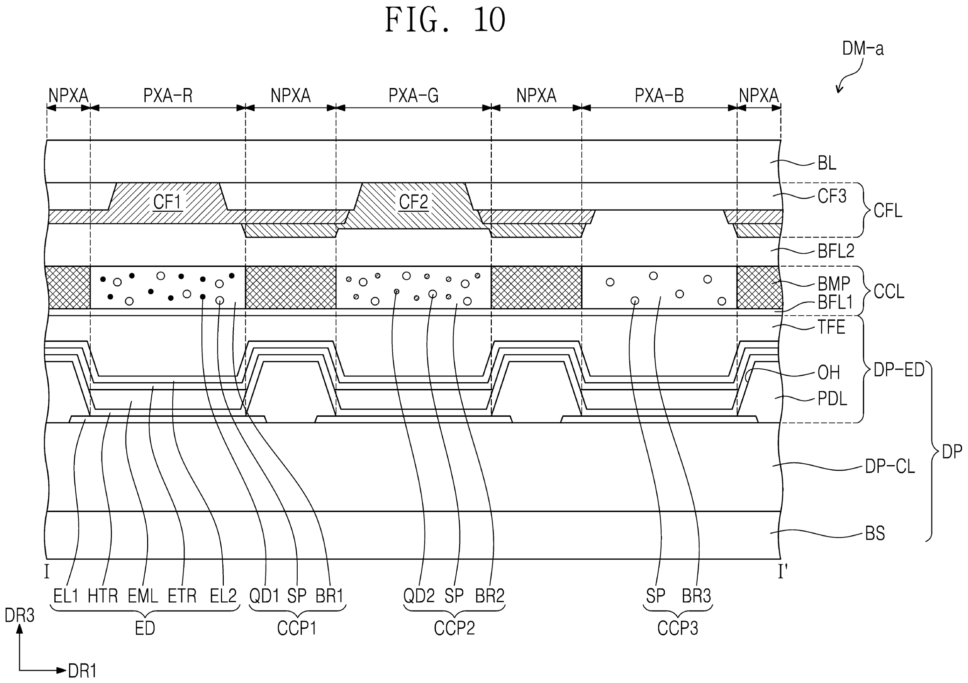

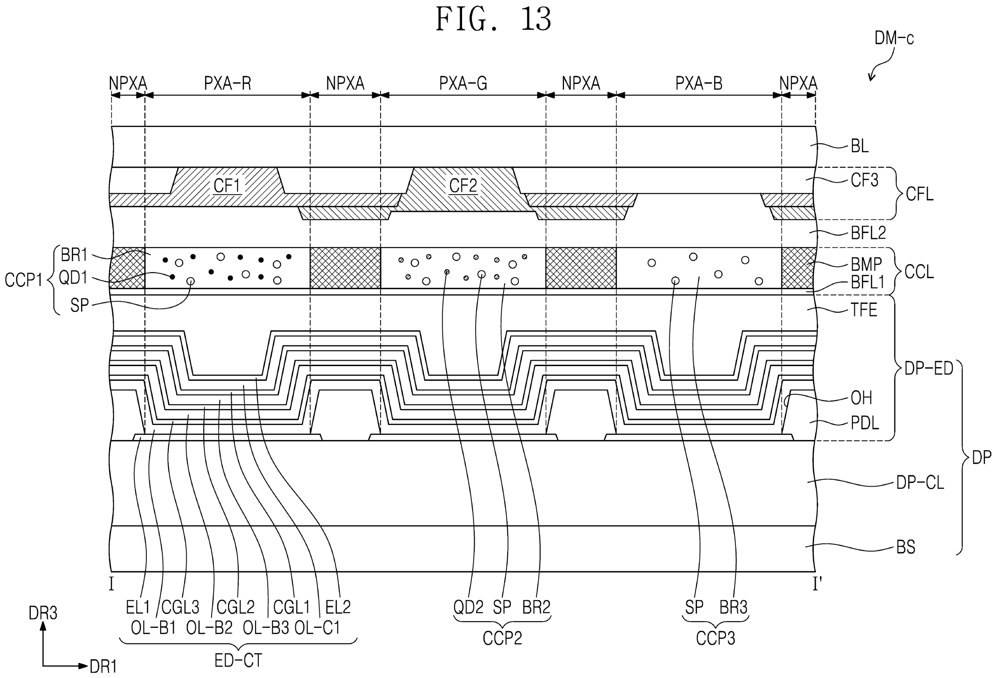

Each of FIGS. 10 to 13 is a cross-sectional view of a display module according to an embodiment of the inventive concept. Hereinafter, in describing the display modules of embodiments with reference to FIGS. 10 to 13, the duplicated features which have been described in FIGS. 1 to 9 are not described again, but their differences will be mainly described.

Referring to FIG. 10, the display module DM-a according to an embodiment may include a display panel DP including a display device layer DP-ED, a light control layer CCL disposed on the display panel DP, and a color filter layer CFL. In an embodiment illustrated in FIG. 10, the display panel DP may include a base layer BS, a circuit layer DP-CL provided on the base layer BS, and the display device layer DP-ED, and the display device layer DP-ED may include a light emitting device ED.

The light emitting device ED may include a first electrode EL1, a hole transport region HTR disposed on the first electrode EL1, an emission layer EML disposed on the hole transport region HTR, an electron transport region ETR disposed on the emission layer EML, and a second electrode EL2 disposed on the electron transport region ETR. The structures of the light emitting devices of FIGS. 5 to 9 as described above may be equally applied to the structure of the light emitting device ED illustrated in FIG. 10.

Referring to FIG. 10, the emission layer EML may be disposed in an opening OH defined in a pixel defining film PDL. For example, the emission layer EML which is divided by the pixel defining film PDL and provided corresponding to each light emitting regions PXA-R, PXA-G, and PXA-B may emit light in the same wavelength range. In the display module DM-a of an embodiment, the emission layer EML may emit blue light. Unlike the configuration illustrated, in an embodiment, the emission layer EML may be provided as a common layer in the entire light emitting regions PXA-R, PXA-G, and PXA-B.

The light control layer CCL may be disposed on the display panel DP. The light control layer CCL may include a light conversion body. The light conversion body may be a quantum dot, a phosphor, or the like. The light conversion body may emit provided light by converting the wavelength thereof. That is, the light control layer CCL may a layer containing the quantum dot or a layer containing the phosphor.

The light control layer CCL may include a plurality of light control parts CCP1, CCP2 and CCP3. The light control parts CCP1, CCP2, and CCP3 may be spaced apart from each other.

Referring to FIG. 10, divided patterns BMP may be disposed between the light control parts CCP1, CCP2 and CCP3 which are spaced apart from each other, but the embodiments of the inventive concept are not limited thereto. FIG. 10 illustrates that the divided patterns BMP do not overlap the light control parts CCP1, CCP2 and CCP3, but at least a portion of the edges of the light control parts CCP1, CCP2 and CCP3 may overlap the divided patterns BMP.

The light control layer CCL may include a first light control part CCP1 containing a first quantum dot QD1 which converts first color light provided from the light emitting device ED into second color light, a second light control part CCP2 containing a second quantum dot QD2 which converts the first color light into third color light, and a third light control part CCP3 which transmits the first color light.

In an embodiment, the first light control part CCP1 may provide red light that is the second color light, and the second light control part CCP2 may provide green light that is the third color light. The third light control part CCP3 may provide blue light by transmitting the blue light that is the first color light provided from the light emitting device ED. For example, the first quantum dot QD1 may be a red quantum dot, and the second quantum dot QD2 may be a green quantum dot. The same as described above may be applied with respect to the quantum dots QD1 and QD2.

In addition, the light control layer CCL may further include a scatterer SP. The first light control part CCP1 may include the first quantum dot QD1 and the scatterer SP, the second light control part CCP2 may include the second quantum dot QD2 and the scatterer SP, and the third light control part CCP3 may not include any quantum dot but include the scatterer SP.

The scatterer SP may be inorganic particles. For example, the scatterer SP may include at least one of TiO₂, ZnO, Al₂O₃, SiO₂, and hollow sphere silica. The scatterer SP may include any one among TiO₂, ZnO, Al₂O₃, SiO₂, and hollow sphere silica, or may be a mixture of at least two materials selected from among TiO₂, ZnO, Al₂O₃, SiO₂, and hollow sphere silica.

The first light control part CCP1, the second light control part CCP2, and the third light control part CCP3 each may include base resins BR1, BR2, and BR3 in which the quantum dots QD1 and QD2 and the scatterer SP are dispersed. In an embodiment, the first light control part CCP1 may include the first quantum dot QD1 and the scatterer SP dispersed in a first base resin BR1, the second light control part CCP2 may include the second quantum dot QD2 and the scatterer SP dispersed in a second base resin BR2, and the third light control part CCP3 may include the scatterer SP dispersed in a third base resin BR3.

The base resins BR1, BR2, and BR3 are media in which the quantum dots QD1 and QD2 and the scatterer SP are dispersed, and may be formed of various resin compositions, which may be generally referred to as a binder. For example, the base resins BR1, BR2, and BR3 may be acrylic-based resins, urethane-based resins, silicone-based resins, epoxy-based resins, *etc.* The base resins BR1, BR2, and BR3 may be transparent resins. In an embodiment, the first base resin BR1, the second base resin BR2, and the third base resin BR3 may be the same as or different from each other.

The light control layer CCL may include a barrier layer BFL1. The barrier layer BFL1 may serve to prevent the penetration of moisture and/or oxygen (hereinafter, referred to as 'moisture/oxygen'). The barrier layer BFL1 may block the light control parts CCP1, CCP2 and CCP3 from being exposed to moisture/oxygen. The barrier layer BFL1 may cover the light control parts CCP1, CCP2, and CCP3. In addition, the barrier layer BFL2 may be provided between the light control parts CCP1, CCP2, and CCP3 and the color filter layer CFL.

The barrier layers BFL1 and BFL2 may include at least one inorganic layer. That is, the barrier layers BFL1 and BFL2 may include an inorganic material. For example, the barrier layers BFL1 and BFL2 may include a silicon nitride, an aluminium nitride, a zirconium nitride, a titanium nitride, a hafnium nitride, a tantalum nitride, a silicon oxide, an aluminium oxide, a titanium oxide, a tin oxide, a cerium oxide, a silicon oxynitride, a metal thin film which secures a transmittance, *etc.* The barrier layers BFL1 and BFL2 may further include an organic film. The barrier layers BFL1 and BFL2 may be formed of a single layer or a plurality of layers.

In the display module DM-a of an embodiment, the color filter layer CFL may be disposed on the light control layer CCL. For example, the color filter layer CFL may be directly disposed on the light control layer CCL. In this case, the barrier layer BFL2 may be omitted.

The color filter layer CFL may include color filters CF1, CF2, and CF3. The color filter layer CFL may include a first filter CF1 configured to transmit the second color light, a second filter CF2 configured to transmit the third color light, and a third filter CF3 configured to transmit the first color light. For example, the first filter CF1 may be a red filter, the second filter CF2 may be a green filter, and the third filter CF3 may be a blue filter. The filters CF1, CF2, and CF3 each may include a polymeric photosensitive resin and a pigment or dye. The first filter CF1 may include a red pigment or dye, the second filter CF2 may include a green pigment or dye, and the third filter CF3 may include a blue pigment or dye.

The embodiments of the inventive concept are not limited thereto, and the third filter CF3 may not include a pigment or dye. The third filter CF3 may include a polymeric photosensitive resin and may not include a pigment or dye. The third filter CF3 may be transparent. The third filter CF3 may be formed of a transparent photosensitive resin.

Furthermore, in an embodiment, the first filter CF1 and the second filter CF2 may be a yellow filter. The first filter CF1 and the second filter CF2 may not be separated but be provided as one filter.

Although not illustrated, the color filter layer CFL may further include a light shielding part (not shown). The light shielding part may be a black matrix. The light shielding part may include an organic light shielding material or an inorganic light shielding material containing a black pigment or dye. The light shielding part may prevent light leakage, and may separate boundaries between the adjacent filters CF1, CF2, and CF3.

The first to third filters CF1, CF2, and CF3 may be disposed corresponding to the red light emitting region PXA-R, the green light emitting region PXA-G, and the blue light emitting region PXA-B, respectively.

A base substrate BL may be disposed on the color filter layer CFL. The base substrate BL may be a member which provides a base surface in which the color filter layer CFL, the light control layer CCL, and the like are disposed. The base substrate BL may be a glass substrate, a metal substrate, a plastic substrate, *etc.* However, the embodiments of the inventive concept are not limited thereto, and the base substrate BL may be an inorganic layer, an organic layer, or a composite material layer. In addition, unlike the configuration illustrated, in an embodiment, the base substrate BL may be omitted.

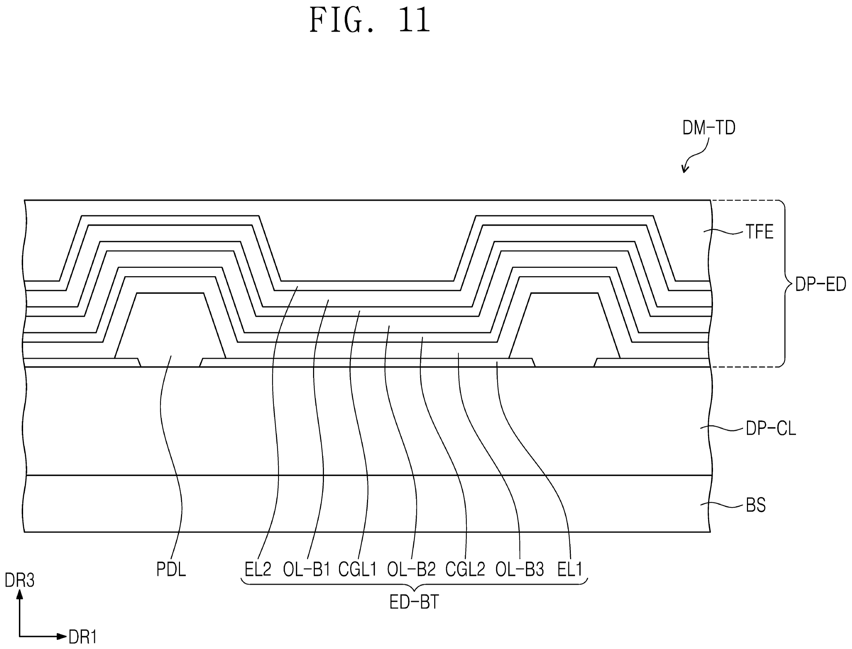

FIG. 11 is a cross-sectional view illustrating a portion of a display module according to an embodiment. In the display module DM-TD of an embodiment, the light emitting device ED-BT may include a plurality of light emitting structures OL-B1, OL-B2, and OL-B3.

At least one of the plurality of light emitting structures OL-B1, OL-B2 and OL-B3 include the polycyclic compound of an embodiment. Accordingly, the light emitting element ED-BT may exhibit high efficiency and long-life characteristics. In addition, the light emitting element ED-BT of an embodiment may exhibit high light efficiency and long-life characteristics in a blue light emitting range. The light emitting device ED-BT may include a first electrode EL1 and a second electrode EL2 which face each other, and the plurality of light emitting structures OL-B1, OL-B2, and OL-B3 sequentially stacked in the thickness direction between the first electrode EL1 and the second electrode EL2. The light emitting structures OL-B1, OL-B2, and OL-B3 each may include an emission layer EML (FIG. 5) and a hole transport region HTR and an electron transport region ETR disposed with the emission layer EML (FIG. 8) located therebetween.

That is, the light emitting device ED-BT included in the display module DM-TD of an embodiment may be a light emitting device having a tandem structure and including a plurality of emission layers.

In an embodiment illustrated in FIG. 11, all light beams respectively emitted from the light emitting structures OL-B1, OL-B2, and OL-B3 may be blue light. However, the embodiments of the inventive concept are not limited thereto, and the light beams respectively emitted from the light emitting structures OL-B1, OL-B2, and OL-B3 may have wavelength ranges different from each other. For example, the light emitting device ED-BT including the plurality of light emitting structures OL-B1, OL-B2, and OL-B3 which emit light beams having wavelength ranges different from each other may emit white light.

Charge generation layers CGL1 and CGL2 may be respectively disposed between two of the neighboring light emitting structures OL-B1, OL-B2, and OL-B3. The charge generation layers CGL1 and CGL2 may include a p-type charge generation layer and/or an n-type charge generation layer.

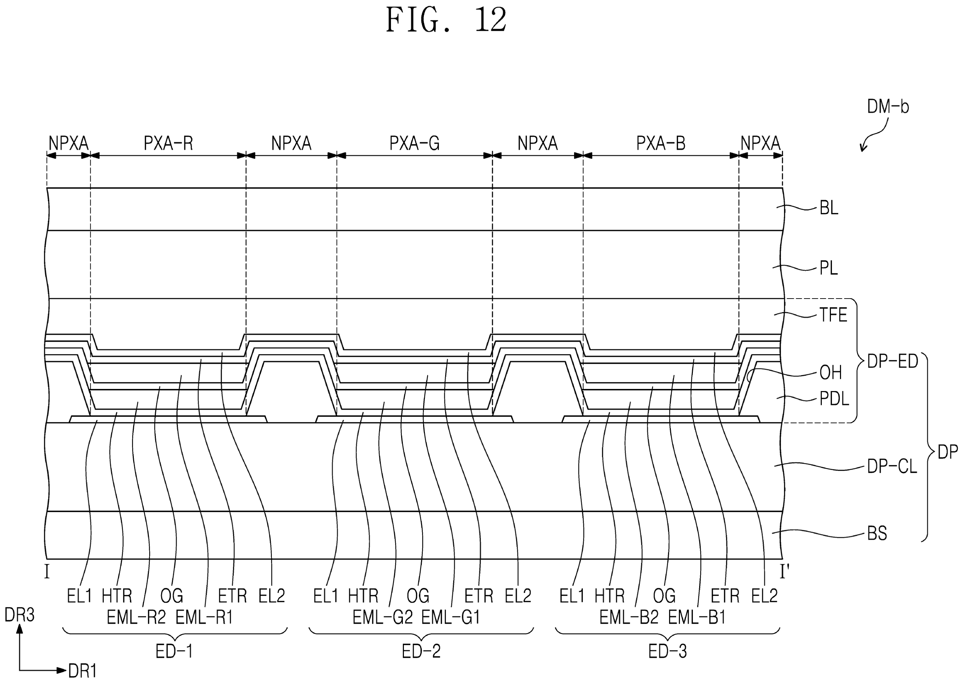

Referring to FIG. 12, the display module DM-b according to an embodiment may include light emitting devices ED-1, ED-2, and ED-3 in which two emission layers are stacked. At least one of the light emitting elements ED-1, ED-2 and ED-3 includes the polycyclic compound of an embodiment. Accordingly, the light emitting elements ED-1, ED-2 and ED-3 may exhibit high efficiency and long-life characteristics. In addition, the light emitting element ED-3 of an embodiment may exhibit high light efficiency and long-life characteristics in a blue light emission range. Compared with the display module DM of an embodiment illustrated in FIG. 3, an embodiment illustrated in FIG. 12 has a difference in that the first to third light emitting devices ED-1, ED-2, and ED-3 each include two emission layers stacked in the thickness direction. In each of the first to third light emitting devices ED-1, ED-2, and ED-3, the two emission layers may emit light in the same wavelength region.

The first light emitting device ED-1 may include a first red emission layer EML-R1 and a second red emission layer EML-R2. The second light emitting device ED-2 may include a first green emission layer EML-G1 and a second green emission layer EML-G2. In addition, the third light emitting device ED-3 may include a first blue emission layer EML-B1 and a second blue emission layer EML-B2. An emission auxiliary part OG may be disposed between the first red emission layer EML-R1 and the second red emission layer EML-R2, between the first green emission layer EML-G1 and the second green emission layer EML-G2, and between the first blue emission layer EML-B1 and the second blue emission layer EML-B2.

The emission auxiliary part OG may include a single layer or a multilayer. The emission auxiliary part OG may include a charge generation layer. More specifically, the emission auxiliary part OG may include an electron transport region ETR, a charge generation layer, and a hole transport region HTR that are sequentially stacked. The emission auxiliary part OG may be provided as a common layer in the whole of the first to third light emitting devices ED-1, ED-2, and ED-3. However, the embodiments of the inventive concept are not limited thereto, and the emission auxiliary part OG may be provided by being patterned within the openings OH defined in the pixel defining film PDL.

The first red emission layer EML-R1, the first green emission layer EML-G1, and the first blue emission layer EML-B1 may be disposed between the emission auxiliary part OG and the electron transport region ETR. The second red emission layer EML-R2, the second green emission layer EML-G2, and the second blue emission layer EML-B2 may be disposed between the hole transport region HTR and the emission auxiliary part OG.

That is, the first light emitting device ED-1 may include the first electrode EL1, the hole transport region HTR, the second red emission layer EML-R2, the emission auxiliary part OG, the first red emission layer EML-R1, the electron transport region ETR, and the second electrode EL2 that are sequentially stacked. The second light emitting device ED-2 may include the first electrode EL1, the hole transport region HTR, the second green emission layer EML-G2, the emission auxiliary part OG, the first green emission layer EML-G1, the electron transport region ETR, and the second electrode EL2 that are sequentially stacked. The third light emitting device ED-3 may include the first electrode EL1, the hole transport region HTR, the second blue emission layer EML-B2, the emission auxiliary part OG, the first blue emission layer EML-B1, the electron transport region ETR, and the second electrode EL2 that are sequentially stacked.

An optical auxiliary layer PL may be disposed on the display device layer DP-ED. The optical auxiliary layer PL may include a polarizing layer. The optical auxiliary layer PL may be disposed on the display panel DP and control reflected light in the display panel DP due to external light. Unlike the configuration illustrated, the optical auxiliary layer PL in the display apparatus according to an embodiment may be omitted.

Unlike FIGS. 11 and 12, FIG. 13 illustrates that a display module DM-c includes four light emitting structures OL-B1, OL-B2, OL-B3, and OL-C1. A light emitting device ED-CT may include a first electrode EL1 and a second electrode EL2 which face each other, and first to fourth light emitting structures OL-B1, OL-B2, OL-B3, and OL-C1 that are sequentially stacked in the thickness direction between the first electrode EL1 and the second electrode EL2. At least one of the first to fourth light emitting structures OL-B1, OL-B2, OL-B3 and OL-C1 includes the polycyclic compound of an embodiment. Accordingly, the light emitting element ED-CT may exhibit high efficiency and long-life characteristics. In addition, the light emitting element ED-CT of an embodiment may exhibit high light efficiency and long-life characteristics in a blue light emitting range.

Charge generation layers CGL1, CGL2, and CGL3 may be disposed between the first to fourth light emitting structures OL-B1, OL-B2, OL-B3, and OL-C1. Among the four light emitting structures, the first to third light emitting structures OL-B1, OL-B2, and OL-B3 may emit blue light, and the fourth light emitting structure OL-C1 may emit green light. However, the embodiments of the inventive concept are not limited thereto, and the first to fourth light emitting structures OL-B1, OL-B2, OL-B3, and OL-C1 may emit light beams in different wavelength regions.

The charge generation layers CGL1, CGL2, and CGL3 disposed between adjacent light emitting structures OL-B1, OL-B2, OL-B3, and OL-C1 may include a p-type charge generation layer and/or an n-type charge generation layer.

In an embodiment, an electronic device may include a display device including a plurality of light emitting elements, and a control part for controlling the display device. In an embodiment, in the electronic device, at least one of the plurality of light emitting elements may include the polycyclic compound of an embodiment described above in the light emitting layer.

The electronic device of an embodiment may be a device activated according to electrical signals. The electronic device of an embodiment may include display devices including the display modules according to embodiments described with reference to FIG. 3, FIGS. 10 to 13, or the like. For example, the electronic device may include large-size display devices such as televisions, monitors and outside billboards, and medium- and small-size display devices such as personal computers, laptop computers, personal digital terminals, display devices for automobiles, game consoles, portable electronic devices, and cameras.

The electronic device of an embodiment includes a display module including the polycyclic compound of an embodiment, and may exhibit high efficiency and long-life characteristics. The electronic device of an embodiment may have improved display efficiency and display lifespan and may exhibit excellent display quality.

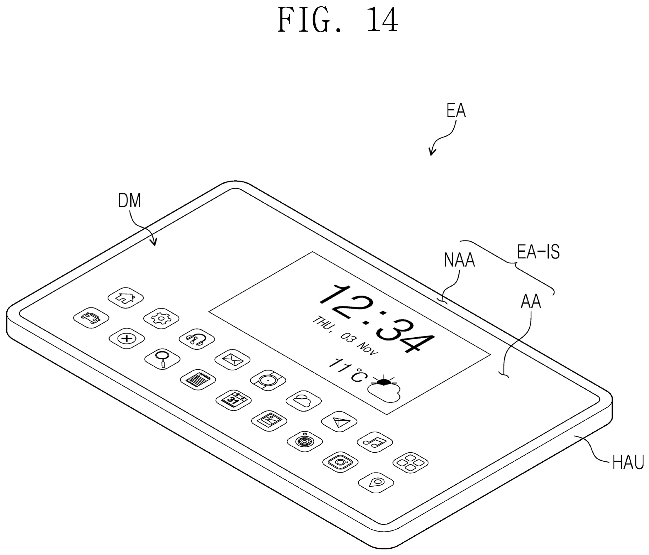

FIG. 14 illustrates a tablet terminal as an embodiment of an electronic device EA. The electronic device EA of an embodiment may include a display module DM according to an embodiment. For example, electronic modules, a camera module, a power module, or the like, mounted on a main board may be disposed in a bracket/housing HAU together with the display module DM to configure a tablet terminal.

The electronic device EA of an embodiment illustrated in FIG. 14 may include the display module of an embodiment described with reference to FIG. 3, FIGS. 10 to 13, or the like.

In an embodiment, an electronic device EA including a display module DM having a flat display surface is shown, but an embodiments of the inventive concept are not limited thereto. The electronic device EA may also include a curved display surface or a three-dimensional display surface. For example, a three-dimensional display surface may include a plurality of display areas pointing in different directions and may also include a bent display surface. The electronic device EA according to this embodiment may be a flexible electronic device. The flexible electronic device may be a foldable electronic device that is foldable.

As shown in FIG. 14, a display surface EA-IS includes an active area AA on which an image is displayed and a bezel area NAA adjacent to the active area AA. The bezel area NAA is an area where an image is not displayed. FIG. 14 illustrates icon images as embodiments of the image. The active area AA may be referred to as a display area of the display module DM, and the bezel area NAA may be referred to as a non-display area of the display module DM.

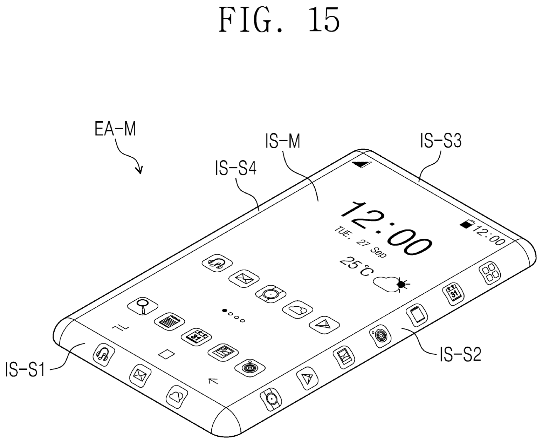

FIG. 15 illustrates a portable terminal as an embodiment of an electronic device EA-M according to an embodiment. Referring to FIG. 15, the electronic device EA-M according to an embodiment may include a plurality of display surfaces. The electronic device EA-M of an embodiment may include display surfaces IS-M, IS-S1, IS-S2, IS-S3 and IS-S4, having different main display directions.

In an embodiment, the electronic device EA-M may be a three-dimensional display device including an upper display surface IS-M and a plurality of side display surfaces IS-S1, IS-S2, IS-S3 and IS-S4. Each of the plurality of side display surfaces IS-S1, IS-S2, IS-S3 and IS-S4 may be a display surface extending from one side of the upper display surface IS-M. In an embodiment, the electronic device EA-M may include a main display surface that mainly provides images in one direction and a plurality of sub-display surfaces that provide images in a direction different from the one direction. In an embodiment of the electronic device EA-M illustrated in FIG. 15, the main display surface may be the upper display surface IS-M, and the sub-display surfaces may be the side display surfaces IS-S1, IS-S2, IS-S3 and IS-S4.

The side display surfaces IS-S 1, IS-S2, IS-S3 and IS-S4 may have display surfaces that are not parallel to the upper display surface IS-M. The plurality of side display surfaces IS-S1, IS-S2, IS-S3 and IS-S4 may be display areas that are each bent and extended from one side of the upper display surface IS-M, and for example, the plurality of side display surfaces IS-S1, IS-S2, IS-S3 and IS-S4 may be bending display areas.

The electronic device EA-M illustrated in FIG. 15 may include the display modules according to embodiments described with reference to FIG. 3, FIGS. 10 to 13, or the like.

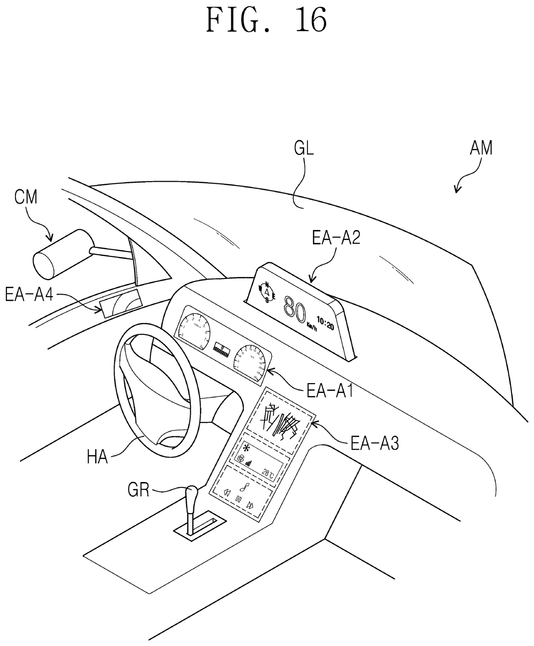

FIG. 16 is a view illustrating a vehicle AM in which first to fourth electronic apparatuses EA-A1, EA-A2, EA-A3, and EA-A4 are disposed. At least one among the first to fourth electronic apparatuses EA-A1, EA-A2, EA-A3, and EA-A4 may include the same configuration as the display modules DM, DM-TD, DM-a, DM-b, and DM-c as described with reference to FIGS. 3, and 4, and 10 to 13.

FIG. 16 illustrates a vehicle AM, but this is an example, and the first to fourth electronic apparatuses EA-A1, EA-A2, EA-A3, and EA-A4 may be disposed in another transportation means such as bicycles, motorcycles, trains, ships, and airplanes. In addition, at least one among the first to fourth electronic apparatuses EA-A1, EA-A2, EA-A3, and EA-A4 including the same configuration as the display modules DM, DM-TD, DM-a, DM-b, and DM-c of an embodiment may be employed in a personal computer, a laptop computer, a personal digital terminal, a game console, a portable electronic device, a television, a monitor, an outdoor billboard, or the like. In addition, these are merely provided as embodiments, and thus may be employed in other electronic apparatuses unless departing from the inventive concept.

At least one among the first to fourth electronic apparatuses EA-A1, EA-A2, EA-A3, and EA-A4 may include the light emitting device ED of an embodiment as described with reference to FIGS. 5 to 9.

Referring to FIG. 16, the vehicle AM may include a steering wheel HA and a gear GR for driving the vehicle AM. In addition, the vehicle AM may include a front window GL disposed so as to face the driver.

The first electronic apparatus EA-A1 may be disposed in a first region overlapping the steering wheel HA. For example, the first electronic apparatus EA-A1 may be a digital cluster which displays first information of the vehicle AM. The first information may include a first scale which indicates a driving speed of the vehicle AM, a second scale which indicates an engine speed (that is, revolutions per minute (RPM)), an image which indicates a fuel state, *etc.* A first scale and a second scale may be indicated as a digital image.

The second electronic apparatus EA-A2 may be disposed in a second region facing the driver's seat and overlapping the front window GL. The driver's seat may be a seat in which the steering wheel HA is disposed. For example, the second electronic apparatus EA-A2 may be a head up display (HUD) which displays second information of the vehicle AM. The second electronic apparatus EA-A2 may be optically transparent. The second information may include digital numbers which indicate a driving speed, and may further include information such as the current time. Unlike the configuration illustrated, the second information of the second electronic apparatus EA-A2 may be projected to the front window GL to be displayed.

The third electronic apparatus EA-A3 may be disposed in a third region adjacent to the gear GR. For example, the third electronic apparatus EA-A3 may be disposed between the driver's seat and the passenger seat and may be a center information display (CID) for a vehicle for displaying third information. The passenger seat may be a seat spaced apart from the driver's seat with the gear GR disposed therebetween. The third information may include information about traffic (*e.g.*, navigation information), playing music or radio or a video (or an image), temperatures inside the vehicle AM, *etc.*

The fourth electronic apparatus EA-A4 may be spaced apart from the steering wheel HA and the gear GR, and may be disposed in a fourth region adjacent to the side of the vehicle AM. For example, the fourth electronic apparatus EA-A4 may be a digital side-view mirror which displays fourth information. The fourth electronic apparatuse EA-A4 may display an image outside the vehicle AM taken by a camera module CM disposed outside the vehicle AM. The fourth information may include an image outside the vehicle AM.

The above-described first to fourth information may be examples, and the first to fourth electronic apparatuses EA-A1, EA-A2, EA-A3, and EA-A4 may further display information about the inside and outside of the vehicle AM. The first to fourth information may include different information. However, the embodiments of the inventive concept are not limited thereto, and a part of the first to fourth information may include the same information as one another.

FIGS. 14 to 16 present embodiments of electronic devices or embodiments including electronic devices, and the display module including the light emitting element including the polycyclic compound of an embodiment may be employed in other electronic devices without departing from the concept of the inventive concept.

Hereinafter, the polycyclic compound according to an embodiment of the inventive concept and the light emitting element of an embodiment will be specifically described with reference to examples and comparative examples. In addition, the examples shown below are examples to help understanding of the inventive concept, and the scope of the inventive concept is not limited thereto.

### Examples

### 1. Example Preparation

The preparation method of the polycyclic compound according to an embodiment of the inventive concept will be explained in particular, illustrating the preparation methods of Example 1 to Example 17. In addition, the preparation methods of the Examples explained hereinafter are embodiments, and the synthetic method of the compound according to an embodiment of the inventive concept is not limited to the examples below.

### (1) Preparation of Example 1

### (Synthesis of Reactant 10-1)

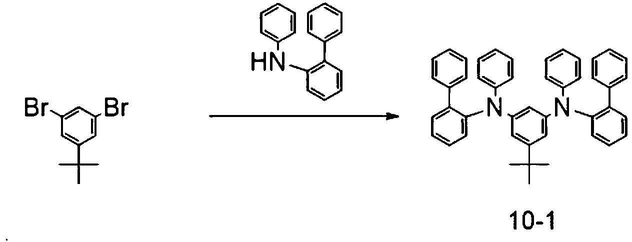

Under an Ar atmosphere, C₆H₃-1,3-Br₂-5-*t*Bu (29.0 g), PhNH(C₆H₄-2-Ph) (51.5 g), Pd₂(dba)₃ (6.1 g), P(*t*Bu)₃HBF₄ (9.2 g), and NaOtBu (25.0 g) were added to a 2000 mL three-necked flask and dissolved in toluene (700 mL), followed by heating and reacting at about 90 °C for about 3 hours. After returning the reaction mixture to room temperature, water was added, and the product was extracted with CH₂Cl₂. An organic layer was dried over MgSO₄, and the solvent was removed under reduced pressure. The product thus obtained was purified by silica gel column chromatography to obtain Reactant 10-1 (50.2 g, yield 87%). The mass number of Reactant 10-1, determined by FAB-MS, was 620.

### (Synthesis of Compound 10)

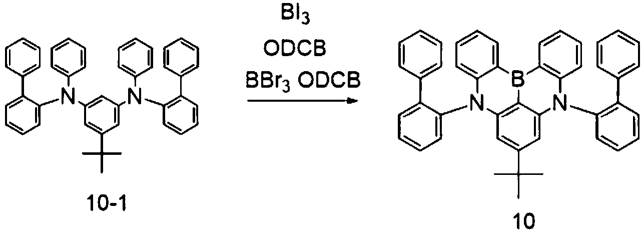

A light emitting layer composition including a first material, a second material, and a third material was prepared in a 100 mL three-necked flask. Reactant 10-1 (3.25 g, 5.24 mmol) of the first material was put in the 100 mL three-necked flask, with a condenser and a thermometer attached. After putting the reaction vessel under an argon (Ar) stream, the second material, BI₃ (4.10 g, 10.5 mmol), 1,2-dichlorobenzene (ODCB) (10.5 mL), and the third material, 2 M BBr₃ ODCB solution (15.7 mL, 31.4 mmol) were added, and the reaction vessel was heated in an oil bath (about 150 °C) and stirred for about 15 hours while the internal temperature of the reaction solution was about 140 °C. The progress of the reaction was tracked by thin layer chromatography (TLC). Upon completion the reaction solution was cooled to room temperature, N,N-diisopropylethylamine (DIPEA) (29 mL, 168 mmol) was added, and the product mixture was stirred for about 5 minutes. Toluene (30 mL) and H₂O (30 mL) were added, and the resultant mixture was stirred for about 20 minutes.

1.0 mL of the resulting organic layer was removed, concentrated using an evaporator, and dissolved in CDCl₃. The yield was calculated by measuring the ¹H NMR spectrum (NMR yield: 55%). The NMR yield was calculated by comparing with the ¹H NMR peak area of 2,6-diisopropylnaphthalene, which was added as an internal standard. The reaction solution was transferred to a 1 L separatory flask and extracted four times with toluene (50 mL). An organic layer obtained was washed three times with H₂O (50 mL) and dried over MgSO₄. The dried organic layer was filtered from the drying agent with a silica gel pad and concentrated using an evaporator. The product obtained was dissolved in dichloromethane (300 mL) and purified by silica gel column chromatography (eluent: dichloromethane/hexane = 1/4) to obtain Compound 10 (1.54 g, 2.46 mmol, 47%). The mass number of Compound 10, as determined by FAB-MS, was 628. The purity of Compound 10, as determined by high-performance liquid chromatography (HPLC), was 98.0%.

### (2) Preparation of Examples 2 to 12

The polycyclic compounds of Examples 2 to 12 were prepared according to the experimental conditions in Table 1. The polycyclic compounds of Examples 2 to 12 were prepared using the same method as in Example 1, except for the experimental conditions of the equivalent of the second material, the equivalent of the third material, the reaction temperature, and the reaction time as indicated in Table 1. In Table 1, the reaction temperature refers to the heating temperature for the reaction vessel containing the light emitting layer composition, and the reaction time refers to the time for stirring the light emitting layer composition at the reaction temperature.

### (3) Preparation of Example 13

### (Synthesis of Reactant 15-1)

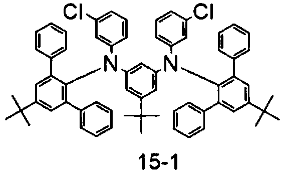

Reactant 15-1 was synthesized according to the method disclosed in document US20240343743.

### (Synthesis of Compound 15)

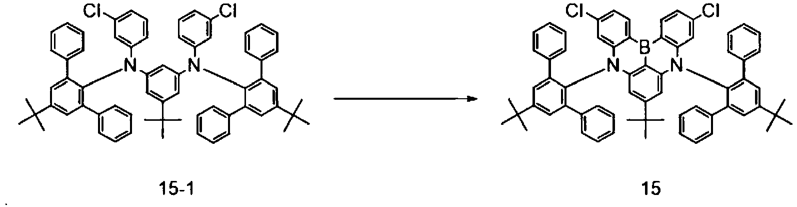

Compound 15 was synthesized in the same manner as the synthesis of Compound 10 described above, except that Reactant 15-1 (4.99 g, 5.24 mmol) of the first material was used. The mass number of Compound 15 determined by FAB-MS measurement was 960. The yield of Compound 15 was calculated by measuring the ¹H NMR spectrum in the same manner as for Compound 10 (NMR yield: 57%).

### (4) Preparation of Example 14

### (Synthesis of Reactant 22-1)

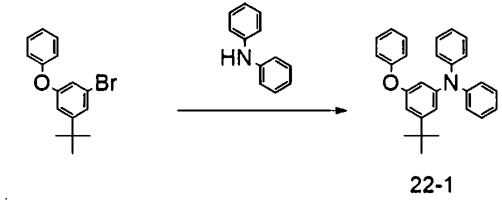

Under an Ar atmosphere, C₆H₃-1-Br-3-OPh-5-*t*Bu (30.4 g) (CAS#: 2699631-66-6), Ph₂NH (20.1 g), Pd₂(dba)₃ (3.1 g), P(*t*Bu)₃HBF₄ (4.6 g), and NaOtBu (11.0 g) were added to a 2000 mL three-necked flask and dissolved in toluene (500 mL), followed by heating and reacting at about 90 °C for about 4 hours. Thereafter, the reaction mixture is brought to room temperature, water was added, and the product was extracted with CH₂Cl₂. An organic layer was dried over MgSO₄, and the solvent was distilled off under reduced pressure. The product thus obtained was purified by silica gel column chromatography to obtain Reactant 22-1 (35.8 g, yield 91%). The mass number of Reactant 22-1, determined by FAB-MS, was 393.

### (Synthesis of Compound 22)

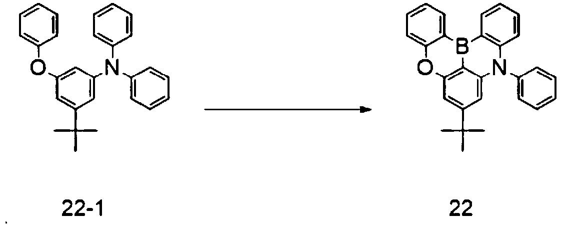

Compound 22 was synthesized using the same method as for Compound 10, except that Reactant 22-1 (2.06 g, 5.24 mmol) of the first material was used. The mass number of Compound 22, as determined by FAB-MS, was 401. The yield of Compound 22 was calculated by measuring the ¹H NMR spectrum in the same manner as for Compound 10 (NMR yield: 43%).

### (5) Preparation of Example 15

### (Synthesis of Reactant 23-1)

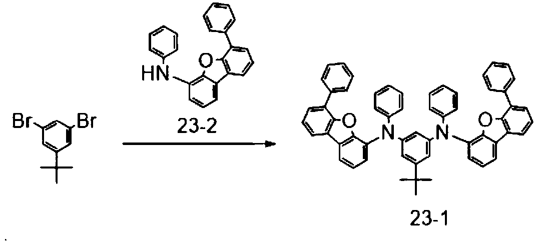

Reactant 23-1 was synthesized using the same method as Reactant 10-1, using C₆H₃-1,3-Br₂-5-*t*Bu (29.0 g) and 23-2 (70.5 g) (CAS#: 1438401-26-3) to obtain 60.1 g (75% yield) of Reactant 23-1. The mass number of Reactant 23-1 measured by FAB-MS was 800.

### (Synthesis of Compound 23)

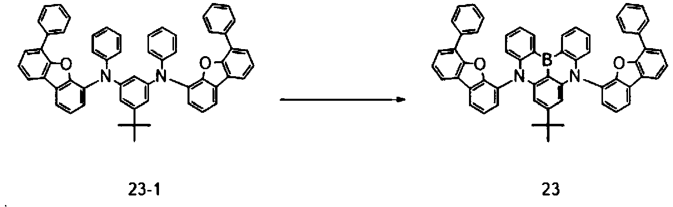

Compound 23 was synthesized using the same method as for Compound 10, except that Reactant 23-1 (4.20 g, 5.24 mmol) of the first material was used. The mass number of Compound 23 measured by FAB-MS was 808. The yield of Compound 23 was calculated by measuring the ¹H NMR spectrum in the same manner as for Compound 10 (NMR yield: 65%).

### (6) Preparation of Example 16

### (Synthesis of Reactant 24-1)

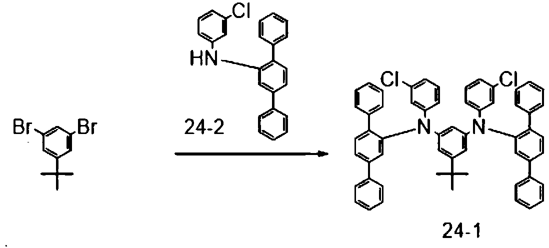

Reactant 24-1 was synthesized using the same method as Reactant 10-1, using C₆H₃-1,3-Br₂-5-*t*Bu (29.0 g) and Intermediate 24-2 (74.6 g) to obtain Reactant 24-1 (58.8 g, 70% yield). The mass number of Reactant 24-1 measured by FAB-MS was 800.

### (Synthesis of Compound 24)

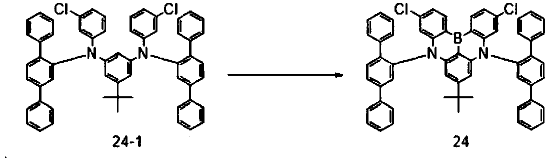

Compound 24 was synthesized using the same method as for Compound 10, except that Reactant 24-1 (4.41 g, 5.24 mmol) of the first material was used. The mass number of Compound 24 measured by FAB-MS was 848. The yield of Compound 24 was calculated by measuring the ¹H NMR spectrum in the same manner as for Compound 10 (NMR yield: 66%).

### (7) Preparation of Example 17

### (Synthesis of Reactant 25-1)

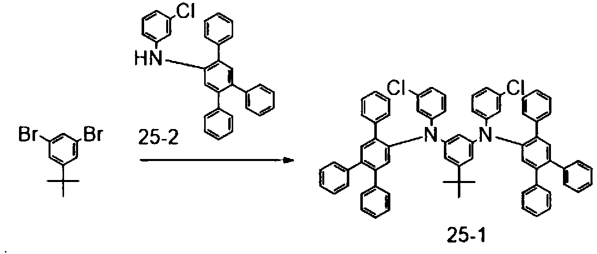

Reactant 25-1 was synthesized using the same method as Reactant 10-1, using C₆H₃-1,3-Br₂-5-*t*Bu (29.0 g) and 25-2 (90.6 g) to obtain Reactant 25-1 (165.4 g, 66% yield). The mass number of Reactant 25-1 determined by FAB-MS was 800.

### (Synthesis of Compound 25)

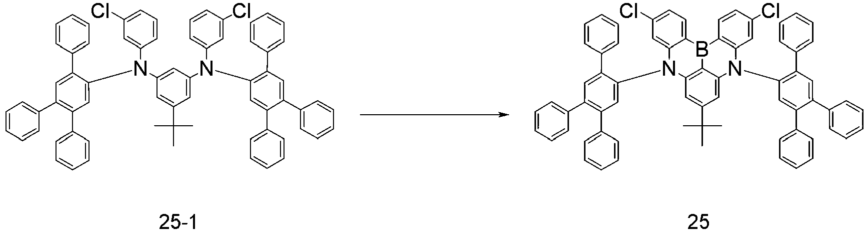

Compound 25 was synthesized using the same method as for Compound 10, except that Reactant 25-1 (5.21 g, 5.24 mmol) of the first material was used. The mass number of Compound 25 measured by FAB-MS was 1000. The yield of Compound 25 was calculated by measuring the ¹H NMR spectrum in the same manner as for Compound 10 (NMR yield: 67%).

### (8) Preparation of Comparative Examples

According to the experimental conditions in Table 1 below, polycyclic compounds of Comparative Examples 1 to 12 were prepared.

The second material used in Comparative Examples 1 to 3 and Comparative Examples 5 to 12 included BI₃, and the third material used in Comparative Example 4 included BBr₃.

Comparative Examples 1, 2, and 3 differ from the preparation method in Example 1 in that the third material was not included in the light emitting layer composition. Comparative Example 4 differs from the preparation method in Example 1 in that the second material was not included in the light emitting layer composition. Comparative Examples 5 to 12 differ from the preparation method in Example 1 in that the fourth material was included in the light emitting layer composition. Except for the differences, the compounds were prepared using the same method as Example 1.

Comparative Example 5 used pyridine as the fourth material, Comparative Example 6 used NaCl as the fourth material, Comparative Example 7 used Ag₂O as the fourth material, Comparative Example 8 used 2-chloro-1,3-bis(2,6-diisopropylphenyl)-1H-imidazolium chloride (NHC) as the fourth material, Comparative Example 9 used I₂ as the fourth material, Comparative Example 10 used N,N-diisopropylethylamine (DIPEA) as the fourth material, Comparative Example 11 used triphenylborane (BPh₃) as the fourth material, and Comparative Example 12 used 1,8-bis(dimethylamino)naphthalene (DMAN) as the fourth material.

**Table 1**

| Example | Light emitting layer composition | | | Polycyclic comp. | R_{X} temp. (°C) | Rx time (h) |
|---|---|---|---|---|---|---|
| | 1st Material Reactant | 2^{nd} / 3^{rd} materials (eq/eq) | 4^{th} material (eq) | | | |
| Ex. 1 | 10-1 | 2/6 | - | Comp. 10 | 140 | 15 |
| Ex. 2 | 10-1 | 8/8 | - | Comp. 10 | 140 | 11 |
| Ex. 3 | 10-1 | 4/4 | - | Comp. 10 | 140 | 4 |
| Ex. 4 | 10-1 | 3/3 | - | Comp. 10 | 140 | 24 |
| Ex. 5 | 10-1 | 3/2 | - | Comp. 10 | 140 | 15 |
| Ex. 6 | 10-1 | 3/1 | - | Comp. 10 | 140 | 15 |
| Ex. 7 | 10-1 | 2/4 | - | Comp. 10 | 140 | 15 |
| Ex. 8 | 10-1 | 2/4 | - | Comp. 10 | 110 | 11 |
| Ex. 9 | 10-1 | 2/3 | - | Comp. 10 | 140 | 12 |
| Ex. 10 | 10-1 | 2/2 | - | Comp. 10 | 110 | 24 |
| Ex. 11 | 10-1 | 1.5/4.5 | - | Comp. 10 | 140 | 15 |
| Ex. 12 | 10-1 | 1/3 | - | Comp. 10 | 140 | 21 |
| Ex. 13 | 15-1 | 2/6 | - | Comp. 15 | 140 | 15 |
| Ex. 14 | 22-1 | 2/6 | - | Comp. 22 | 150 | 15 |
| Ex. 15 | 23-1 | 2/4 | - | Comp. 23 | 150 | 15 |
| Ex. 16 | 24-1 | 2/5 | - | Comp. 24 | 130 | 15 |
| Ex. 17 | 25-1 | 2/5 | - | Comp. 25 | 130 | 15 |
| Comp. Ex. 1 | 10-1 | 4/0 | - | Comp. 10 | 140 | 24 |
| Comp. Ex. 2 | 10-1 | 3/0 | - | Comp. 10 | 140 | 24 |
| Comp. Ex. 3 | 10-1 | 2/0 | - | Comp.10 | 140 | 24 |
| Comp. Ex. 4 | 10-1 | 0/4 | - | Comp. 10 | 150 | 12 |
| Comp. Ex. 5 | 10-1 | 2/0 | 2 | Comp. 10 | 140 | 15 |
| Comp. Ex. 6 | 10-1 | 2/0 | 2 | Comp. 10 | 140 | 15 |
| Comp. Ex. 7 | 10-1 | 2/0 | 2 | Comp. 10 | 140 | 15 |
| Comp. Ex. 8 | 10-1 | 2/0 | 2 | Comp. 10 | 140 | 15 |
| Comp. Ex. 9 | 10-1 | 2/0 | 2 | Comp. 10 | 140 | 15 |
| Comp. Ex. 10 | 10-1 | 2/0 | 2 | Comp. 10 | 140 | 15 |
| Comp. Ex. 11 | 10-1 | 2/0 | 2 | Comp. 10 | 140 | 15 |
| Comp. Ex. 12 | 10-1 | 2/0 | 3 | Comp. 10 | 140 | 15 |

### 2. Example Evaluation

Table 2 below shows the yields of the compounds of the Examples 1 to 17 and Comparative Examples 1 to 12. Table 2 shows the NMR yields of the compounds of the Examples 1 to 17 and Comparative Examples 1 to 12, and the relative yields are shown by setting the NMR yield of Comparative Example 1 to 1.00. The NMR yields were measured by the measurement methods in the respective preparation methods of the above-described Examples 1, and 13 to 17.

**Table 2**

| | NMR yield (%) | Relative yield |
|---|---|---|
| Example 1 | 55 | 2.20 |
| Example 2 | 36 | 1.44 |
| Example 3 | 36 | 1.44 |
| Example 4 | 39 | 1.56 |
| Example 5 | 37 | 1.48 |
| Example 6 | 35 | 1.40 |
| Example 7 | 50 | 2.00 |
| Example 8 | 41 | 1.64 |
| Example 9 | 40 | 1.60 |
| Example 10 | 38 | 1.52 |
| Example 11 | 41 | 1.64 |
| Example 12 | 33 | 1.32 |
| Example 13 | 57 | 2.28 |
| Example 14 | 43 | 1.72 |
| Example 15 | 65 | 2.60 |
| Example 16 | 66 | 2.64 |
| Example 17 | 67 | 2.68 |
| Comp. Ex. 1 | 25 | 1.00 |
| Comp. Ex. 2 | 29 | 1.16 |
| Comp. Ex. 3 | 25 | 1.00 |
| Comp. Ex. 4 | 0 | 0 |
| Comp. Ex. 5 | 14 | 0.56 |
| Comp. Ex. 6 | 21 | 0.84 |
| Comp. Ex. 7 | 0 | 0 |
| Comp. Ex. 8 | 0 | 0 |
| Comp. Ex. 9 | 0 | 0 |
| Comp. Ex. 10 | 10 | 0.40 |
| Comp. Ex. 11 | 3 | 0.12 |
| Comp. Ex. 12 | 4 | 0.16 |

Referring to the results in Table 2, the polycyclic compound preparation methods of the Examples 1 to 17 showed increased NMR yields compared to the polycyclic compound preparation methods of the Comparative Examples 1 to 12. In particular, in light of the yield values in Examples 1 to 12, it can be found that under experimental conditions where the second material includes BI₃ and the third material includes BBr₃, a relatively higher yield may be obtained if the amount of the third material increases to the amount of the second material, i.e., a decrease in the ratio second/third material. For example see, the relative yields of Example 1 to Examples 2 and 3 or Example 7 to Example 4. It is thought that the yield of the polycyclic compound preparation method according to an embodiment of the inventive concept is improved by mixing both the second material and the third material into the light emitting layer composition. Specifically, it is thought that the yield is improved because the inclusion of both BI₃ and BBr₃ in the light emitting layer composition generates BI₂Br and BIBr₂, which exhibit higher reaction activity.

Referring to Tables 1 and 2, it can be found that Comparative Examples 1 to 4 have relatively lower yield values compared to the Examples 1 to 17. It is thought that the yields were relatively low because neither the second nor third material was included in the light emitting layer compositions when preparing the polycyclic compounds of Comparative Examples 1 to 4.

Comparative Examples 5 to 12 were found to have relatively low yields compared to the Examples 1 to 17. It is thought that the yields were relatively low because the light emitting layer compositions of Comparative Examples 5 to 12 included the second material but did not include a third material different from the second material. Furthermore, although the light emitting layer compositions of Comparative Examples 5 to 12 included the fourth material, the yield increase effect was not achieved. Even when preparing the polycyclic compounds of Comparative Examples 5 to 12, the addition of BPh₃, a Brønsted base, and a neutral salt separately included in the light emitting layer compositions did not improve the yield of the preparation method, and in many cases, the reaction was inhibited.

### 3. Manufacture and Evaluation of Light Emitting Elements

### (1) Manufacture of Light Emitting Elements

A light emitting element including a polycyclic compound prepared according to the preparation method of a polycyclic compound of an embodiment was manufactured using the following method. The light emitting elements of Examples 1 to 6 were manufactured using the polycyclic compounds as dopant materials in the light emitting layer.

A glass substrate patterned with about 150 nm thick ITO as the first electrode was ultrasonically cleaned using isopropyl alcohol and pure water for about 5 minutes each. After ultrasonic cleaning, the substrate was UV-irradiated for about 30 minutes and then ozone-treated. HAT-CN was sequentially deposited to a thickness of about 10 nm, α-NPD to a thickness of about 80 nm, and mCP to a thickness of about 5 nm to form a hole transport region.

Next, the Example Compound or the Comparative Compound was co-deposited with mCBP to form a light emitting layer with a thickness of about 20 nm. The Example Compound or the Comparative Compound and mCBP were co-deposited at a weight ratio of about 1:99. In the manufacture of light emitting elements, the Example Compounds or Comparative Compounds were used as dopant materials. TPBi was sequentially deposited to a thickness of about 30 nm and LiF to a thickness of about 0.5 nm to form an electron transport region. Al was deposited to a thickness of about 100 nm to form a second electrode.

In the Example Light Emitting Element, the hole transport region, the light emitting layer, the electron transport region, and the second electrode were formed using a vacuum deposition apparatus.

The compounds used in the light emitting layers of the Examples 1 to 6 are shown in Table 3 below.

**Table 3**

| Division | Compound | Division | Compound |
|---|---|---|---|
| Element Example 1 | 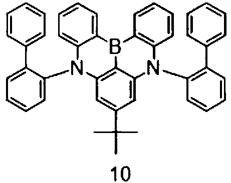 | Element Example 2 | 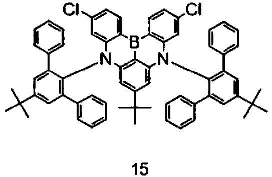 |
| Element Example 3 | 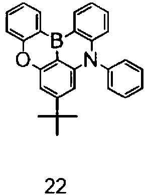 | Element Example 4 | 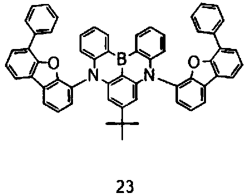 |
| Element Example 5 | 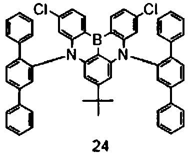 | Element Example 6 | 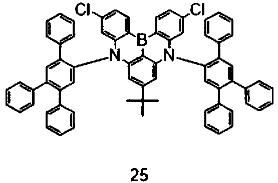 |

Besides, the compounds used for the manufacture of the light emitting elements are as follows.

### (Materials used for the manufacture of light emitting elements)

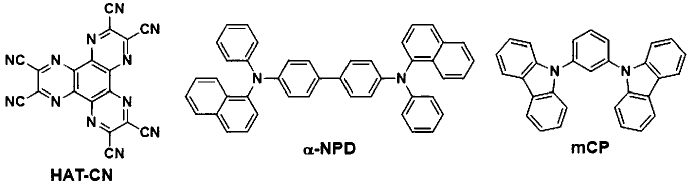

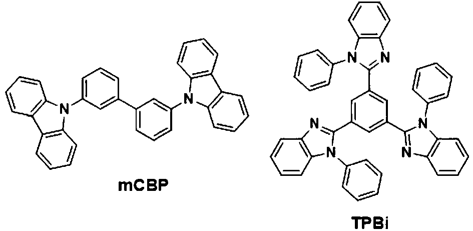

### (2) Evaluation of Light Emitting Elements

Table 4 below shows the evaluation results of the light emitting elements of the Examples 1 to 6. Table 4 also lists the color of the emitted light of the Examples 1 to 6.

**Table 4**

| Element Mfg, Ex. | Dopant material | Color emitted light |
|---|---|---|
| Element Ex. 1 | Comp. 10 | Blue light |
| Element Ex. 2 | Comp. 15 | Blue light |
| Element Ex. 3 | Comp. 22 | Blue light |
| Element Ex. 4 | Comp. 23 | Blue light |
| Element Ex. 5 | Comp. 24 | Blue light |
| Element Ex. 6 | Comp. 25 | Blue light |

Referring to the results in Table 4, the light emitting elements of the Examples 1 to 6 include the polycyclic compound prepared according to the preparation method of a polycyclic compound of an embodiment of the present disclosure as a dopant material in the light emitting layer. Accordingly, it can be confirmed that blue light is emitted.

Referring to Tables 1 to 4, the method for manufacturing a polycyclic compound in accordance with the described preparation method may relatively increase the product yield by including the described second material and the third material in the light emitting layer composition. In addition, the light emitting element including the polycyclic compound manufactured through the method may emit blue light.

In the case of the many of the above dopants present in a blue light emitting layer there were problems in that the manufacturing product yield was relatively low, e.g., about 30% or less, or high-temperature experimental conditions of reaction temperatures of about 200 °C or higher were required to secure a yield above a certain level. In contrast, the method of manufacturing a polycyclic compound of an embodiment may form the polycyclic compound even under experimental conditions below about 200 °C by including both the second material and the third material in the light emitting layer composition. In particular, the method of manufacturing the polycyclic compounds of Examples 8 and 10 may be prepared at reaction temperatures below about 120 °C by controlling the reaction time. Furthermore, the method of manufacturing the polycyclic compound of an embodiment may achieve a yield of about 30% or more by including the second and third materials as the light emitting layer composition, thereby increasing the reaction activity (or efficiency). In particular, the method of manufacturing the polycyclic compounds of Examples 1, 13, 15, 16, and 17 may obtain yields of about 50% or more. For example, it is thought that the method of manufacturing the polycyclic compound of an embodiment includes both BI₃ and BBr₃ as the light emitting layer composition, and the resulting generation of BI₂Br and BIBr₂ is believed to improve upon the reaction activity and achieving the effect of increasing the yield.

The light emitting element of an embodiment, which includes the polycyclic compound prepared according to the polycyclic compound preparation method of an embodiment in the light emitting layer, emits light in the blue light emission range and may have improved efficiency during manufacture. Additionally, an electronic device including the light emitting element of an embodiment may have improved efficiency during manufacturing.

The method of manufacturing a polycyclic compound for a light emitting element according to an embodiment may exhibit improved process efficiency.

The light emitting element of an embodiment and an electronic device including the same may exhibit excellent display quality and improved reliability characteristics.

In the above, description has been made with reference to embodiments of the inventive concept, but those skilled or of ordinary skill in the art may understand that various modifications and changes may be made to the inventive concept insofar as such modifications and changes do not depart from the technical scope of the inventive concept set forth in the claims to be described later.

Therefore, the technical scope of the inventive concept is not to be limited to the contents stated in the detailed description of the specification, but should be determined by the claims.

## Claims

1. A method of manufacturing a polycyclic compound represented by Formula 1, the method comprising:
providing a first material represented by Formula 2; and
adding a second material and a third material different from the second material to the first material, wherein the second material and the third material are each independently represented by Formula 3: 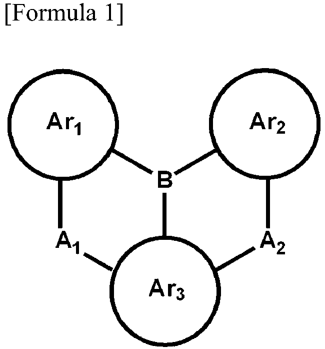 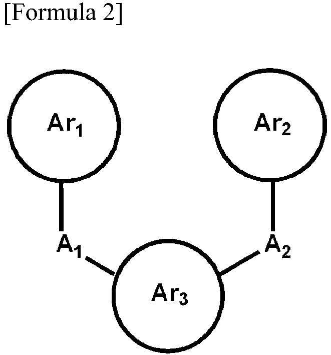
in Formulae 1 and 2,
Ar₁, Ar₂, Ar₃ are each independently a substituted or unsubstituted hydrocarbon ring of 6 to 30 ring-forming carbon atoms, or a substituted or unsubstituted heterocyclic ring of 2 to 30 ring-forming carbon atoms,
A₁ and A₂ are each independently O, S, Se or NRₓ, and
Rₓ is a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ can join with an adjacent ring group to form a fused ring: 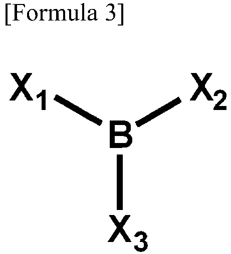
in Formula 3,
X₁, X₂, and X₃ are each independently a hydrogen atom, a deuterium atom, a halogen atom, a hydroxyl group, or a substituted or unsubstituted alkyl group of 1 to 20 carbon atoms.

2. The method of manufacturing a polycyclic compound of claim 1, wherein the polycyclic compound represented by Formula 1 is represented by Formula 1-1, and the first material represented by Formula 2 is represented Formula 2-1: 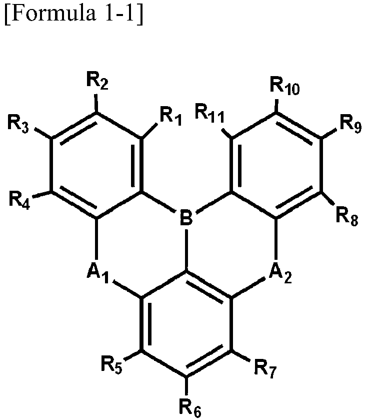 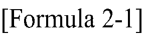 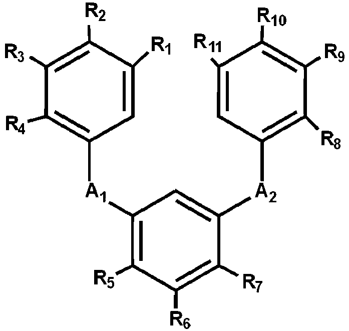 in Formula 1-1 and Formula 2-1,
R₁ to R₁₁ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or any one or more R₁ to R₁₁ can join with an adjacent R₁ to R₁₁ to form a ring, and
A₁ and A₂ are the same as defined in Formula 1.

3. The method of manufacturing a polycyclic compound of claim 2, wherein the polycyclic compound represented by Formula 1 is represented by Formula 1-2, and the first material represented by Formula 2 is represented by Formula 2-2: 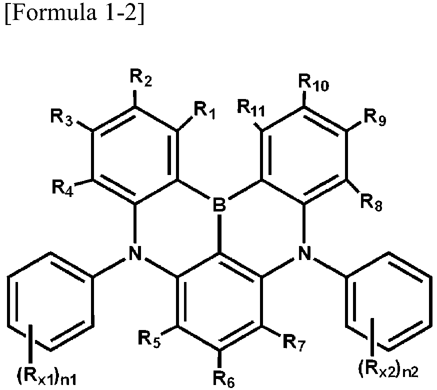 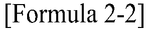 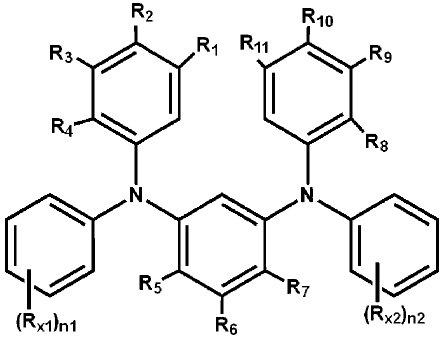 in Formula 1-2 and Formula 2-2,
Rₓ₁ and Rₓ₂ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₁ and Rₓ₂ can join with an adjacent Rₓ₁ and Rₓ₂, respectively, to form a ring, or Rₓ₁ can join with R₅ to form a ring, or Rₓ₂ can join with R₇ to form a ring,
n1 and n2 are each independently an integer of 0 to 5, and
R₁ to R₁₁ are the same as defined in Formula 1-1 and Formula 2-1.

4. The method of manufacturing a polycyclic compound of claim 2, wherein
the polycyclic compound represented by Formula 1 is represented by Formula 1-3, and the first material represented by Formula 2 is represented by Formula 2-3: 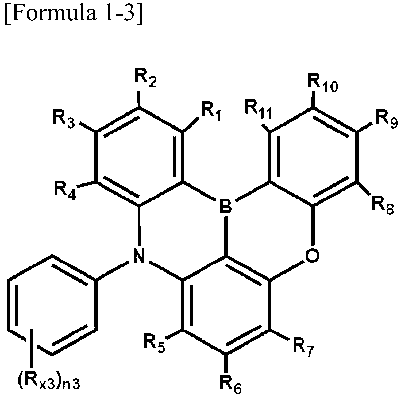 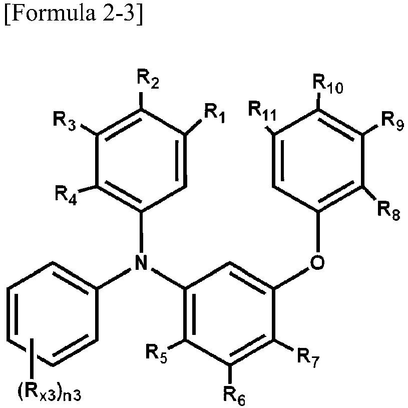
in Formula 1-3 and Formula 2-3,
Rₓ₃ is a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₃ can join with an adjacent Rₓ₃ to form a ring, or Rₓ₃ can join with R₄ or R₅ to form a ring,
n3 is an integer of 0 to 5, and
R₁ to R₁₁ are the same as defined in Formula 1-1.

5. The method of manufacturing a polycyclic compound of claim 2, wherein the polycyclic compound represented by Formula 1 is represented by Formula 1-4, and the first material represented by Formula 2 is represented by Formula 2-4: 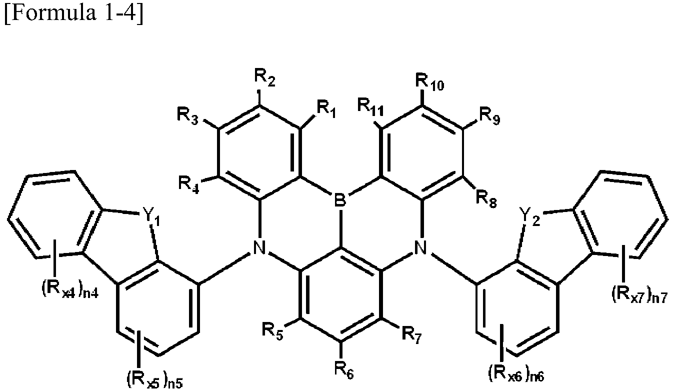 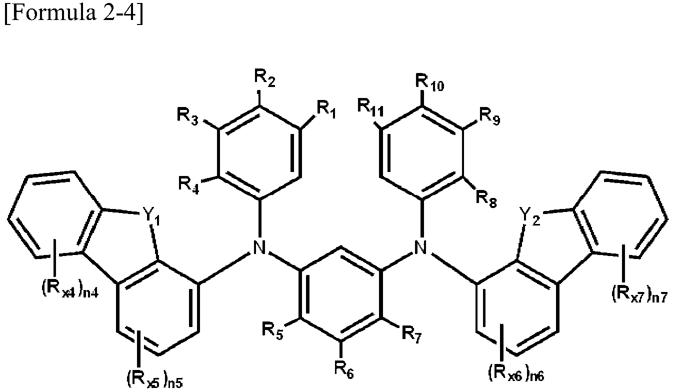 in Formula 1-4 and Formula 2-4,
Rₓ₄ to Rₓ₇ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or each Rₓ₄ to Rₓ₇ can join with an adjacent Rₓ₄ to Rₓ₇, respectively, to form a ring,
Y and Y₂ are each independently O or S,
n4 to n7 are each independently an integer of 0 to 5, and
R₁ to R₁₁ are the same as defined in Formula 1-1.

6. The method of manufacturing a polycyclic compound of any one of claims 1 to 5, wherein at least one hydrogen atom in each of the polycyclic compound and the first material is substituted with a deuterium atom.

7. The method of manufacturing a polycyclic compound of any one of claims 1 to 6, wherein:
(i) in Formula 3, X₁, X₂, and X₃ are each independently a fluorine atom, a chlorine atom, a bromine atom or an iodine atom; and/or
(ii) the second material comprises boron trichloride, and
the third material comprises boron tribromide.

8. The method of manufacturing a polycyclic compound of claim 1, wherein the polycyclic compound represented by Formula 1 includes at least one compound of Compound Group 1: 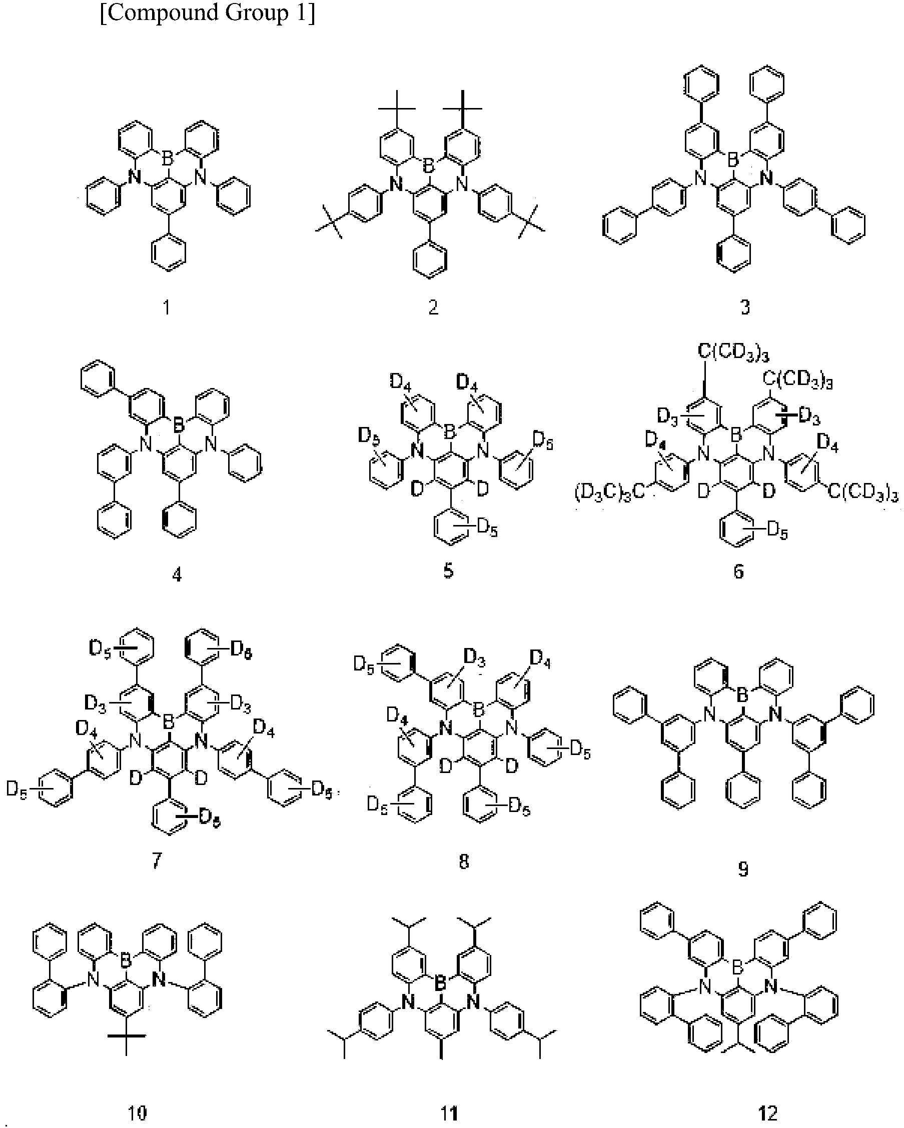 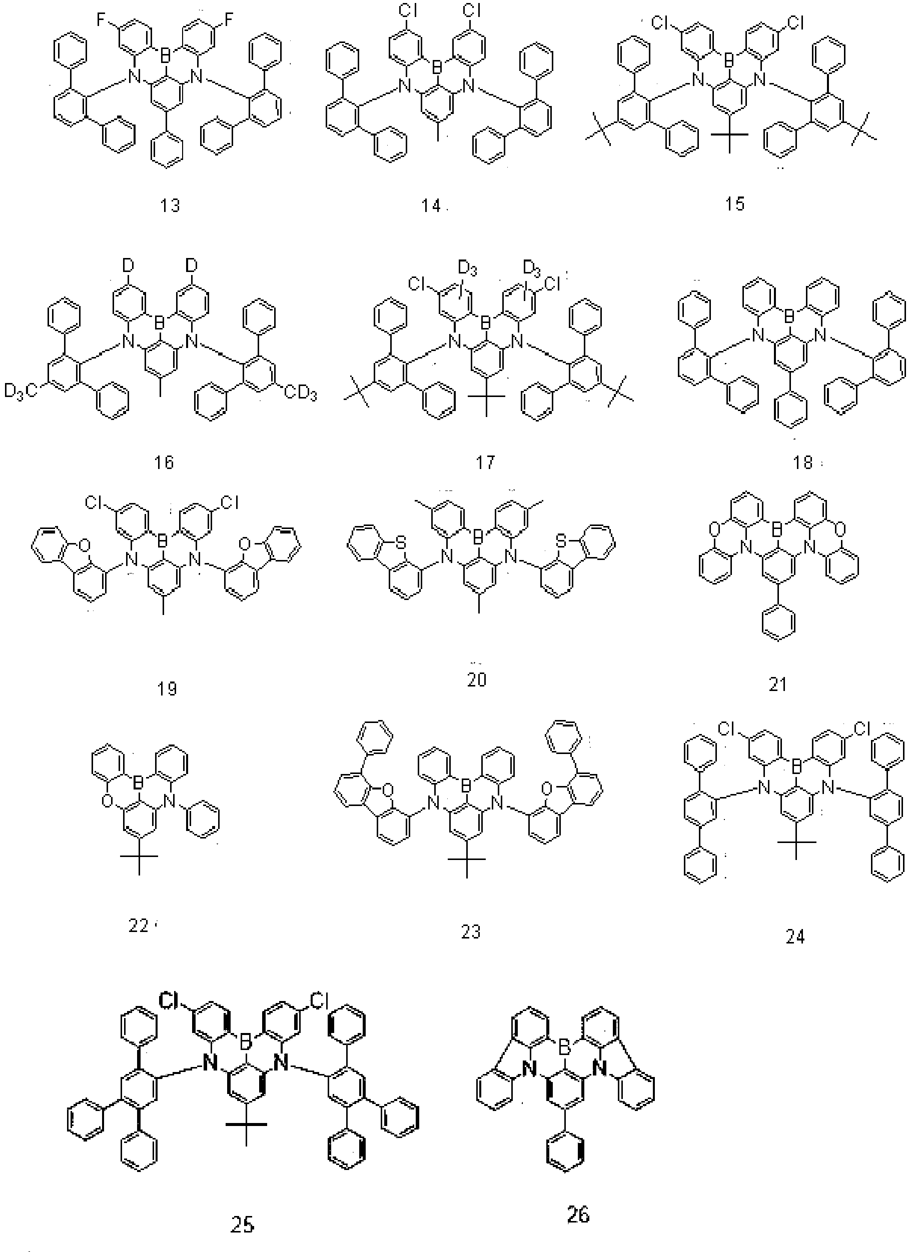 in Compound Group 1, "D" is a deuterium atom.

9. A light emitting element (ED) comprising:
a first electrode (EL1) and a second electrode (EL2); and
a light emitting layer (EML) disposed between the first electrode (EL1) and the second electrode (EL2); wherein the light emitting layer (EML) comprises a first compound represented by the following Formula 1, and the first compound is derived from a light emitting layer composition,
wherein the light emitting layer composition comprises a first material represented by the following Formula 2, a second material represented by the following Formula 3, and a third material represented by the following Formula 3 and different from the second material: 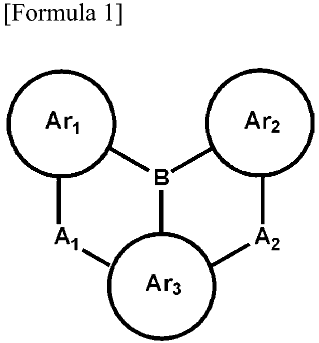 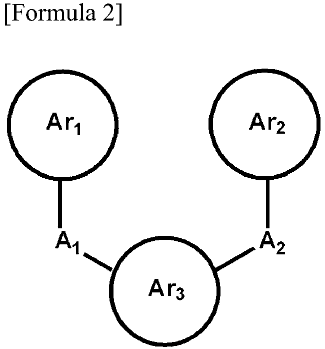
in Formulae 1 and 2,
Ar₁, Ar₂, and Ar₃ are each independently a substituted or unsubstituted hydrocarbon ring of 6 to 30 ring-forming carbon atoms, or a substituted or unsubstituted heterocycle of 2 to 30 ring-forming carbon atoms,
A₁ and A₂ are each independently O, S, Se or NRₓ, and
Rₓ is a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ can join with an adjacent ring group to form a ring: 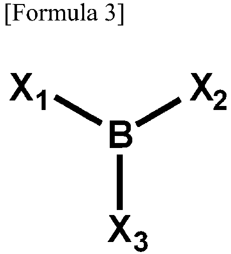
in Formula 3,
X₁, X₂, and X₃ are each independently a hydrogen atom, a deuterium atom, a halogen atom, a hydroxyl group, or a substituted or unsubstituted alkyl group of 1 to 20 carbon atoms.

10. The light emitting element (ED) of claim 9, wherein the light emitting layer (EML) further comprises at least one of a second compound represented by Formula HT-1, and a third compound represented by Formula ET-1: 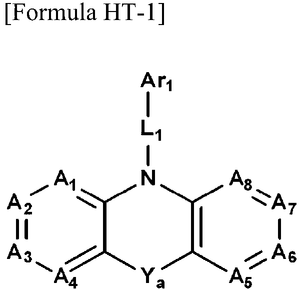 in Formula HT-1,
A₁ to A₈ are each independently N or CR₅₁,
L₁ is a direct linkage, a substituted or unsubstituted arylene group of 6 to 30 ring-forming carbon atoms, or a substituted or unsubstituted heteroarylene group of 2 to 30 ring-forming carbon atoms,
Yₐ is a direct linkage, CR₅₂R₅₃, or SiR₅₄R₅₅,
Ar₁ is a substituted or unsubstituted aryl group of 6 to 30 ring-forming carbon atoms, or a substituted or unsubstituted heteroaryl group of 2 to 30 ring-forming carbon atoms, and
R₅₁ to R₅₅ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group of 1 to 20 carbon atoms, a substituted or unsubstituted alkenyl group of 2 to 20 carbon atoms, a substituted or unsubstituted aryl group of 6 to 60 ring-forming carbon atoms, or a substituted or unsubstituted heteroaryl group of 2 to 60 ring-forming carbon atoms, or any one of R₅₁ to R₅₅ can join with an adjacent group to form a ring: 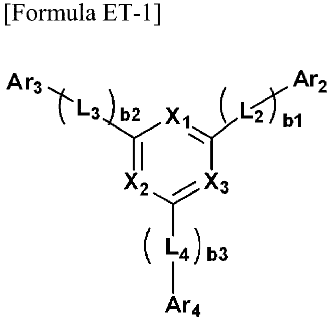
in Formula ET-1,
at least one among X₁, X₂, and X₃ is N, and the remainder are CR₅₆,
R₅₆ is a hydrogen atom, a deuterium atom, a substituted or unsubstituted alkyl group of 1 to 20 carbon atoms, a substituted or unsubstituted aryl group of 6 to 60 ring-forming carbon atoms, or a substituted or unsubstituted heteroaryl group of 2 to 60 ring-forming carbon atoms,
b1, b2, and b3 are each independently an integer of 0 to 10, Ar₂, Ar₃, and Ar₄ are each independently a hydrogen atom, a deuterium atom, a substituted or unsubstituted alkyl group of 1 to 20 carbon atoms, a substituted or unsubstituted aryl group of 6 to 30 ring-forming carbon atoms, a substituted or unsubstituted heteroaryl group with 2 to 30 ring-forming carbon atoms, or a nonaromatic ring with 2 to 30 ring-forming carbon atoms and
L₂, L₃, and L₄ are each independently a direct linkage, a substituted or unsubstituted arylene group of 6 to 30 ring-forming carbon atoms, or a substituted or unsubstituted heteroarylene group of 2 to 30 ring-forming carbon atoms.

11. The light emitting element (LD) of claim 10, wherein the light emitting layer (EML) further comprises a fourth compound represented by Formula D-1: 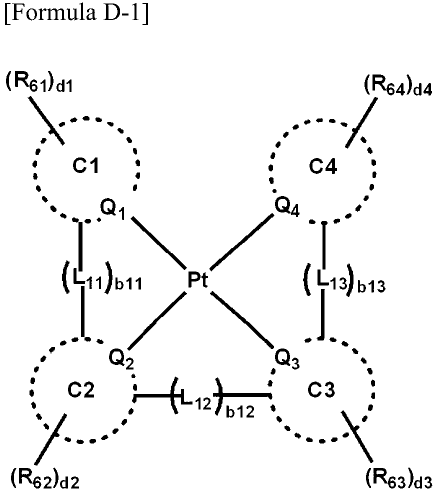 in Formula D-1,
Q₁ to Q₄ are each independently C or N,
Cl to C4 are each independently a substituted or unsubstituted hydrocarbon ring of 5 to 30 ring-forming carbon atoms, a substituted or unsubstituted aryl group having 6 to 30 ring-forming carbon atoms, or a substituted or unsubstituted heterocycle of 2 to 30 ring-forming carbon atoms,
L₁₁, L₁₂, and L₁₃ are each independently a direct linkage, 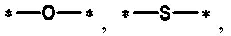 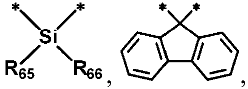 a substituted or unsubstituted divalent alkyl group of 1 to 20 carbon atoms, a substituted or unsubstituted arylene group of 6 to 30 ring-forming carbon atoms, or a substituted or unsubstituted heteroarylene group of 2 to 30 ring-forming carbon atoms,
b11, b12, and b13 are each independently 0 or 1,
R₆₁ to R₆₆ are each independently a hydrogen atom, a deuterium atom, a halogen atom, a cyano group, a substituted or unsubstituted silyl group, a substituted or unsubstituted thio group, a substituted or unsubstituted oxy group, a substituted or unsubstituted amine group, a substituted or unsubstituted boron group, a substituted or unsubstituted alkyl group of 1 to 20 carbon atoms, a substituted or unsubstituted alkenyl group of 2 to 20 ring-forming carbon atoms, a substituted or unsubstituted aryl group of 6 to 60 ring-forming carbon atoms, or a substituted or unsubstituted heteroaryl group of 2 to 60 ring-forming carbon atoms, and
d1 to d4 are each independently an integer of 0 to 4.

12. The light emitting element (ED) of any one of claims 9 to 11, wherein:
(i) the light emitting layer (EML) exhibits thermally-activated delayed fluorescence; and/or
(ii) the light emitting layer (EML) emits blue light.

13. The light emitting element (ED) of any one of claims 9 to 12, wherein
the first compound represented by Formula 1 is represented by Formula 1-1, and
the first material represented by Formula 2 is represented by Formula 2-1: 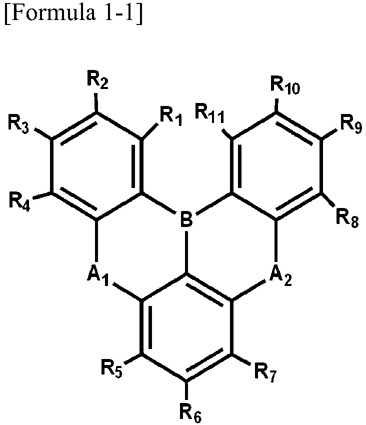 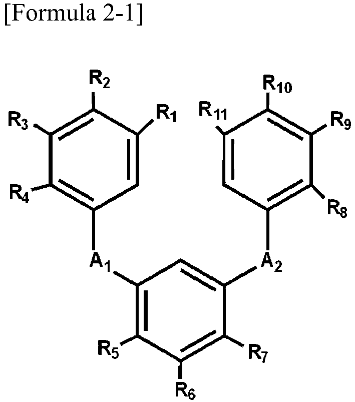
in Formula 1-1 and Formula 2-1,
R₁ to R₁₁ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or any one or more R₁ to R₁₁ can join with an adjacent group to form a ring, and
A₁ and A₂ are the same as defined in Formula 1.

14. The light emitting element of claim 13, wherein:
(i) the first compound represented by Formula 1 is represented by Formula 1-2, and
the first material represented by Formula 2 is represented by Formula 2-2: 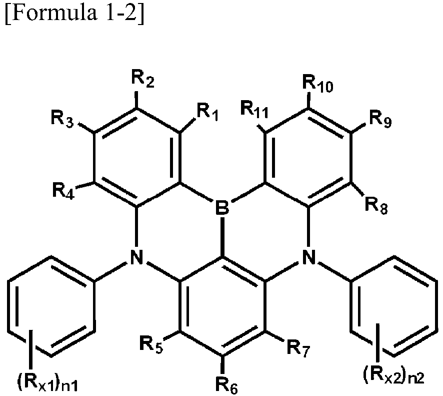 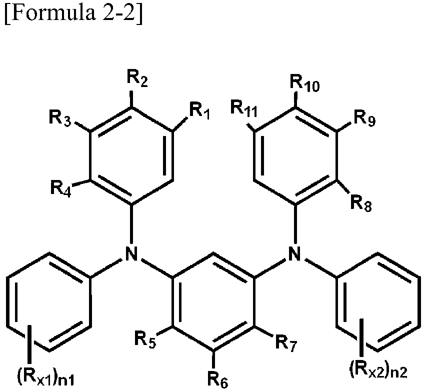
in Formula 1-2 and Formula 2-2,
Rₓ₁ and Rₓ₂ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₁ and Rₓ₂ can join with an adjacent Rₓ₁ and Rₓ₂, respectively, to form a ring, or Rₓ₁ can join with R₅ to form a ring, or Rₓ₂ can join with R₇ to form a ring,
n1 and n2 are each independently an integer of 0 to 5, and
R₁ to R₁₁ are the same as defined in Formula 1-1 and Formula 2-1; or
(ii) the first compound represented by Formula 1 is represented by Formula 1-3, and
the first material represented by Formula 2 is represented by Formula 2-3: 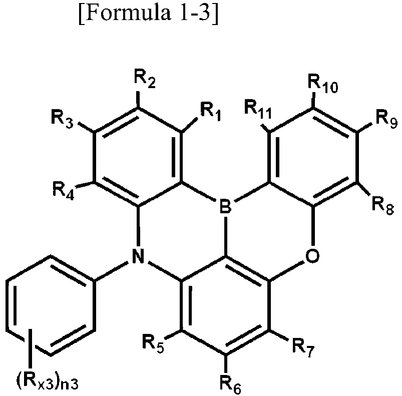 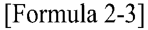 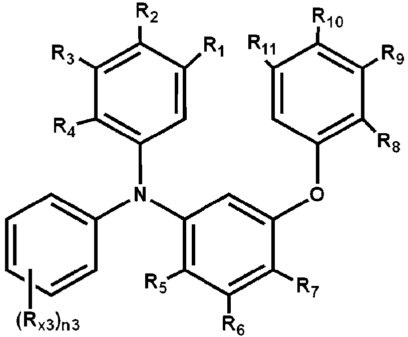
in Formula 1-3 and Formula 2-3,
Rₓ₃ is a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ₃ can join with an adjacent group Rₓ₃ to form a ring, or Rₓ₃ can join with R₄ or R₅ to form a ring,
n3 is an integer of 0 to 5, and
R₁ to R₁₁ are the same as defined in Formula 1-1; or
(iii) the first compound represented by Formula 1 is represented by Formula 1-4, and
the first material represented by Formula 2 is represented by Formula 2-4: 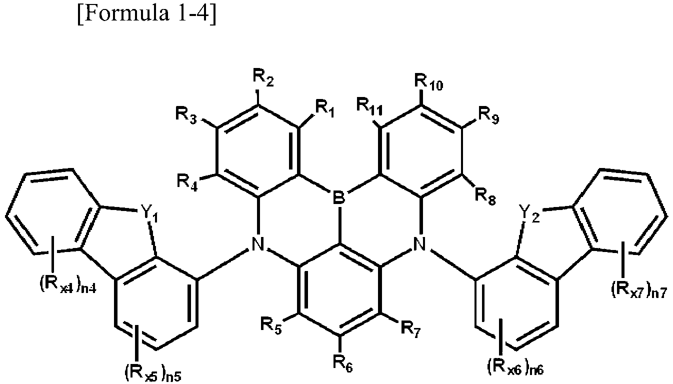 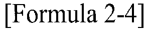 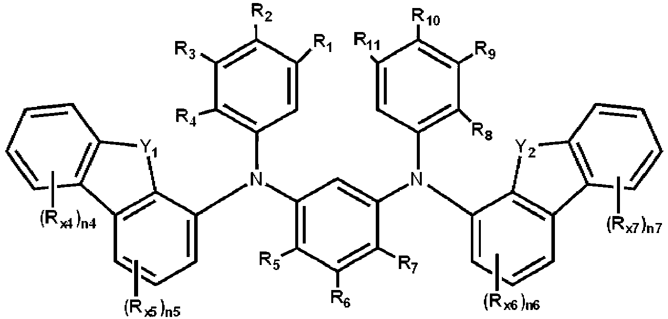
in Formula 1-4 and Formula 2-4,
Rₓ₄ to Rₓ₇ are each independently a hydrogen atom, a deuterium atom, a halogen atom, a cyano group, a substituted or unsubstituted oxy group, a substituted or unsubstituted thio group, a substituted or unsubstituted boron group, a substituted or unsubstituted amine group, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or each Rₓ₄ to Rₓ₇ can join with an adjacent Rₓ₄ to Rₓ₇ group, respectively, to form a ring,
Y and Y₂ are each independently O or S,
n4 to n7 are each independently an integer of 0 to 5, and
R₁ to R₁₁ are the same as defined in Formula 1-1.

15. An electronic device (EA) comprising a display module (DM) comprising a plurality of light emitting elements (ED), wherein
at least one of the plurality of light emitting elements (ED) comprises a first electrode (EL1) and a second electrode (EL2), and a light emitting layer (EML) disposed between the first electrode (EL1) and the second electrode (EL2), the light emitting layer (EML) comprising a first compound represented by Formula 1,
the first compound of Formula 1 is derived from a light emitting layer composition,
wherein the light emitting layer composition comprises a first material represented by the following Formula 2, a second material represented by Formula 3, and a third material represented by Formula 3 and different from the second material: 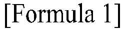 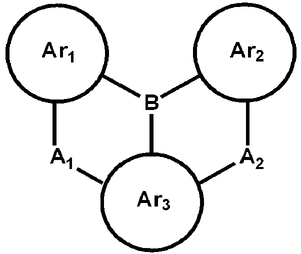 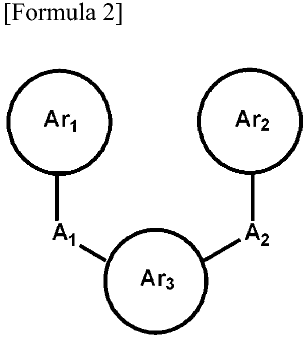
in Formulae 1 and 2,
Ar₁, Ar₂, and Ar₃ are each independently a substituted or unsubstituted hydrocarbon ring of 6 to 30 ring-forming carbon atoms, or a substituted or unsubstituted heterocycle ring of 2 to 30 ring-forming carbon atoms,
A₁ and A₂ are each independently O, S, Se or NRₓ, and
Rₓ is a hydrogen atom, a deuterium atom, a substituted or unsubstituted alkyl group of 1 to 20 carbon atoms, a substituted or unsubstituted aryl group of 6 to 30 ring-forming carbon atoms, or a substituted or unsubstituted heteroaryl group of 2 to 30 ring-forming carbon atoms, or Rₓ can join with an adjacent ring group to form a ring: 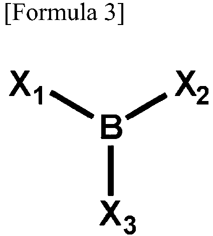
in Formula 3,
X₁, X₂, and X₃ are each independently a hydrogen atom, a deuterium atom, a halogen atom, a hydroxyl group, or a substituted or unsubstituted alkyl group of 1 to 20 carbon atoms, optionally wherein the electronic device (EA) further comprises at least one of a processor (PR), a memory (MR), and a power module (PM).
